(19) Europäisches Patentamt
European Patent Office
Office européen des brevets

(11) Publication number: **0 430 190 B1**

(12) # EUROPEAN PATENT SPECIFICATION

(45) Date of publication of patent specification: **05.07.95**

(51) Int. Cl.6: **C07D 453/02**, C07D 451/14, C07D 451/04, C07D 471/08, A61K 31/435, //(C07D471/08, 221:00,221:00)

(21) Application number: **90122689.4**

(22) Date of filing: **27.11.90**

(54) **New tricyclic compounds.**

(30) Priority: **28.11.89 US 442082**

(43) Date of publication of application:
**05.06.91 Bulletin 91/23**

(45) Publication of the grant of the patent:
**05.07.95 Bulletin 95/27**

(84) Designated Contracting States:
**AT BE CH DE DK ES FR GB GR IT LI LU NL SE**

(56) References cited:
**EP-A- 0 093 488**
**EP-A- 0 315 390**

(73) Proprietor: **SYNTEX (U.S.A.) INC.**
**3401 Hillview Avenue**
**Palo Alto**
**California 94303 (US)**

(72) Inventor: **Berger, Jacob**
**12135 Dawn Lane**
**Los Altos Hills, CA 94022 (US)**
Inventor: **Clark, Robin D.**
**2025 Columbia Street**
**Palo Alto, CA 94306 (US)**
Inventor: **Eglen, Richard M.**
**2091 Sunnyview Lane**
**Mountain View, CA 94040 (US)**
Inventor: **Smith, William L.**
**1169 Sesame Drive**
**Sunnyvale, CA 94087 (US)**
Inventor: **Weinhardt, Klaus K.**
**155 Jackson**
**San Francisco, CA 94111 (US)**

(74) Representative: **Braun, Axel et al**
**F.Hoffmann-La Roche AG**
**Grenzacherstrasse 124**
**Postfach 32 55**
**CH-4002 Basel (CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

This invention relates to novel pharmaceutical tricyclic compounds, pharmaceutical compositions containing them and methods for their use and methods for preparing these compounds. In particular, it relates to tricyclic $5\text{-}HT_3$ receptor antagonists containing a bridged bicyclic amine substituent. The invention also relates to novel intermediates for making the new tricyclic compounds.

Compounds with highly selective actions on 5-HT (serotonin or 5-hydroxytryptamine) receptor subtypes show clear potential for therapeutic benefit and provide tools with which scientists can better understand the role of 5-HT in disease. A number of different 5-HT receptor subtypes have been identified. Some of these are designated as $5\text{-}HT_1$, $5\text{-}HT_2$ and $5\text{-}HT_3$ receptors. Certain compounds having $5\text{-}HT_3$ receptor mediating activity are useful for treating emesis, CNS disorders, cognitive performance disorders, drug dependency disorders, pain (e.g. migraine), cardiovascular disorders and gastrointestinal disorders. See, for example, an article entitled "Drugs Acting On 5-Hydroxytryptamine Receptors" appearing in The Lancet September 23, 1989.

Bicyclic compounds having one or more nitrogen hetero atoms, such as e.g. benzopyrrolinone, quinazolinone and benzotriazinone derivatives are disclosed in EP-A-93488 and EP-A-315390. These compounds exhibit dopamine antagonist and $5\text{-}HT_3$ receptor antagonist activity.

Novel tricyclic compounds have now been discovered that are useful inter alia for treating a variety of conditions influenced by the $5\text{-}HT_3$ receptor. The compounds of this invention are active at very low levels, particularly in the treatment of emesis but show also activity in the treatment of other disorders as, shown below.

In a first aspect, this invention provides a compound of Formula I:

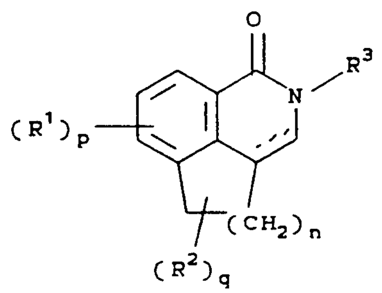

I

in which

the dashed line denotes an optional double bond;

n is 1, 2 or 3;

p is 0, 1, 2 or 3;

q is 0, 1 or 2;

each $R^1$ is independently selected from halogen, hydroxy, lower alkoxy (optionally substituted with phenyl), lower alkyl, nitro, amino, amino carbonyl, (lower alkyl)amino, di(lower alkyl)amino, and (lower alkanoyl)amino;

each $R^2$ is lower alkyl; and

$R^3$ is selected from

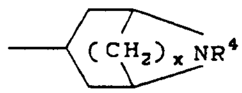

(a)

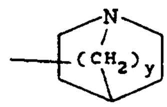

(b)

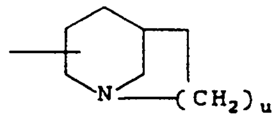

(c)

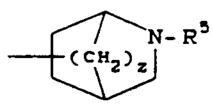

(d)

in which

u, x, y and z are each independently an integer from 1 to 3; and

$R^4$ and $R^5$ are independently $C_{1-7}$ alkyl, $C_{3-8}$ cycloalkyl, $C_{3-8}$ cycloalkyl-$C_{1-2}$ alkyl, or a group $(CH_2)_tR_6$ where t is 1 or 2 and $R_6$ is thienyl, pyrrolyl or furyl optionally further substituted by one or two substituents selected from $C_{1-6}$ alkyl, $C_{1-6}$ alkoxy, trifluoromethyl or halogen, or is phenyl optionally substituted by one or two substituents selected from $C_{1-4}$ alkoxy, trifluoromethyl, halogen, nitro, carboxy, esterified carboxy, and $C_{1-4}$ alkyl (optionally substituted by hydroxy, $C_{1-4}$ alkoxy, carboxy, esterified carboxy or *in vivo* hydrolyzable acyloxy); or

a pharmaceutically acceptable salt thereof or *N*-oxide derivative thereof, or an individual isomer or mixture of isomers thereof.

In a second aspect, this invention provides a pharmaceutical composition which contains a compound of Formula I, preferably in admixture with one or more suitable excipients.

In a third aspect, this invention relates to the use of a compound of Formula I in the manufacture of a medicament for treating emesis, gastrointestinal disorders, CNS disorders including cognitive performance disorders and drug dependency, cardiovascular disorders or pain.

In a fourth aspect, this invention provides compounds of Formula II which are useful intermediates for preparing compounds of Formula I:

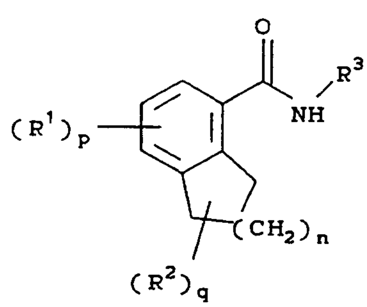

II

wherein n, p, q, $R^1$, $R^2$ and $R^3$ are as defined for Formula I.

A fifth aspect of this invention is a process for preparing compounds of Formula I and is set forth in the "Detailed Description Of The Invention."

## Definitions

Unless otherwise stated, the following terms used in the specification and claims have the meanings given below:

"Alkyl" means a straight or branched saturated hydrocarbon radical having from one to the number of carbon atoms designated. For example $C_{1-7}$ alkyl is alkyl having at least one but no more than seven carbon atoms, e.g. methyl, ethyl, *i*-propyl, *n*-propyl, *n*-butyl, pentyl, heptyl and the like.

"Lower alkyl" means an alkyl of one to six carbon atoms.

"Lower alkoxy", "(lower alkyl)amino", "di(lower alkyl)amino", "(lower alkanoyl)amino", and similar terms mean alkoxy, alkylamino, dialkylamino, alkanoylamino, etc. in which the or each alkyl radical is a "lower alkyl" as described above.

"Halogen" means fluorine, chlorine, bromine, or iodine. Preferred halogens are chlorine and bromine;

"Pharmaceutically acceptable" means that which is useful in preparing a pharmaceutical composition that is generally safe and non-toxic and includes that which is acceptable for veterinary use as well as human pharmaceutical use.

"Pharmaceutically acceptable salts" means salts which possess the desired pharmacological activity and which are neither biologically nor otherwise undesirable. Such salts include acid addition salts formed with inorganic acids such as hydrochloric acid, hydrobromic acid, sulfuric acid, nitric acid, phosphoric acid, and the like; or with organic acids such as acetic acid, propionic acid, hexanoic acid, heptanoic acid, cyclopentanepropionic acid, glycolic acid, pyruvic acid, lactic acid, malonic acid, succinic acid, malic acid, maleic acid, fumaric acid, tartaric acid, citric acid, benzoic acid, o-(4-hydroxy-benzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, p-chlorobenzenesulfonic acid, 2-naphthalenesulfonic acid, *p*-toluenesulfonic acid, camphorsulfonic acid, 4-methyl-bicyclo[2.2.2]oct-2-ene-1-carboxylic acid, glucoheptonic acid,
4,4'-methylenebis(3-hydroxy-2-naphthoic) acid, 3-phenylpropionic acid, trimethyl-acetic acid, tertiary butylacetic acid, lauryl sulfuric acid, gluconic acid, glutamic acid, hydroynaphthoic acid, salicylic acid, stearic acid, muconic acid, and the like. Preferred pharmaceutically acceptable salts are those formed with hydrochloric acid.

The compounds of Formula I with a $R^1$ hydroxy group are capable of forming salts with inorganic or organic bases. Preferred pharmaceutically acceptable bases include sodium hydroxide, sodium carbonate, potassium hydroxide, aluminum hydroxide, calcium hydroxide and organic bases such as diethanolamine, tromethamine, N-methylglucamine, ethanolamine, triethanolamine and others.

"*N*-oxide derivative" of a compound means the form of a compound of Formula I wherein the nitrogen of the $R^3$ moiety of Formula I is in the oxidized state, e.g.

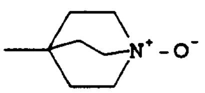

In defining the subject matter of this invention reference is made in the structure to substituents $(R^1)_p$ and $(R^2)_q$. It should be noted that the list of substituents of $R^1$ and $R^2$ does not include hydrogen, and each of p and q can be 0. It is to be understood that when p or q is 0, the respective ring structures will not be substituted and thus will have only hydrogens around the ring.

"Animal" includes humans, non-human mammals (such as dogs, cats, rabbits, cattle, horses, sheep, goats, swine, and deer) and non-mammals such as birds and the like.

"Disease" specifically includes any unhealthy condition of an animal or part thereof and may be caused by, or incident to, medical or veterinary therapy applied to that animal, i.e. the "side effects" of such therapy. Thus, "disease" here includes the nausea and emesis caused by therapy with agents having emetogenic side effects, in particular by therapy for cancer, such as chemotherapy with cytotoxic agents and radiotherapy.

"Treatment" means any treatment of a disease in an animal and includes:

(1) preventing the disease from occurring in an animal which may be predisposed to the disease but does not yet experience or display symptoms of the disease,

(2) inhibiting the disease, i.e. arresting its development, or

(3) relieving the disease, i.e causing regression of the disease.

"Effective amount" for a disease means that amount which, when administered to an animal in need thereof, is sufficient to effect treatment, as defined above, for that disease.

Certain compounds of Formula I and Formula II may exist as optical isomers. In the compounds of the invention, any isomer or mixture of isomers may be used and the claims are intended to cover the individual isomer and mixtures thereof, unless otherwise restricted. The invention includes all optical

isomers of any asymmetrical compound of Formula I, as well as mixtures thereof.

"Isomerism" refers to compounds having the same atomic mass and atomic number but differing in one or more physical or chemical properties. Various types of isomers include the following:

"Stereoisomer" refers to a chemical compound having the same molecular weight, chemical composition, and constitution as another, but with the atoms grouped differently. That is, certain identical chemical moieties are at different orientations in space and, therefore, when pure, have the ability to rotate the plane of polarized light. However, some pure stereoisomers may have an optical rotation that is so slight that it is undetectable with present instrumentation.

"Optical isomer" describes one type of stereo isomerism which manifests itself by the rotation that the isomer, either pure or in solution, imparts to the plane of polarized light. It is caused in many instances by the attachment of four different chemical atoms or groups to at least one of the carbon atoms in a molecule.

Stereoisomers or optical isomers that are mirror images of one another are termed "enantiomers" and may be said to be enantiomeric. Chiral groups that are mirror images of one another are termed enantiomeric groups.

Enantiomers whose absolute configurations are not known may be differentiated as dextrorotatory (prefix +) or laevorotatory (prefix -) depending on the direction in which, under specified experimental conditions, they rotate the plane of polarized light.

When equal amounts of enantiomeric molecules are present together, the product is termed racemic, independently of whether it is crystalline, liquid, or gaseous. A homogeneous solid phase composed of equimolar amounts of enantiomeric molecules is termed a racemic compound. A mixture of equimolar amounts of enantiomeric molecules present as separate solid phases is termed a racemic mixture. Any homogeneous phase containing equimolar amounts of enantiomeric molecules is termed a racemate.

"Diastereoisomer" refers to stereoisomers some or all of which are dissymmetric but which are not mirror images of each other. Diastereoisomers corresponding to a given structural formula must have at least two asymmetric atoms. A compound having two asymmetric atoms will usually exist in four diastereoisomeric forms, i.e. (-)-erythro, (+)-erythro, (-)-threo and (+)-threo.

The optically active compounds herein can be designated by a number of conventions; i.e., the *R*- and *S*-sequencing rules of Cahn and Prelog; erythro and threo isomers; D- and L-isomers; d- and l-isomers; and (+) and (-) isomers, which indicates the direction a plane of polarized light is rotated by the chemical structure, either pure or in solution. These conventions are well known in the art and are described in detail by E.L. Eliel in Stereochemistry of Carbon Compounds, published by McGraw Hill Book Company, Inc. of New York in 1962 and references cited therein. Thus, these isomers may be described as d-, l-, or a d,l-pair; or D-, L-, or a D,L-pair; or *R*-, *S*-, or an *R,S*-pair; depending upon the nomenclature system employed. In general, this application will use the (*R*), (*S*) and (*RS*) designation.

"Optional" or "optionally" means that the subsequently described event or circumstance may or may not occur, and that the description includes instances where said event or circumstance occurs and instances in which it does not. For example, "optionally substituted phenyl" means that the phenyl may or may not be substituted and that the description includes both unsubstituted phenyl and phenyl wherein there is substitution; "optionally followed by converting the free base to the acid addition salt" means that said conversion may or may not be carried out in order for the process described to fall within the invention, and the invention includes those processes wherein the free base is converted to the acid addition salt and those processes in which it is not.

Certain $R^3$ substituents are of particular interest for the compounds of this invention and are therefore defined specifically. In some cases the $R^3$ substituent will exhibit a chiral center at the ring carbon which is bonded to the amide nitrogen. It is to be understood that a straight line representing the covalent bond between the chiral carbon and the amide nitrogen is understood to represent either the *R* or *S* configuration, or a mixture (not necessarily racemic) thereof. These $R^3$ substituents of particular interest are as follows:

(1) subformula (b) where y is 2 having the specific formula

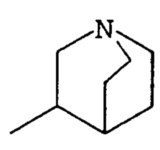

is referred to as 1-azabicyclo[2.2.2]oct-3-yl;

(2) subformula (b) where y is 2 having the specific formula

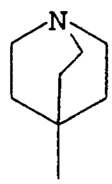

is referred to as 1-azabicyclo[2.2.2]oct-4-yl;
(3) subformula (a) where x is 3 and $R^4$ is methyl having the specific formula

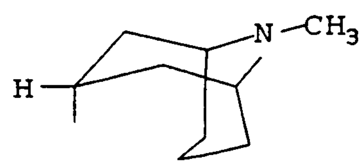

is referred to as
*endo*-9-methyl-9-azabicyclo[3.3.1]non-3-yl;
(4) subformula (a) where x is 2 and $R^4$ is methyl having the specific formula

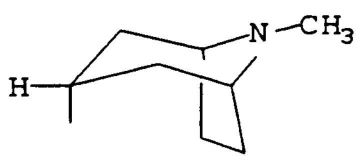

is referred to as
*endo*-8-methyl-8-azabicyclo[3.2.1]oct-3-yl;
(5) subformula (a) where x is 2 and $R^4$ is methyl having the specific formula

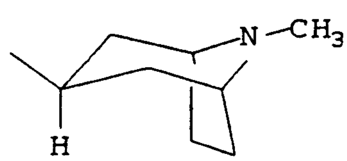

is referred to as
*exo*-8-methyl-8-azabicyclo[3.2.1]oct-3-yl; and
(6) subformula (c) wherein u is 2 having the specific formula

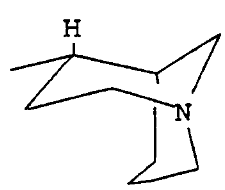

is referred to as *endo*-1-azabicyclo[3.3.1]non-4-yl.

The compounds of Formula I are named in accordance with generally acceptable nomenclature rules established by "Chemical Abstracts." The naming depends primarily on whether n is 1, 2 or 3. For example, the compound of the following formula where n = 1 and $R^3$ = 1-azabicyclo-[2.2.2]oct-4-yl:

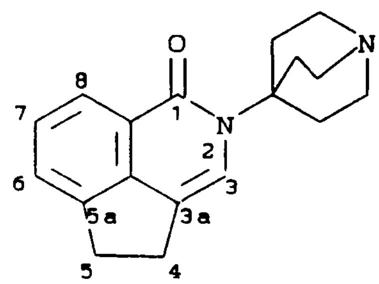

is named 2-(1-azabicyclo[2.2.2]oct-4-yl)-1,2,4,5-tetrahydrocyclopent[*de*]isoquinolin-1-one.

The compound of the following formula where n = 2 and $R^3$ = 1-azabicyclo[2.2.2]oct-4-yl:

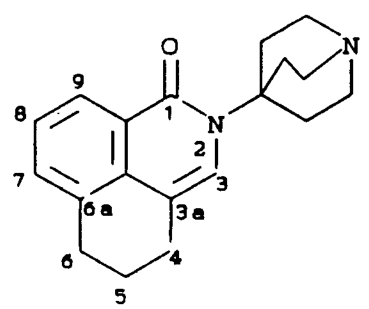

is named 2-(1-azabicyclo[2.2.2]oct-4-yl)-2,4,5,6-tetrahydro-1*H*-benz[*de*]isoquinolin-1-one.

The compound of the following formula where n = 3 and $R^3$ = 1-azabicyclo[2.2.2]oct-4-yl:

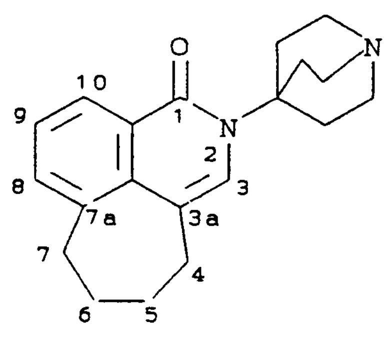

is named 2-(azabiyclo[2.2.2]oct-4-yl)-1,2,4,5,6,7-hexahydrocyclohept[*de*]isoquinolin-1-one.

Utility

The compounds of this invention, as defined by Formula I, exhibit pharmaceutical activity and in particular $5\text{-}HT_3$ receptor antagonist activity. As such, these compounds are useful for treating a broad range of conditions in animals, particularly humans, in which the $5\text{-}HT_3$ receptor plays a role. Examples of conditions that may be treated using the compounds of this invention include emesis, gastrointestinal disorders, central nervous system (CNS) disorders including cognitive performance disorders, drug dependency, etc., cardiovascular disorders and pain.

For purposes of this patent application, particularly the claims the term "emesis" will have a meaning that is broader than the normal, dictionary definition and includes not only vomiting, but also nausea and retching. Such a condition of emesis may be induced by or result from the administration of chemotherapeutic or cytotoxic agents or radiation in cancer treatment or from the exposure to radiation, surgical operations or anesthesia or motion sickness (caused by riding in a vehicle, airplane, vessel, etc.). The compounds of this invention can be referred to as anti-emetics and are particularly valuable for treating (especially preventing) emesis induced in cancer patients by treatments with cytotoxic pharmaceutical agents or radiation. Such cytotoxic agents include platinum anti-cancer agents such as cisplatin (*cis*-diamminedichloroplatinum), as well as non-platinum anti-cancer drugs such as cyclophosphamide (cytoxin), vincristrine (leurocristine), procarbazine (*N*-(1-methylethyl)-4-[(2-methylhydrazino)methyl]benzamide), methotrexate, fluorouracil, mechlorethamine hydrochloride (2-chloro-*N*-(2-chloroethyl)-*N*-methylethanamine hydrochloride), doxorubicin, adriamycin, dactinomycin (actinomycin-D) cytarabine, carmustine, dacarbazine, and others listed at page 1143 of the Journal of Clinical Oncology 1989; 7(*8*): 1143.

The compounds of the invention may also be useful for treating post-operative nausea and vomiting and motion sickness and for the treatment of all conditions described hereinbefore.

The compounds of Formula I are useful in the treatment of gastrointestinal disorders (e.g. of the stomach, esophagus and both the large and small intestines). Examples of specific conditions that may be treated using the compounds of this invention include, but are not limited to, dyspepsia (including non-ulcer dyspepsia), gastric stasis, peptic ulcer, reflux esophagitis, flatulence, bile reflux gastritis, pseudo-obstruction syndrome, irritable colon syndrome (which may result in chronic constipation or diarrhea), diverticular disease, biliary dysmotility (which may result in sphincter of Oddi dysfunction and "sludge" or microscopic crystals in the gall bladder), gastroparesis (e.g. diabetic, postsurgical or idiopathic), irritable bowel syndrome and retarded gastric emptying. The compounds of the invention are also useful as short-term prokinetics to facilitate diagnostic radiology and intestinal intubation. In addition the compounds are useful for treating diarrhea, particularly diarrhea induced by cholera and carcinoid syndrome.

The compounds of Formula I are useful in treating CNS disorders. Some of the categories of treatable CNS disorders include cognitive disorders, psychoses, anxiety/depression and obsessive/compulsive behavior. Cognitive disorders include attentional or memory deficit, dementia states (including senile dementia of the Alzheimer's type and aging), cerebral vascular deficiency and Parkinson's disease. Psychoses that may be treated using the compounds of this invention include paranoia and schizophrenia. Representative, treatable anxiety/depressive states include anticipatory anxiety (e.g. prior to surgery, dental work, etc.), depression, mania, convulsions and anxiety caused by withdrawal from addictive substances such as nicotine, alcohol, common narcotics, cocaine and other drugs of abuse. Finally obsessive/compulsive behavior, e.g. that which results in obesity, may be treated using the compounds of this invention.

Cardiovascular disorders that may be treated using a compound of this invention are those that are mediated by the presence of serotonin. Examples of such disorders include arrhythmias and hypertension.

It is thought that the compounds of this invention prevent certain adverse nervous transmissions and/or prevent vasodilation and thus reduce the perceived level of pain. Examples of pain treatable using a compound of this invention include cluster headaches, migraines, trigeminal neuralgia and visceral pain (e.g. that caused by abnormal distension of hollow visceral organs).

To determine the 5-$HT_3$ antagonist activity of compounds of this invention one of ordinary skill may use the Rat Cerebral Cortex Binding Assay, a predictive *in vitro* assay which assesses the binding affinity of a compound for the 5-$HT_3$ receptor. The method is described in Kilpatrick, G.J., Jones, B.J. and Tyers, M.B., Nature 1987; *330*: 24-31. The assay as adapted for testing compounds of the invention and results are set out in Example 11 of this application. The compounds of Formula I exhibit affinity for the 5-$HT_3$ receptor in this assay.

The von Bezold-Jarisch test for 5-$HT_3$ antagonist activity in rats is an accepted test for determining 5-$HT_3$ antagonist activity *in vivo* by measuring the von Bezold-Jarisch reflex in anesthetized rats. See, e.g., Butler, A., Hill, J.M., Ireland, S.J., Jordan, C.C., Tylers, M.B., Brit. J. Pharmacol. 1988; *94*: 397-412; Cohen, M.L., Bloomquist, W., Gidda, J.S., Lacefield, W., J. Pharmacol. Exp. Ther. 1989; *248*: 197-201; and Fozard, J.R., MDL 72222: Arch. Pharmacol. 1984; *326*: 36-44. The compounds of the invention exhibit activity in the von Bezold-Jarisch test. The details of the procedure (as modified for testing the compounds of the invention) and results are set out in Example 14 of this application. Compounds of Formula I reduce cisplatin-induced emesis in the ferret.

The cisplatin-induced emesis test in ferrets is an accepted test for determining anti-emetic activity *in vivo,* see e.g. Costall, B., Domeney, A.M., Naylor, R.J., and Tattersall, F.D., Neuropharmacology 1986; *25–(8)*: 959-961; and Miner, W.D. and Sanger G.J., Brit. J. Pharmacol. 1986; *88*: 497-499. A general description and results are set out in Example 13 of this application. Compounds of Formula I reduce

cisplatin-induced emesis in the ferret.

Anti-emetic properties in the control of emesis in dogs due to administration of platinum anti-cancer drugs are also determined by a modification of the method described by Smith, W.L., Alphin, R.S., Jackson, C.B., and Sancilio, L.F., J. Pharm. Pharmacol. 1989; *41*: 101-105; and Gylys, J.A., Res. Commun. Chem. Pathol. Pharmacol. 1979; *23(1)*: 61-68 as follows: cisplatin (*cis*-diamminedichloroplatinum) is administered at a dose of 3 mg/kg intravenously to non-fasted dogs (both sexes). Sixty minutes after cisplatin administration, the test drug in saline at a dose volume of 0.1 ml/kg is administered intravenously. A control group of dogs is given the cisplatin followed by saline at 60 min, without test drug. The dogs are observed continuously for a period of 5 hr counting the number of emetic episodes and comparing them to emetic episodes observed for the controls.

The utility for treating gastrointestinal disorders is determined by assaying the gastrokinetic pharmacological activity using the method of Droppleman, D., Gregory, R., and Alphin, R.S., J. Pharmacol. Methods 1980; *4(3)*: 227-30 wherein the rate of emptying of a test meal in rats compared to controls was observed. The Droppleman et al. method is an accepted method for determining gastrointestinal activity *in vivo.* The compounds of the invention exhibit activity in the Droppleman et al. method, the detail of which is set out in Example 12. The compounds of Formula I show activity in this assay.

The utility for treatment of a CNS disorder such as anxiety (anxiolytic activity) is determined by the art-recognized Crawley and Goodwin two-compartment exploratory model as described in Kilfoil, T., Michel, A., Montgomery, D., and Whiting, R.L., Neuropharmacology 1989; *28(9)*: 901-905. In brief, the method involves determining whether a compound reduces the natural anxiety of mice in brightly-lit areas. Compounds of the invention are active in this art recognized test. An example is set forth in Example 15 of this application. Compounds of Formula I are active in this test.

Cognition enhancing activity may be determined by the mouse habituation/cognitive enhancement test. See procedures described in Barnes, J.M., Costall, B., Kelly, M.E., Naylor, F.J., Onaivi, E.S., Tomkins, D.M. and Tyers, M.B. Br. J. Pharmacol. 98, 693P (1989). This procedure utilizes the exploratory model described above to test for improvements in the impaired cognitive performance of aged mice. A detailed description is set forth in Example 18 of this application. Compounds of Formula I enhance cognitive performance in this test.

Anxiolytic activity during withdrawal from drugs of abuse is determined by the mouse light/dark withdrawal anxiety test. This procedure utilizes the exploratory model described above to test for anxiolytic activity after administration and subsequent abrupt cessation of alcohol, cocaine or nicotine treatments. A detailed description is set for in Example 17 of this application. Compounds of Formula I are effective at reversing the drug withdrawal-induced anxiety in this test.

All of the aforementioned citations to *in vitro* and *in vivo* methods for determining activity of the compounds of this invention and other documents cited herein are incorporated herein by reference.

In summary then another aspect of this invention is a method for treating an animal exhibiting a condition in which the 5-$HT_3$ receptor plays a role, e.g. where the condition is chosen from emesis, a gastrointestinal disorder, a CNS disorder, a cardiovascular disorder and pain, which method comprises administering a therapeutically effective amount of a compound of Formula I to such mammal. The compounds are particularly valuable for treating humans.

A therapeutically effective amount of a compound is an amount that is efficacious in treating the condition, i.e. the disease. The exact amount administered may vary over a wide range depending on the degree of severity of the specific condition being treated, age of the subject, relative health of the subject and other factors. A therapeutically effective amount may vary from about 0.000001 mg (1 nanogram [ng]) per Kg body weight per day to about 10.0 mg/Kg body weight per day. Preferably the amount will be about 10 ng/Kg/day to about 1.0 mg/Kg/day, especially for anti-emetic purposes. Thus, for a 70 Kg human, a therapeutically effective amount may be from about 70 ng/day to 700 mg/day, preferably about 700 ng/day to about 70 mg/day.

## Administration and Pharmaceutical Composition

The compounds of this invention may be administered via any of the usual and acceptable modes known in the art, either singly or in combination with another compound of this invention or with another therapeutic agent. Generally a compound of this invention is administered as a pharmaceutical composition with a pharmaceutically acceptable excipient and is administered orally, systemically (e.g. transdermally, intranasally or by suppository) or parenterally (e.g. intramuscularly [im], intravenously [iv] or subcutaneously [sc]). The compounds of the invention can thus be administered in a composition that is a semisolid, powder, aerosol, solution, suspension or other appropriate composition, as discussed hereinafter.

A pharmaceutical composition comprises a compound of Formula I, wherein each substituent is defined hereinabove, preferably in combination with a pharmaceutically acceptable excipient. Such excipient is one that is non-toxic and acts to aid in the administration of the compound of this invention. Such excipient may be any solid, liquid, semisolid, gaseous (in case of an aerosol) excipient that is generally available to one of skill in the art and that does not adversely affect the activity of the active agent.

In general, the pharmaceutical composition of this invention will contain a therapeutically effective amount of a compound in combination with at least one excipient. Depending on the type of formulation, size of a unit dosage, kind of excipients and other factors known to those of skill in the art of pharmaceutical sciences the amount of compound of this invention may vary over a wide range in the composition. In general, the final composition will comprise about 0.001%w to about 99.5%w of a compound of the invention with the remainder being the excipient or excipients. Preferably the level of active compound will be about 0.01%w to about 10.0% and most preferably about 0.1%w to about 1.0%w, with the remainder being a suitable excipient or excipients.

Useful pharmaceutical excipients for the preparation of the pharmaceutical compositions hereof can be solids, semisolids, liquids or gases. Thus, the compositions can take the form of tablets, pills, capsules, powders, sustained release formulations, solutions, suspensions, elixirs, aerosols, and the like. Solid pharmaceutical excipients include starch, cellulose, talc, glucose, lactose, sucrose, gelatin, malt, rice, flour, chalk, silica gel, magnesium stearate, sodium stearate, glycerol monostearate, sodium chloride, dried skim milk, and the like. Liquid and semisolid excipients may be selected from water, ethanol, glycerol, propylene glycol, various oils, including those of petroleum, animal, vegetable or synthetic origin, for example, peanut oil, soybean oil, mineral oil, sesame oil, and the like. Water, saline, aqueous dextrose, and glycols are preferred liquid carriers, particularly for injectable solutions. Compressed gases are frequently used to dispense the active ingredient in aerosol form. Inert gases suitable for this purpose are nitrogen, carbon dioxide, nitrous oxide, etc. Other suitable pharmaceutical carriers and their formulations are described in "Remington's Pharmaceutical Sciences" by E. W. Martin.

Preferably the pharmaceutical composition is administered in a single unit dosage form for continuous treatment or in a single unit dosage form ad libitum when relief of symptoms is specifically required.

Presently Preferred Embodiments

While the broadest definitition of this invention is set forth in the Summary of the Invention as a compound of Formula I wherein each of n, p, q, $R^1$, $R^2$, $R^3$, $R^4$, $R^5$, u, x, y and z is defined in its broadest aspect, certain compounds of the invention are preferred. For example, the compounds of Formula I wherein p is 0, 1, or 2; n is 1 or 2; q is 0, $R^1$ is halogen, lower alkoxy or amino; and if $R^3$comprises $R^4$ and $R^5$ they are each lower alkyl or $R^3$ is a bicyclic substituent as specified in the Summary of the Invention without further substituents. Of this subgroup those of particular interest include compounds of Formula I wherein p is 0, the dashed line represents a double bond and if $R^3$ comprises $R^4$ and $R^5$ they are each methyl, with particularly preferred compounds being those wherein $R^3$ is 1-azabicyclo[2.2.2]oct-3-yl;
1-azabicyclo-[2.2.2]oct-4-yl;
*endo*-9-methyl-9-azabicyclo[3.3.1]non-3-yl;
*endo*-8-methyl-8-azabicyclo[3.2.1]oct-3-yl;
*exo*−8-methyl-8-azabicyclo[3.2.1]oct-3-yl; or
*endo*-1-azabicyclo[3.3.1]non-4-yl. Representative examples are set out in Examples 4 and 5.

Another subgroup of particular interest includes compounds of Formula I wherein n is 1 or 2; p and q are both 0; the dashed line represents 2 hydrogens; and if $R^3$ comprises $R^4$ and $R^5$ they are each methyl or $R^3$ is a bicyclic substituent as specified above without further substituents. Particularly preferred compounds are those wherein $R^3$ is 1-azabicyclo[2.2.2]oct-3-yl;
1-azabicyclo-[2.2.2]oct-4-yl;
*endo*-9-methyl-9-azabicyclo[3.3.1]non-3-yl;
*endo*-8-methyl-8-azabicyclo[3.2.1]oct-3-yl;
*exo*-8-methyl-8-azabicyclo[3.2.1]oct-3-yl; or
*endo*-1-azabicyclo[3.3.1]non-4-yl. Representative compounds are made by following the procedure set forth in Example 7.

Still another subgroup of compounds of particular interest include those of Formula I wherein p is 0, 1 or 2 (especially where p is 0); n is 3; q is 0; R is halogen, lower alkoxy or amino; and if $R^3$comprises $R^4$ and $R^5$ they are each lower alkyl (particularly methyl) or $R^3$ is a bicyclic substituent as specified above without further substituents. Of these, compounds where the dashed line is a double bond are of particular interest, particularly those wherein

$R^3$ is 1-azabicyclo[2.2.2]oct-3-yl;
1-azabicyclo-[2.2.2]oct-4-yl;
*endo*-9-methyl-9-azabicyclo[3.3.1]non-3-yl;
*endo*-8-methyl-8-azabicyclo[3.2.1]oct-3-yl;
*exo*-8-methyl- 8-azabicyclo[3.2.1]oct-3-yl; or
*endo*-1-azabicyclo[3.3.1] non-4-yl. Representative examples are set forth in Example 6.

It is understood that these subgroups of particular interest are particularly useful in the pharmaceutical compositions and methods of treatment of this invention.

Processes for Preparing Compounds of the Invention

The compounds of Formula I are prepared by a variety of methods. The synthetic approaches are apparent from the numbered dotted lines (1 to 6) in Formula I below. The dotted lines point schematically to the respective reaction sites and the ensuing table gives a brief description of the various methods that will be described in more detail below. The last column in the table and the letter symbols in parentheses refer to the respective step in the process claim(s).

Formula I

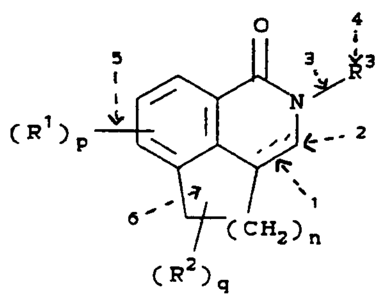

| Approach | Method | Step |
|---|---|---|
| 1. | Hydrogenation | (b) |
| 2. | Formylation | (a) |
| 3. | Alkylation | (d) |
| 4. | N-Oxidation or Amine oxide Reduction | (f) |
| 5. | Elaboration of Substituent $R^1$ | (g) |
| 6. | Hydrogenation | (h) |
| 7.* | Salt Formation | (e) |
| 8.* | Liberation of Free Base | (c) |
| 9.* | Optical Resolution | (f) |
| 10.* | Separation of Diastereomers | (i) |

*Not shown on Formula I

Accordingly, the process for the preparation of a compound of Formula I comprises one or more of the following steps:

(a) reactively contacting a compound of Formula II

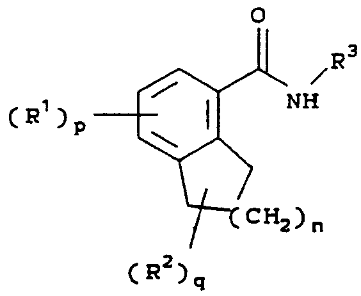

II

in which n, p, q, $R^1$, $R^2$, and $R^3$ are as defined above with a formylating agent in the presence of a strong base to form a compound of Formula I wherein the dashed line is a double bond,

(b) reducing the double bond represented by the dashed line in Formula I by hydrogenation to form a compound of Formula I wherein the dashed line represents 2 hydrogens,

(c) converting a salt of a compound of Formula I to the corresponding free compound,

(d) condensing a compound of the formula $R^3L$, wherein $R^3$ has the above meanings and L is a leaving group with a compound of the Formula XIII,

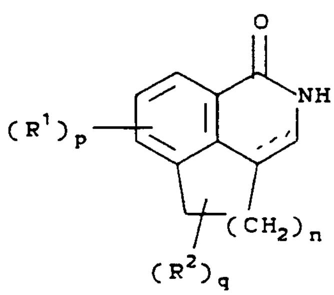

XIII

wherein $R^1$, $R^2$, n, p, q, and the dashed line have the above meanings,

(e) converting a compound of Formula I to the corresponding pharmaceutically acceptable salt,

(f) oxidizing a compound of Formula I to form the corresponding *N*-oxide of the $R^3$ component of Formula I, or reducing an *N*-oxide of the $R^3$ component to the corresponding amine,

(g) reducing a $R^1$ nitro substituent to a $R^1$ amino substituent or alkylating or acylating a $R^1$ amino substituent or alkylating a $R^1$ hydroxy substituent or dealkylating a $R^1$ alkoxy substitutent or debenzylating a $R^1$ benzyloxy substituent to the corresponding compound of Formula I,

(h) hydrogenating in positions 3a, 4, 5 and 6 a compound of the Formula XIV

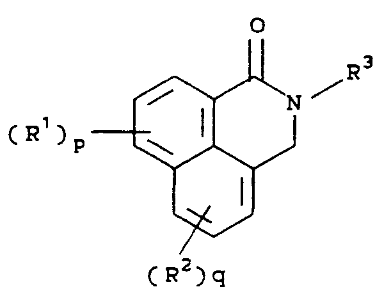

XIV

wherein $R^1$, $R^2$, $R^3$, p and q have the above meanings,

(i) separating a mixture of isomers or diastereomers of a compound of Formula I into a single isomer or diastereomer, or

(j) conducting any of steps (a) through (i) with optically active reactants.

In the formylation step, the compounds of Formula I are prepared by the reaction sequence shown below in Reaction Scheme I.

REACTION SCHEME I

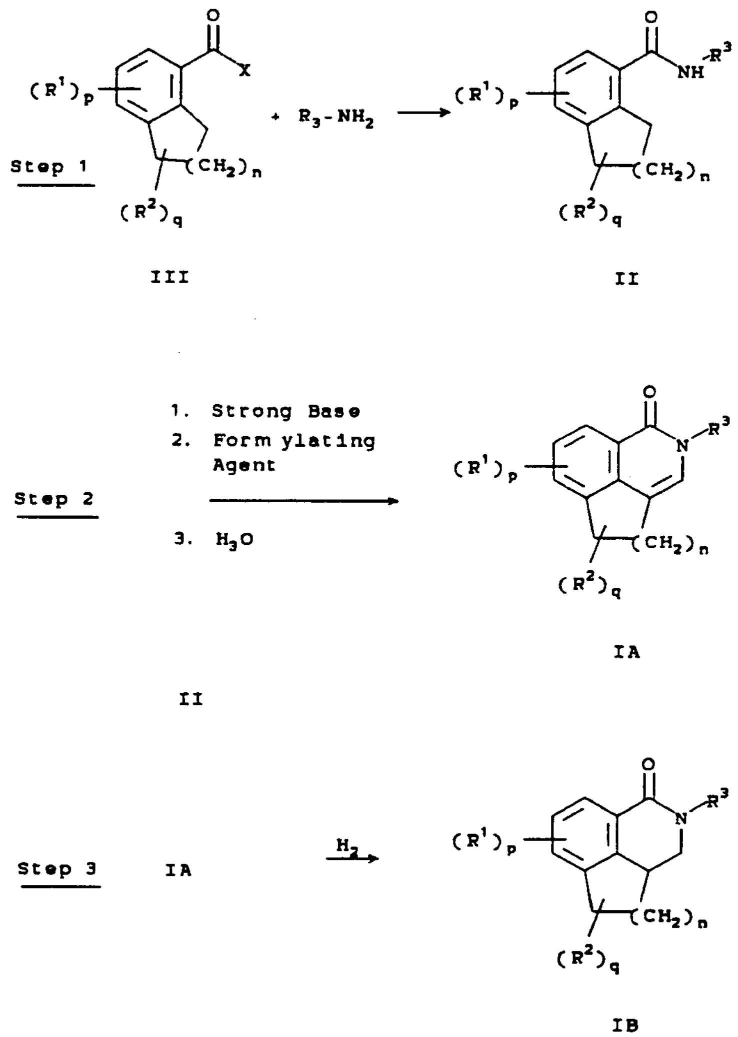


wherein

X = OH, -OR (R = alkyl) or halogen; and

n, p, q, $R^1$, $R^2$ and $R^3$ are as broadly defined above in the Summary of the Invention.

## Step 1

In step 1 of the process of this invention, the fused-ring bicyclic acid, ester or halide of Formula III is reacted with an appropriate amine to form the corresponding amide of Formula II.

In general compounds of Formula III and the amines of Formula $R^3NH_2$ are known in the art, are commercially available or are prepared in accordance with methods available to those of ordinary skill in the art. For example, the compounds of Formula III where X is OH, p is 1, $R^1$ is methoxy (particularly meta to the acid), q is 0 and n is 1 or 2 have been reported by Lowenthal, H.J. and Schatzmiller, S., J. Chem. Soc. Perkin Trans. I 1976; 944. Unsubstituted compounds (where p and q are both 0, and n is 1, 2 or 3) are readily available or may be prepared in accordance with methods known in the art.

Other starting materials that are useful for preparing compounds of the invention are commercially available 1-cyano-4-alkoxynaphthalenes or 1-cyano-2-alkoxynaphthalenes which can be hydrolyzed and reduced to the corresponding starting acid of Formula III where X is OH, $R^1$ is 4-alkoxy or 2-alkoxy, q is 0

and n is 2. Halogen-substituted tetralones are well known and are prepared from *o*-halo-$\gamma$-phenylbutyric acids. All these tetralones can be reduced with alkali boranates such as sodium or lithium boranate to the appropriate alcohol (Formula X of Reaction Scheme II), converted to an acid and reacted with the $R^3NH_2$ compound as a lactone to form an amide of Formula II according to Reaction Scheme II below. Amines of formula $R^3NH_2$ that are useful in this step include those where $R^3$ is defined in the Summary of the Invention section of this application. Particularly useful are the amines where $R^3$ is one of the following radicals:

1-azabicyclo[2.2.2]oct-3-yl;
1-azabicyclo[2.2.2]oct-4-yl;
*endo*−9-methyl-9-azabicyclo[3.3.1]non-3-yl;
*endo*-8-methyl-8-azabicyclo[3.2.1]oct-3-yl;
*exo*-8-methyl-8-azabicylo[3.2.1]oct-3-yl; or
*endo*-1-azabicyclo[3.3.1]non-4-yl.

The reaction conditions under which Step 1 is carried out are those conditions that are standard for amide formation. Generally a solution of the amine in an inert organic solvent is reacted under normal conditions such as those set forth in March, J. Advanced Organic Chemistry 1985; 3rd Ed.: 370-376. A particularly useful method is the reaction of the amide as a dimethylaluminum amine ($Me_2AlNHR^3$) with an alkyl ester (Formula III) where X is lower alkoxy, such as ethoxy. Suitable non-reactive, organic solvents (e.g. toluene or dichloroethane) may be used to carry out the reaction at mild conditions such as ambient pressure and less than ambient temperature, preferably temperatures of from about -10°C to about +20°C. The reaction generally is completed within a few hours.

Step 2

In this step the novel, intermediate amide of this invention shown as Formula II is reacted with a formylating agent in the presence of a strong base. The reaction is carried out in a non-reactive ethereal solvent such as diethyl ether, dimethoxyethane or tetrahydrofuran (THF), the last being preferred. The formylating agent useful for this reaction is any compound that achieves reaction of the amide of Formula II with the formyl group (-CH=O), particularly a dialkylformamide, such as dimethyl formamide (DMF), diethyl formamide, etc., a N-aryl-N-alkylformamide, such as N-phenyl-N-methylformamide, etc. The formylating agent is generally used in molar excess relative to the amide II, for example at a ratio of about 1.1 to about 5.0, with 1.5 to 2.5 being preferred. The strong base useful in this reaction is one that aids the progression of the reaction and can be any appropriate alkyllithium or Grignard reagent. *n*-Butyllithium is particularly useful because of its availability. In general, the reaction takes place under an inert atmosphere (e.g. argon) to prevent the oxidation of the alkyllithium and at a temperature range of about -70°C to ambient temperature. Preferably the temperature is about -20°C to about 0°C, as the reduced temperature is thought to stabilize the intermediate anions formed in this step.

Step 3

In this step the double bond (represented by the dashed line in Formula I and specifically shown in Formula IA) is reduced by hydrogenation. This reaction takes place under standard hydrogenation conditions using a standard hydrogenation catalyst under pressure varying from about atmospheric to about 2000 pounds per square inch (psi) and temperatures ranging from about ambient temperature to about 100°C. The hydrogenation takes place in a suitable polar, organic solvent such as ethanol, DMF, acetic acid, ethyl acetate, tetrahydrofuran, toluene, and the like.

While a standard catalyst (e.g. rhodium on alumina, etc.) may be used, particularly useful catalysts are 20% palladium hydroxide on carbon, l0% palladium on carbon, Pearlman's catalyst from Aldrich (50%$H_2O$ ~ 20% palladium content), palladium/$BaSO_4$. The reduction will take place over a few hours to two or more days depending on the catalyst used, pressure, solvent and temperature. For example using acetic acid with 70% perchloric acid and 20% palladium hydroxide on carbon, a compound can be fully reduced in about 24 hours at about 50 psi and about 85°C.

The compound to be reduced can be used as the free base or in the form of a salt selected from the acid addition salts previously described, in particular the hydrochloride, hydrobromide, camphorsulfonate, acetate, etc. If an optically active acid is used this would frequently influence the preferential formation of one isomer.

Preparation of Isomers

From the Formula (I) it is apparent that some of the compounds of the invention may have at least one or two asymmetric carbon atoms (chiral centers). If the dashed line between carbon atoms 3 and 3a denotes a bond, the compounds of Formula I may have one asymmetric carbon atom in the $R^3$ substituent, i.e., positions 3' or 4' (which are attached to the N-atom denoted as ring atom 2).

Some $R^3$ substituents, for example, the 1-azabicyclo [2.2.2]oct-4-yl, endo-8-methyl-8-azabicyclo[3.2.1] oct-3-yl, exo-8-methyl-8-azabicyclo[3.2.1]oct-3-yl and the endo-9-methyl-9-azabicyclo[3.3.1]non-3-yl substituents have no asymmetric carbon atom (center of chirality). Therefore, the compounds of Formula I containing an achiral $R^3$ substituent and a double bond between carbon atoms 3 and 3a are achiral compounds.

If the dashed line between carbon atoms 3 and 3a represents two hydrogen atoms, the compounds of Formula I have at least one asymmetric carbon atom, i.e. the carbon atom numbered 3a. If $R^3$ is a chiral substituent the compounds of Formula I have two asymmetric carbon atoms.

For the compounds of Formula I which have one asymmetric carbon atom, two enantiomeric forms exist, the (R)- and (S)- form as determined by the rules of Cahn et al.

A number of methods suitable for the resolution of enantiomers can be used but the preferred methods depend on the preparation of diastereomeric compounds derived from the enantiomers. While the resolution can be achieved with covalent diastereomeric compounds derived from the compounds of Formula I and diastereomeric complexes, the preferred diastereomeric compounds are dissociable. In general, the covalent diastereomers are separated by chromatography but preferred are separation/resolution techniques depending on differences in solubility.

In a preferred method the compounds of Formula I with one asymmetric carbon atom are separated by the formation of crystalline diastereomeric salts between the racemic substrate (R, S) and an optically active acid. Examples of suitable resolving agents which form dissociable salts with the enantiomers of formula I are tartaric acid, o-nitrotartranilic acid, mandelic acid, malic acid, 2-phenoxypropionic acid, hydratropic acid and 2-arylpropionic acids in general, or camphorsulfonic acid. Alternatively, selective crystallization, direct crystallization or chromotography can be used. Specifics of the resolution techniques applicable to the preparation of enantiomers of the Formula I are described in Jean Jacques, André Collet, Samuel H. Wilen, Enantiomers, Racemates and Resolutions, John Wiley & Sons, Inc. (1981).

The compounds of Formula I which have two asymmetric carbon atoms occur as diastereomeric compounds with a total of four isomers, since the first asymmetric carbon atom may be R or S, and so may be the second. For example the (3aR,3'R) molecule is the mirror image of the (3aS,3'S) compound, i.e., they are enantiomers. The (3aS,3'R) compound in turn is the enantiomer of the (3aR,3'S) compound. The two pairs of enantiomers stand in the relationship to each other of diastereomers, i.e., they are nonenantiomeric pairs in a set. As enantiomers the (3aR,3'R) compound and the (3aS,3'S) compound have identical physical properties except that they rotate the plane of polarized light in equal amounts in opposite directions. In addition, they react at different rates with other optically active compounds.

The properties of the enantiomers of one diastereomeric pair, however, are not identical with those of the other diastereomeric pair. In other words, the (3aS,3'S) compound has different physical properties from the (3aR,3'S) and the (3aS,3'R) compound. They have different melting points, boiling points, solubilities, reactivity and other properties.

Since the nonenantiomeric pairs that make out the diastereomers have different melting points, boiling points, and solubilities, the pairs can be easily separated by conventional means such as salt formation that allows to apply crystallization techniques based upon differences in solubility. However, the free bases or their salts that form diastereomers can also be separated by chromotographic techniques. Since the diastereomers have different physical properties, no optically active auxillary reagents such as resolving agents need to be employed. For salt formation all the salt-forming acids described above, as long as they form crystalline salts, may be used for the separation techniques. A particular advantage of chromatographic resolutions is that they provide both diastereomers generally in a state of high purity. Every type of preparative chromatography can be used for the purpose of such diastereomer separation (gravity column, thin-layer, dry-column and high- and medium-pressure liquid chromatography). Specifics of the applicable methodology are described in Jean Jacques, André Collet, Samuel H. Wilen, Entiomers, Racemates, and Resolutions, John Wiley & Sons, Inc. (1981), Chapter 5.

After separation of the two pairs of enantiomers each pair can be resolved into its enantiomers by the methods generally used for the separation of enantiomers, e.g., resolution by direct crystallization which depends on differences in the rates of crystallization of the enantiomers in a supersaturated solution with respect to the racemate. Alternatively, resolving agents and fractional crystallization can be employed as

described for the compounds of Formula I with one asymmetric carbon atom.

Alternatively, the compounds of the invention may be prepared using optically active reactants. For example, using (R)-or (S)-amines of the Formula $R^3NH_2$ (wherein $R^3$ has the above meanings) individual isomers of the Formula II may be prepared which may be converted to individual isomers of Formulae IA or IB. This is shown by Examples 1A(2), 2A for the amides of Formula II and by Examples 4A(2), 5A,C,D for the compounds of Formula IA and by Example 7 for the compounds of Formula IB.

The stereoconfiguration at the chiral centers of the compounds of Formula I can be assigned by circular dichroism, preferably by Single Crystal X-Ray Analysis.

Other Methods of Preparation

One of ordinary skill in the art will also recognize that a compound of Formula I may be prepared as an acid addition salt or as the corresponding free base. If prepared as an acid addition salt, the compound is converted to the free base by treatment with a suitable base such as ammonium hydroxide solution, sodium hydroxide, potassium hydroxide or the like. When converting a free base to an acid addition salt, the compound is reacted with a suitable organic or inorganic acid (described earlier).

It is also understood that compounds of this invention that are the *N*-oxides of compounds of Formula I (the *N*-oxides of the cyclic amine portion of $R^3$) are prepared by means known in the art by reacting a compound of Formula I with oxidizing agents such as pertrifluoroacetic acid, permaleic acid, perbenzoic acid, peracetic acid, *m*-chloroperoxybenzoic acid. With m-chloroperoxybenzoic acid the oxidation is conducted under cooling in an inert, organic solvent such as a halogenated hydrocarbon, e.g. dichloromethane. For this oxidation to take place effectively, the compound of Formula I is preferably in the free base form.

The N-oxides of the compounds of Formula I can also be reduced by methods known in the art. A number of reducing agents are suitable for this purpose, specifically sulfur dioxide, sulfur itself, triaryl phosphines such as triphenyl phosphine, alkali boranates such as lithium or sodium boranate, or phosphorous trichloride or tribromide. The reaction will be conducted at a temperature between 0 and 80 °C, with gradual raising of the temperature and the reaction mixture is occasionally shaken. As some of the N-oxides have a low melting point the reduction can be conducted without an additional solvent. If a solvent is being used then the following solvents are preferred: acetonitrite, ethanol, or aqueous dioxane.

Because of the hazardous nature of many of the reducing agents or of the reaction products, the preparation should be conducted in a closed system to avoid exposure to irritating fumes.

The condensation of the compounds of the formula $R^3L$ wherein $R^3$ has the above meanings and L is a leaving group (halogen, mesyloxy, benzenesulfonyloxy, ethanesulfonyloxy or tosyloxy) with a compound of the Formula XIII is carried out under the usual amide alkylating conditions. The amides of Formula XIII are very weak bases. In order to activate the amides, they must first be converted to their anions in order to increase their reactivity. This is effected with strong bases such as sodium, preferably sodium hydride but also alkyl alkali compounds such as butyl lithium, and by adding the alkylating agent $R^3L$. Because the alkylation involves the release of the acid HL, one usually employs the strong base in stoichiometric excess. The alkylation is generally carried out in an inert solvent at a temperature between 20 and 100 °C. The following inert solvents are employed: N, N-dialkylformamides such as N,N-dimethylformamide or tetahydrofuran.

Alternatively, the alkylation can be carried out by phase-transfer catalysis. The reaction can be carried out in a conventional catalytic two-phase system comprising a concentrated alkali hydroxide solution as the aqueous phase and a non-aqueous phase comprising an inert solvent for the product. The reaction is effected with an alkylating agent $R^3Br$ used in 10 to 50% excess added slowly to the system containing a phase-transfer catalyst such as tetra-n-butyl-aminium hydrogen sulfate. The liquid-liquid two-phase system can be replaced with a solid-liquid system comprising powdered alkali hydroxide/alkali carbonate suspended in an inert solvent in the presence of a phase-transfer catalyst such as tetra-n-butylaminium hydrogen sulfate. In both instances the reaction mixture is kept at reflux after addition of the alkylating agent until the reaction is completed. The resultant mixture is cooled to room temperature and the compound of Formula I isolated after separation of the organic phase or by other conventional methods such as extraction. Details of the procedure are described in Synthesis, Communications 1005 (1981).

The amides of Formula XIII are obtained by the formylation method described for step (a) above.

The compounds of Formula I with $R^1$ hydroxy, nitro, or amino substituents can be converted to other $R^1$ substituents in a manner known per se. The $R^1$ nitro group can be converted to an amino group by a number of well-described methods, either the metallic reducing agents such as zinc, tin or iron and acid, catalytic hydrogenation, sulfides such as sodium hydrogensulfide, ammonium sulfide, complexes of aluminum trihydride with aluminum chloride or hydrazine and a catalyst. Specifically useful methods are

described by Joffe, Tartakovskii, and Novikov, Russ. Chem. Rev. 35, 19-32 (1966).

The resulting amino compounds in turn can be alkylated or acylated. The alkylation is conducted with alkyl halides, sulfates, such as dimethyl sulfate or sulfonates. The amino compounds can also be acylated in a manner known per se with acyl halides, anhydrides, esters or with acids. The conditions follow that of the well-known Schotten-Baumann procedure. Frequently aqeuous alkali is added to combine with the liberated hydrogen halide. If acids are being used the reaction is made to proceed in good yield at room temperature with dehydrating agents such as dicyclohexylcarbodiimide or N,N'-carbonyl diimidazole. The acylation can also be carried out as anhydrous coupling of the acid chloride with the free base in a suitable organic solvent (toluene, tetrahydrofuran, ethyl acetate) with good mechanical stirring.

The compounds of Formula I with $R^1$ being alkoxy can also be prepared from the compounds with $R^1$ being a hydroxy group by alkylation. The reaction proceeds quickly with diazo compounds such as diazomethane under mild conditions with high yields. The best method, however, is the Williamson reaction which is carried out in the presence of a base.

The hydrogenation of the compounds of Formula XIV to the compounds of Formula I can be effected by methods known for the partial hydrogenation of aromatic compounds.

The compounds of Formula XIV are prepared by heating a mixture of an optionally substituted 1,8-naphthalic acid anhydride and an amine of the formula $R^3NH_2$ in an inert solvent at 80 to 200 °C until completion, preferably in the presence of a dehydrating agent such as an anhydride or diimide, followed by reduction of the carbonyl group at the 3-position of the benz[de]isoquinoline-1,3-dione derivative with alkali boranates. The hydrogenation of the compounds of Formula XIV to the compounds of Formula I is effected by methods known to convert naphthalene derivatives to tetralines. The compounds of Formula I with $R^1$ being hydroxy are prepared by dealkylation or debenzylation of starting materials of the Formula I with $R^1$ being either alkyloxy or phenylalkyloxy, in particular benzyloxy.

The dealkylation is carried out preferably by acid cleavage of the alkyl aryl ether, preferably with hydrogen iodide or hydrogen bromide. Other suitable cleaving agents are Lewis acids such $BF_3$, $BCl_3$, $BBr_3$ or $AlCl_3$ or anhydrous sulfonic acids or Grignard reagents. If HBr or HJ are used the reaction is generally carried out with the acid addition salt of the alkyl aryl ether using an excess of cleaving agent without a solvent at elevated temperatures, i.e., 40 °C to the boiling point of the reaction mixture, preferably 60 to 95 °C.

The debenzylation is carried out around room temperature under hydrogen with a palladium, platinum or rhodium catalyst with the benzyl aryl ether in solution in an inert solvent. Frequently the catalyst is removed by filtration for recovery and regeneration.

Accordingly, therapeutically active compounds of this invention are especially prepared by

(1) reacting an amide of Formula II with a formylating agent in the presence of a strong base to form a compound of Formula IA,

(2) hydrogenating a compound of Formula IA to form a compound of Formula IB,

(3) converting an acid addition salt of a compound of Formula I to the corresponding free base,

(4) converting a free base of a compound of Formula I to the corresponding pharmaceutically acceptable salt,

(5) oxidizing a compound of Formula I to form the corresponding *N*-oxide of the $R^3$ component of Formula I, or

(6) separating a mixture of isomers of a compound of Formula I into a single isomer.

In any of the above last step processes, a reference to Formula I, IA, IS, or II refers to such Formulae wherein n, p, q, $R^1$, $R^2$, $R^3$, $R^4$, $R^5$, u, x, y, and z are as defined in their broadest definitions set forth in the Summary of the Invention, with the processes applying particularly well to the presently preferred embodiments.

## PREPARATION 1

A. This preparation presents a generic description of a method for preparing compounds of Formula II (particularly where q is 0), which compounds are useful as intermediates for the preparation of compounds of Formula I of this invention. In general, the process involves three steps.

REACTION SCHEME II

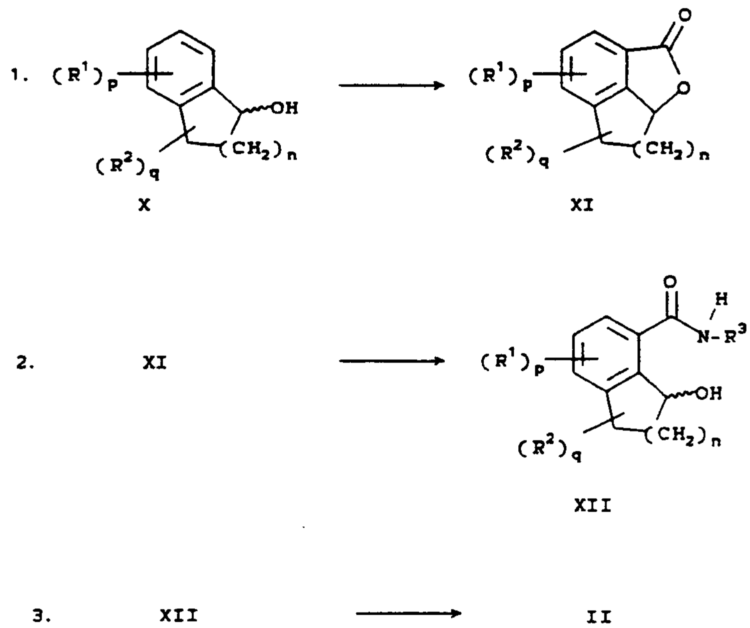
1. X XI

2. XI XII

3. XII II

While this 3-step process may be used to prepare compounds of Formula II (and thus Formula I) where p, n, $R^1$ and $R^3$ are broadly defined, it is particularly useful for preparing compounds of Formula II where n is 3 and p is 0.

In Step 1, an alcohol of Formula X is reacted with with a strong base such as an alkyllithium (e.g. *n*-butyllithium) at elevated temperatures to form an intermediate anion. The reaction takes place in a suitable, inert organic solvent such as an alkane, e.g. hexane generally at reflux temperature for a period of time sufficient to form the intermediate. Then carbon dioxide is bubbled through to complete the reaction (about 5 hours) and form the lactone of Formula XI.

In step 2, the resulting lactone is reacted with an amine of the Formula $R^3NH_2$ under conditions similar to those discussed hereinbefore regarding amide formation from an ester of Formula III in Reaction Scheme I.

In step 3, the resulting hydroxy amide is reduced to form a compound of Formula II under standard reduction (hydrogenation) conditions using a standard reducing catalyst such as 20% palladium hydroxide on carbon.

B. 2,6,7,8,9,9a-HEXAHYDROCYCLOHEPT[*cd*]ISOBENZOFURAN-2-ONE (A compound of Formula XI wherein n = 3 and p = 0).

(1) To a solution of 5,6,7,8-tetrahydro-9*H*-benzocyclohepten-9-ol (4.03 g, 31.9 mmol) in hexane (100 ml) heated under reflux was added dropwise during a 5 minute period, a 2.5 molar solution of *n*-butyllithum in hexane (32 ml, 80.0 mmol). After being heated under reflux for 20 hours, the stirred mixture was cooled to 10 ° C and dry carbon dioxide was bubbled through for 5 h, during which time a white precipitate separated. The reaction mixture was diluted with water (100 ml) and extracted with ethyl acetate. The aqueous solution was adjusted to pH 2.0 with concentrated hydrochloric acid while being stirred in an ice-water bath. The resulting precipitate was filtered and recrystallized from hexane to afford 2,6,7,8,9,9a-hexahydrocyclohept[*cd*]isobenzofuran-2-one (2.63 g), m.p. 84-85 ° C.

(2)-(3) For a further exemplification of these steps see Example 3.

EXAMPLE 1

PREPARATION OF COMPOUNDS OF FORMULA II WHEREIN n IS 1.

A. *N*-(1-AZABICYCLO[2.2.2]OCT-3-YL)-4-INDANCARBOXAMIDE (A compound of Formula II wherein n = 1, p = q = 0, and $R^3$ = 1-azabicyclo[2.2.2]oct-3-yl)

From ethyl 4-indancarboxylate (Formula III wherein x = $OC_2H_5$; Reaction Scheme I, Step 1)

(1) A solution of (*RS*)-3-amino-1-azabicyclo[2.2.2]octane (1.51 g, 12 mmol) in toluene (20 ml) was added dropwise to a stirred solution of trimethylaluminum (12 mmol) in toluene (6 ml), so that the temperature did not exceed 10°C. The mixture was stirred for 30 minutes, and a solution of ethyl 4-indancarboxylate (2.16 g, 11.3 mmol) in toluene (20 ml) was gradually added. The reaction mixture was heated under reflux for 16 hours, then cooled to room temperature. The reaction mixture was added at 0° C to aqueous hydrochloric acid (10%, 20 ml). After separation of the layers, the aqueous layer was made basic with 10 *N* aqueous sodium hydroxide and extracted with ethyl acetate. The organic layer was dried with anhydrous potassium carbonate, filtered and evaporated to afford 2.42 g (79%) of a white solid. A sample recrystallized from ethyl acetate gave (*RS*)-*N*-(1-azabicyclo[2.2.2]oct-3-yl)-4-indancarboxamide, m.p. 158-158.5°C. Anal.: Calcd. for $C_{17}H_{22}N_2O$: C, 75.52; H, 8:20; N, 10.36. Found: C, 75.95; H, 8.22; N, 10.50.

(2) By following the above procedure of Part A(1), but substituting (*S*)- or (*R*)-3-amino-1-azabicyclo-[2.2.2]octane for the (*RS*) mixture, one obtains (*S*)-*N*-(1-azabicyclo[2.2.2]oct-3-yl)-4-indancarboxamide (60% yield), m.p. 159-160°C; $[\alpha]_D^{25}$ -47.5° (*c* 0.4, $CHCl_3$), or(*R*)-*N*-(1-azabicyclo[2.2.2]oct-3-yl)-4-indancarboxamide.

B. Other 4-indancarboxamides of Formula II where n is 1 and $R^3$ is another substitutent.

By following the procedure of part A(1) of this example but changing

(*RS*)-3-amino-1-azabicyclo[2.2.2]octane to

4-amino-1-azabicyclo[2.2.2]octane;

*endo*-3-amino-9-methyl-9-azabicyclo[3.3.1]nonane;

*endo*-3-amino-8-methyl-8-azabicyclo[3.2.1]octane;

*exo*-3-amino-8-methyl-8-azabicyclo[3.2.1]octane; or

*endo*-4-amino-1-azabicyclo[3.3.1]nonane;

the following compounds of Formula (II) are respectively prepared:

*N*-(1-azabicyclo[2.2.2]oct-4-yl)-4-indancarboxamide;

*N-(endo*-9-methyl-9-azabicyclo[3.3.1]non-3-yl)-4-indancarboxamide;

*N*-(*endo*-8-methyl-8-azabicyclo[3.2.1]oct-3-yl)-4-indancarboxamide;

*N*-(*exo*-8-methyl-8-azabicyclo[3.2.1]oct-3-yl)-4-indancarboxamide; or

*N*-(*endo*-1-azabicyclo[3.3.1]non-4-yl)-4-indancarboxamide.

C. 5-Methoxy-*N*-(1-azabicyclo[2.2.2]oct-3-yl)indancarboxamide.

By following the procedure of Part A(1) or A(2) of this Example, but substituting ethyl 5-methoxy-4-indancarboxylate for ethyl 4-indancarboxylate, one obtains (*RS*)-5-methoxy-*N*-(1-azabicyclo[2.2.2]oct-3-yl)-4-indancarboxamide or the respective (*S*)- or (*R*)-isomer.

## EXAMPLE 2

PREPARATION OF COMPOUNDS OF FORMULA II WHERE n IS 2.

A. (*S*)-*N*-(1-AZABICYCLO[2.2.2]OCT-3-YL)-5,6,7,8-TETRAHYDRO-1-NAPHTHALENECARBOXAMIDE (A compound of Formula II wherein n = 2, p = q = 0 and $R^3$ = (*S*)-1-azabicyclo[2.2.2]oct-3-yl)

From 5,6,7,8-tetrahydro-1-naphthalenecarboxylic acid. (Formula III wherein X = OH, Reaction Scheme I, Step 1)

A solution of 5,6,7,8-tetrahydro-1-naphthalenecarboxylic acid (Ofosu-Asante, K. and Stock, L.M., J. Org. Chem. 1986; *51*: 5452) (2.06 g, 11.7 mmol), oxalyl chloride (1 ml, 11.7 mmol), and dimethylformamide (0.1 ml) in dichloromethane (20 ml) was stirred at room temperature for one hour. The mixture was then concentrated under reduced pressure, and the residue was dissolved in dichloromethane (20 ml). The resulting solution was added dropwise at 0°C to a solution of (*S*)-3-amino-1-azabicyclo[2.2.2]-octane (1.48 g, 11.7 mmol) in dichloromethane (20 ml). The solution was stirred at room temperature for 30 minutes, and the solvent was evaporated under vacuum. The residue was dissolved in water and washed with ethyl acetate. The aqueous layer was basified with $NH_4OH$ and extracted with dichloromethane. The dichloromethane was dried with anhydrous potassium carbonate, filtered and then evaporated to afford 2.75 g of white crystals. A sample recrystallized from ethyl acetate/hexane gave (*S*)-*N*-(1-azabicyclo[2.2.2]oct-3-yl)-5,6,7,8-tetrahydro-1-napthalenecarboxamide, m.p. 159-160°C; $[\alpha]_D^{25}$ -42.1° (c 0.65, $CHCl_3$).

B. Other 5,6,7,8-tetrahydro-1-naphthalenecarboxamides of Formula II where n is 2 and $R^3$ is another substituent.

By following the procedure of Part A of this Example 2, but changing
(*S*)-3-amino-1-azabicyclo[2.2.2]octane to
4-amino-1-azabicyclo[2.2.2]octane;
*endo*-3-amino-9-methyl-9-azabicyclo[3.3.1]nonane;
*endo*-3-amino-8-methyl-8-azabicyclo[3.2.1]octane;
*exo*-3-amino-8-methyl-8-azabicyclo[3.2.1]octane; or
*endo*-4-amino-1-azabicyclo[3.3.1]nonane;
the following compounds of Formula (II) are respectively prepared:
*N*-(1-azabicyclo[2.2.2]oct-4-yl)-5,6,7,8-tetrahydro-1-naphthalenecarboxamide;
*N*-(*endo*-9-methyl-9-azabicyclo[3.3.1]non-3-yl)-5,6,7,8-tetrahydro-1-naphthalenecarboxamide;
*N*-(*endo*-8-methyl-8-azabicyclo[3.2.1]oct-3-yl)-5,6,7,8-tetrahydro-1-naphthalene carboxamide;
*N*-(*exo*-8-methyl-8-azabicyclo[3.2.1]oct-3-yl)-5,6,7,8-tetrahydro-1-naphthalenecarboxamide; or
*N*-(*endo*-1-azabicyclo[3.3.1]non-4-yl)-5,6,7,8-tetrahydro-1-naphthalenecarboxamide.

C. 2-Methoxy-, 4-methoxy-, and 4-benzyloxy-*N*-(1-azabicyclo[2.2.2]oct-3-yl)-5,6,7,8-tetrahydro-1-naphthalenecarboxamide.

By following the procedure of Part A of this Example 2, but substituting 2-methoxy-5,6,7,8-tetrahydro-1-naphthalenecarboxylic acid for 5,6,7,8-tetrahydro-1-naphthalenecarboxylic acid, one obtains (*S*)-2-methoxy-*N*-(1-azabicyclo[2.2.2]oct-3-yl)-5,6,7,8-tetrahydro-1-naphthalenecarboxamide, m.p. 270-271 °C, its 4-methoxy-isomer and (*S*)-4-benzyloxy-*N*-(1-azabicyclo[2.2.2]oct-3-yl)-5,6,7,8-tetrahydro-1-naphthalenecarboxamide.

D. 4-Chloro-*N*-(1-azabicyclo[2.2.2]oct-3-yl)-5,6,7,8-tetrahydro-1-naphthalenecarboxamide.

By following the procedure of Part A of this Example 2, but substituting 4-chloro-5,6,7,8-tetrahydro-1-naphthalenecarboxylic acid for 5,6,7,8-tetrahydro-1-naphthalenecarboxylic acid, one obtains (*S*)-4-chloro-*N*(1-azabicyclo[2.2.2]oct-3-yl)-5,6,7,8-tetrahydro-1-naphthalenecarboxamide.

E. *N*-(*ENDO*-9-METHYL-9-AZABICYCLO[3.3.1]NON-3-YL)-5,6,7,8-TETRAHYDRO-1-NAPHTHALENECARBOXAMIDE (A compound of Formula II wherein n = 2, p = q = 0 and $R^3$ = *endo*-9-methyl-9-azabicyclo[3.3.1]non-3-yl)

A solution of 5,6,7,8-tetrahydro-1-napthalenecarboxylic acid (571 mg, 3.24 mmol), oxalyl chloride (0.44 ml, 5.0 mmol), and dimethylformamide (0.05 ml) in dichloromethane (20 ml) was stirred at room temperature for one hour. The mixture was then concentrated under reduced pressure and the residue was dissolved in toluene (10 ml). The resulting solution was added dropwise to a stirred mixture of *endo*-3-amino-9-methyl-9-azabicyclo[3.3.1]nonane (500 mg, 3.24 mmol) and sodium carbonate (700 mg, 6.5 mmol) in 5 ml water and 25 ml toluene. After 2 hours the mixture was diluted with ethyl acetate (100 ml). The layers were separated and the organic layer was dried with anhydrous magnesium sulfate, filtered, and concentrated under reduced pressure to afford 700 mg of white crystals. A sample recrystallized from ethyl acetate gave *N*-(*endo*-9-methyl-9-azabicyclo[3.3.1]non-3-yl)-5,6,7,8-tetrahydro-1-naphthalenecarboxamide, m.p. 166-167 °C.

## EXAMPLE 3

PREPARATION OF COMPOUNDS OF FORMULA II WHERE n IS 3.

A. (*RS*)-*N*-(1-AZABICYCLO[2.2.2]OCT-3-YL)-5,6,7,8-TETRAHYDRO-9*H*-BENZOCYCLOHEPTEN-1-CARBOXAMIDE (A compound of Formula II wherein n = 3, p = q = 0, and $R^3$ = 1-azabicyclo[2.2.2]oct-3-yl; Reaction Scheme II, Steps 2-3)

A solution of (*RS*)-3-amino-1-azabicyclo[2.2.2]octane (1.00 g, 8 mmol) in toluene (20 ml) was added dropwise to a stirred solution of trimethylaluminum (8 mmol) in toluene (10 ml), so that the temperature did not exceed 10 °C. The mixture was stirred for 30 minutes, and a solution of 2,6,7,8,9,9a-hexahydrocyclohept[*cd*]isobenzofuran-2-one (Preparation 1B) (1.25 g, 6.6 mmol) in toluene (10 ml) was gradually added. The reaction mixture was heated under under reflux 0.5 hours and then cooled to ambient temperature. Water was added gradually until a solid was precipitated, and the mixture was filtered. The solid was washed with ethyl acetate and the combined organic layer was evaporated to afford
(*RS*)-*N*-(1-azabicyclo[2.2.2]oct-3-yl)-9*H*-9-hydroxy-5,6,7,8-tetrahydrobenzocyclohepten-1-carboxamide (1.42 g, 68% yield). Crystallization from ethanolic hydrochloric acid afforded the hydrochloride salt, m.p. 239 °C.

Reduction of (*RS*)-*N*-(1-azabicyclo[2.2.2]oct-3-yl)-9*H*-9-hydroxy-5,6,7,8-tetrahydrobenzocyclohepten-1-carboxamide (1.42 g, 4.5 mmol) in ethanolic hydrochloric acid (20 ml) with 20% palladium hydroxide on

carbon (0.5 g) was carried out at 50 psi for 24 hours. The catalyst was removed by filtration and the filtrate was concentrated under reduced pressure. Purification of the product by column chromatography (10% methanol in methylene chloride and 1% ammonium hydroxide) afforded (*RS*)-*N*−(1-azabicyclo-[2.2.2]oct-3-yl)-5,6,7,8-tetrahydro-9*H*-benzocyclohepten-1-carboxamide (0.52 g, 39% yield).

B. Other compounds of Formula II where n is 3 and $R^3$ is another substituent.

By following the procedure of Part A of this Example 3 but changing
(*RS*)-3-amino-1-azabicyclo[2.2.2]octane to
4-amino-1-azabicyclo[2.2.2]octane,
*endo*-3-amino-9-methyl-9-azabicyclo[3.3.1]nonane,
*endo*-3-amino-8-methyl-8-azabicyclo[3.2.1]octane,
*exo*-3-amino-8-methyl-8-azabicyclo[3.2.1]octane, or
*endo*-4-amino-1-azabicyclo[3.3.1]nonane,
the following compounds of Formula (II) are respectively prepared:
*N*-(1-azabicyclo[2.2.2]oct-4-yl)-5,6,7,8-tetrahydro-9*H*-benzocyclohepten-1-carboxamide;
*N*-(*endo*-9-methyl-9-azabicyclo[3.3.1]non-3-yl)-5,6,7,8-tetrahydro-9*H*-benzocyclohepten-1-carboxamide;
*N*-(*endo*-8-methyl-9-azabicyclo[3.2.1]oct-3-yl)-5,6,7,8-tetrahydro-9*H*-benzocyclohepten-1-carboxamide;
*N*-(*exo*-8-methyl-9-azabicyclo[3.2.1]oct-3-yl)-5,6,7,8-tetrahydro-9*H*-benzocyclohepten-1-carboxamide; or
*N*-(*endo*-1-azabicyclo[3.3.1]oct-3-yl)-5,6,7,8-tetrahydro-9*H*-benzocyclohepten-1-carboxamide.

EXAMPLE 4

PREPARATION OF COMPOUNDS OF FORMULA I WHERE n IS 1 AND THE DASHED LINE IS A BOND.

A. 2-(1-AZABICYCLO[2.2.2]OCT-3-yl)-1,2,4,5-TETRAHYDROCYCLOPENT[*de*]ISOQUINOLIN-1-ONE (A compound of Formula IA wherein n = 1, p = q = 0, and $R^3$ = 1-azabicyclo[2.2.2]oct-3-yl; Reaction Scheme I, Step 2)

(1) A solution of (*RS*)-*N*-(1-azabicyclo[2.2.2]oct-3-yl)-4-indancarboxamide (Example 1) (2.07 g, 7.7 mmol) in dry tetrahydrofuran (100 ml) at -70 °C was treated with *n*-butyllithium (20 mmol). The reaction mixture was stirred at -10 °C for one hour, cooled to -70 °C, and dimethylformamide (15 mmol) added in one portion. The reaction mixture was allowed to warm to room temperature over 1.5 hours, then cooled to 0 °C and acidified with 10% aqueous hydrochloric acid. The layers were separated, and the aqueous layer was washed with ethyl acetate, then made basic with 10 N aqueous sodium hydroxide and extracted with ethyl acetate. The ethylacetate was dried over anhydrous sodium sulfate, filtered, and evaporated to afford 1.75 g (81% yield) of white crystals. A sample recrystallized from ethyl acetate gave (*RS*)-2-(1-azabicyclo[2.2.2]oct-3-yl)-1,2,4,5-tetrahydrocyclopent-[*de*]isoquinolin-1-one (Compound A), m.p. 146-147 °C. Anal.: Calcd. for $C_{18}H_{20}N_{20}$: C, 77.11; H, 7.19; N, 9.99%. Found: C, 76.93; H, 7.23; N, 9.90%.

The hydrochloride salt monoethanol adduct [Compound A (HCl)] was prepared from ethanol-HCl: m.p. 188-190 °C. Anal.: Calcd. for $C_{18}H_{20}N_2O$•HCl•$C_2H_5OH$: C, 66.19; H, 7.50; N, 7.72%. Found: C, 66.08; H, 7.55; N, 7.66%.

(2) By following the above procedure but substituting the (*S*)-isomer of
*N*−(1-aza-bicyclo[2.2.2]oct-3-yl)-4-indancarboxamide for the (*RS*)-mixture, one obtains
(*S*)-2-(1-azabicyclo[2.2.2]oct-3-yl)-1,2,4,5-tetrahydrocyclopent[*de*]isoquinolin-1-one (Compound B) (50% yield). A sample recrystallized from ethyl acetate had a m.p. 155.5-156 °C;
$[\alpha]_D^{25}$ +47.1 ° (*c* 0.41, $CHCl_3$). Anal.: Calcd. for $C_{18}H_{20}N_2O$: C, 77.11; H, 7.19, N, 9.99%. Found: C, 77.45; H, 7.12; N, 9.84%.

The hydrochloric acid salt [Compound B (HCl)] was prepared from ethanol-HCl: m.p. >285 °C; $[\alpha]_D^{25}$ -12.8 °; Anal.: Calcd. for $C_{18}H_{20}N_{20}$.HCl.0.5 $H_2O$: C, 66.35; H,6.81; N, 8.59%. Found: C, 65.96; H, 6.86; N, 8.33%.

*(R)*-2-(1-azabicyclo[2.2.2]oct-3-yl)-1,2,4,5-tetrahydrocyclopent[*de*]isoquinolin-1-one is similarly prepared as the hydrochloride [Compound W (HCL)]: m.p. >285 °C; $[\alpha]^{25}_D$ + 17.1 ($H_2O$, C O.6).

B. Other 1,2,4,5-tetrahydrocyclopent[*de*]isoquinolin-1-ones of Formula II where n is 1 and $R^3$ is another substituent.

By following the procedure of Part A(1) of this Example 4, but substituting other compounds of Example 1B and 1C for
(*RS*)-*N*−(1-azabicyclo[2.2.2]oct-3-yl)-4-indancarboxamide, one obtains the corresponding compounds of Formula I.

EXAMPLE 5

PREPARATION OF COMPOUNDS OF FORMULA I WHERE n IS 2 AND THE DASHED LINE IS A BOND.

A. (*S*)-2-(1-AZABICYCLO[2.2.2]OCT-3-YL)-2,4,5,6-TETRAHYDRO-1*H*-BENZ[*de*]ISOQUINOLIN-1-ONE (A compound of Formula IA wherein n = 2, p = q = 0, and $R^3$ = 1-azabicyclo[2.2.2]oct-3-yl; Reaction Scheme I, Step 2)

A solution of *n*-butyllithium in hexane (60 mmol) was added dropwise at -70 °C to a solution of (*S*)-*N*-(1-azabicyclo[2.2.2]oct-3-yl)-5,6,7,8-tetrahydro-1-naphthalenecarboxamide (Preparation 1) (7.70 g, 21 mmol) in dry tetrahydrofuran (400 ml). The reaction mixture was stirred at -10 °C for one hour, cooled to -70 °C, and dimethylformamide (100 mmol) added in one portion. The reaction mixture was allowed to warm to room temperature over 1.5 hours, then cooled to 0 °C and acidified with 10% aqueous hydrochloric acid. The layers were separated, and the aqueous layer was washed with ethyl acetate, then made basic with 10 *N* aqueous sodium hydroxide and extracted with ethyl acetate. The ethyl acetate was dried over anhydrous sodium sulfate, filtered, and evaporated to afford 7.58 g (95% yield) of (*S*)-2-(1-azabicyclo[2.2.2]oct-3-yl)-2,4,5,6-tetrahydro-1*H*-benz[*de*]isoquinolin-1-one (Compound C) as white crystals; m.p. 117-118 °C; $[\alpha]_D^{25}$ +43.2 ° (*c* 0.98, $CHCl_3$).

Crystallization from ethanolic hydrochloric acid afforded 9.75 g of the hydrochloride salt monoethanol adduct [Compound C (HCl)] as white crystals, m.p. >270 °C, $[\alpha]_D^{25}$ -8.4 ° (*c* 2.4, $H_2O$). Anal.: Calcd. for $C_{19}H_{22}N_2O$•HCl•$C_2H_5OH$: C, 66.91; H, 7.75; N, 7.43%. Found: C, 66.77; H, 7.65; N, 7.27%.

Crystallization from isopropanolic HCl provided the unsolvated hydrochloride salt.

Similarly prepared was (*RS*)-2-(1-azabicyclo[2.2.2] oct-3-yl)-2,4,5,6-tetrahydro-1*H*-benz[*de*]-isoquinolin-1-one; m.p. [HCl salt; Compound D (HCl)] 176-177 °C.

Similarly prepared was

(*R*)-2(1-azabicyclo[2.2.2]oct-3-yl)-2,4,5,6-tetrahydro-1*H*-benz[*de*]isoquinolin-1-one; m.p. >275 °C; $[\alpha]_D^{25}$ (HCl salt) [Compound E (HCl)] +6.8 ° (*c* 2, $H_2O$).

B. 2-(*ENDO*-9-METHYL-9-AZABICYCLO[3.3.1]NON-3-YL)-2,4,5,6-TETRAHYDRO-1*H*-BENZ[*de*]-ISOQUINOLIN-1-ONE (A compound of Formula IA wherein n = 2, p = q = 0 and $R^3$ = *endo*-9-methyl-9-azabicyclo[3.3.1]non-3-yl; Reaction Scheme I, Step 2)

(1) A solution of *n*-butyllithium in hexane (5 mmol) was added dropwise at -70 °C to a solution of 2-(*endo*-9-methyl-9-azabicyclo[3.3.1]non-3-yl)-5,6,7,8-tetrahydro-1-napththalenecarboxamide (Example 2B) (0.7 g, 2.24 mmol) in dry tetrahydrofuran (25 ml). The reaction mixture was stirred at -10 °C for one hour, cooled to -70 °C, and dimethylformamide (13 mmol) was added in one portion. The reaction mixture was allowed to warm to room temperature over 1.5 hours, then cooled to 0 °C and acidified with 10% aqueous hydrochloric acid. The layers were separated, and the aqueous layer was washed with ethyl acetate, then made basic with concentrated ammonium hydroxide and extracted with ethyl acetate (100 ml). The ethyl acetate was dried over anhydrous sodium sulfate, filtered, and evaporated to afford 2-(*endo*-9-methyl-9-azabicyclo[3.3.1]non-3-yl)-2,4,5,6-tetrahydro-1*H*-benz[*de*]isoquinolone-1-one. The hydrochloride salt [Compound F (HCl)] was prepared from ethanolic HCl; m.p. 236 °C. Anal.: Calcd. for $C_{21}H_{27}ClN_2O$•$H_2O$: C, 66.92; H, 7.75; N, 7.43%. Found: C 66.45; H, 7.79; N, 7.32%.

(2) By following this procedure but substituting other 1-naphthalenecarboxamides of Example 2B for 2-(*endo*-9-methyl-9-azabicyclo[3.3.1]non-3-yl)-5,6,7,8-tetrahydro-1-naphthalenecarboxamide, the following compounds are prepared:

2-(1-azabicyclo[2.2.2]oct-4-yl)-2,4,5,6-tetrahydro-1*H*-benz[*de*]isoquinolin-1-one, m.p. [HCl salt; Compound G (HCl)] 335-337 °C;2-(*endo*-8-methyl-8-azabicyclo[3.2.1] oct-3-yl)-2,4,5,6-tetrahydro-1H-benz-[*de*]isoquinolin-1-one; m.p. [HCl salt; Compound H (HCl)] 269-270 °;

2-(*exo*-8-methyl-8-azabicyclo[3.2.1]oct-3-yl)-2,4,5,6-tetrahydro-1*H*-benz[*de*]isoquinolin-1-one; m.p. [HCl salt; Compound I (HCl)] >270 °C; and

2-(*endo*-1-azabicyclo[3.3.1]non-4-yl)-2,4,5,6-tetrahydro-1*H*-benz[*de*]isoquinolin-1-one; m.p. [HCl salt; Compound J (HCl)] >360 °C.

C. (*S*)-2-(1-azabicyclo[2.2.2]oct-3-yl)-9-methoxy-2,4,5,6-tetrahydro-1*H*-benz[*de*]isoquinolin-1-one.

By following the procedure of Part A of this Example 5, but substituting the compound prepared in Example 2C, one obtains

(*S*)-2-(1-azabicyclo[2.2.2]oct-3-yl)-9-methoxy-2,4,5,6-tetrahydro-1*H*-benz[*de*]isoquinolin-1-one.

D. (*S*)-2-(1-Azabicyclo[2.2.2]oct-3-yl)-7-chloro-2,4,5,6-tetrahydro-1*H*-benz[*de*]isoquinolin-1-one.

By following the procedure of Part A of this Example 5, but substituting the compound prepared in Example 2D, one obtains

(*S*)-2-(1-azabicyclo[2.2.2]oct-3-yl)-7-chloro-2,4,5,6-tetrahydro-1*H*-benz[*de*]isoquinolin-1-one.

EXAMPLE 6

PREPARATION OF COMPOUNDS OF FORMULA I WHERE n IS 3 AND THE DASHED LINE REPRESENTS A BOND.

A. (*RS*)-2-(1-AZABICYCLO[2.2.2]OCT-3-YL)-1,2,4,5,6,7-HEXAHYDROCYCLOHEPT[*de*]ISOQUINOLIN-1-ONE (A compound of Formula IA wherein n = 3, p = q = 0 and $R^3$ = 1-azabicyclo[2.2.2]oct-3-yl; Reaction Scheme I, Step 2)

A solution of *n*-butyllithium in hexane (2.7 mmol) was added dropwise at -70 °C to a solution of (*RS*)-*N*−(1-azabicyclo[2.2.2]oct-3-yl)-5,6,7,8-tetrahydro-9*H*-benzocycloheptene-1-carboxamide (Example 3A) (0.37 g, 1.2 mmol) in dry tetrahydrofuran (10 ml). The reaction mixture was stirred at -10 °C for one hour, cooled to -70 °C, and dimethylformamide (1.5 mmol) was added in one portion. The reaction mixture was allowed to warm to room temperature over 1.5 hours, then cooled to 0 °C and acidified with 10% aqueous hydrochloric acid. The layers were separated, and the aqueous layer was washed with ethyl acetate, then made basic with aqueous ammonium hydroxide. The ethyl acetate was dried over anhydrous sodium sulfate, filtered, and the solvent was evaporated to afford 0.15 g (40% yield) of (*RS*)-2-(1-azabicyclo-[2.2.2]oct-3-yl)-1,2,4,5,6,7-hexahydrocyclohept[*de*]isoquinolin-1-one as a foam. The hydrochloride salt [Compound K (HCl)] was prepared from ethanol-HCl; m.p. >285 °C.

B. Other compounds of Formula I where n is 3 and $R^3$ is another substituent.

By following the procedure of Part A of this Example 6, but changing
(*RS*)-*N*-(1-azabicyclo[2.2.2]oct-3-yl)-5,6,7,8-tetrahydro-9*H*-benzocycloheptene-1-carboxamide to:
*N*-(1-azabicyclo[2.2.2]oct-4-yl)-5,6,7,8-tetrahydro-1-naphthalenecarboxamide;
*N*-(*endo*-9-methyl-9-azabicyclo[3.3.1]non-3-yl)-5,6,7,8-tetrahydro-1-naphthalenecarboxamide;
*N*-(*endo*-8-methyl-8-azabicyclo[3.2.1]oct-3-yl)-5,6,7,8-tetrahydro-1-naphthalenecarboxamide;
*N*-(*exo*-8-methyl-8-azabicyclo[3.2.1]oct-3-yl)-5,6,7,8-tetrahydro-1-naphthalenecarboxamide; or
N-(*endo*-1-azabicyclo[3.3.1]non-4-yl)-5,6,7,8-tetrahydro-1-naphthalenecarboxamide;
one obtains the following compounds:
2-(1-azabicyclo[2.2.2]oct-4-yl))-1,2,4,5,6,7-hexahydrocyclohept[*de*]isoquinolin-1-one;
2-(*endo*-9-methyl-9-azabicyclo[3.3.1]non-3-yl)-1,2,4,5,6,7-hexahydrocyclohept[*de*]isoquinolin-1-one;
2-(*endo*-8-methyl-8-azabicyclo[3.2.1]oct-3-yl)-1,2,4,5,6,7-hexahydrocyclohept[*de*]isoquinolin-1-one;
2-(*exo*-8-methyl-8-azabicyclo[3.2.1]oct-3-yl)-1,2,4,5,6,7-hexahydrocyclohept[*de*]isoquinolin-1-one; or
2-(*endo*-1-azabicyclo[3.3.1]non-3-yl)-1,2,4,5,6,7-hexahydrocyclohept[*de*]isoquinolin-1-one.

EXAMPLE 7

PREPARATION OF COMPOUNDS OF FORMULA I WHERE n IS 1, 2 OR 3 AND THE DASHED LINE REPRESENTS 2 HYDROGEN ATOMS.

A. (*S*)-2-(1-AZABICYCLO[2.2.2]OCT-3-YL)-2,3,3a,4,5,6-HEXAHYDRO-1*H*-BENZ[*de*]ISOQUINOLIN-1-ONE (A compound of Formula IB wherein n = 2, p = q = 0 and $R^3$ = 1-azabicyclo[2.2.2]oct-3-yl; Reaction Scheme I, Step 3)

The reduction of 0.32g (1.1 mmol) of the free base of (*S*)-2-(1-azabicyclo[2.2.2]oct-3-yl)-2,4,5,6-tetrahydrobenz[*de*]isoquinolin-1-one (Compound C of Example 5A) in acetic acid (5 ml) and 3 drops of 70% perchloric acid with 20% palladium hydroxide on carbon (0.1 g) was carried out at 85 °C and 50 psi for 24 hours. The catalyst was removed by filtration and the filtrate was concentrated under reduced pressure. The residue was dissolved in water (10 ml), basified with ammonium hydroxide solution, and extracted with ethyl acetate. The ethyl acetate was dried with anhydrous potassium carbonate, filtered, and evaporated to afford a diastereomeric mixture of (*S*)-2-(1-azabicyclo[2.2.2]oct-3-yl)-2,3,3a,4,5,6-hexahydro-1*H*-benz[*de*]isoquinolin-1-one (0.18 g) as a semisolid. Crystallization from a mixture of ethanolic hydrochloric acid, isopropanol, and ether afforded 0.8 g of the hydrochloride salt [Compound L (HCl)] as white crystals; m.p. >270 °C. Anal.: Calcd. for $C_{19}H_{14}N_2O \cdot HCl \cdot 0.25\ H_2O$: C, 67.64; H, 7.62; N, 8.30%. Found: C, 67.38; H, 7.70; N. 8.10%.

B. By substituting other compounds prepared in Examples 4, 5 and 6 for (*S*)-2-(1-azabicyclo[2.2.2]oct-3-yl)-2,4,5,6-tetrahydrobenz[*de*]isoquinolin-1-one, other compounds of Formula I where n is 1, 2 or 3 and the dashed line represents 2 hydrogen atoms are obtained.

C. A solution of 19.7 g (59.5 mmol) of the hydrochloride salt of (*S*)-2-(1-azabicyclo[2.2.2]oct-3-yl)-2,4,5,6-tetrahydro-1H-benz[de]isoquinolin-1-one in 250 ml of acetic acid containing 2 g of 20% $Pd(OH)_2$ was hydrogenated at 60 psi and 80-85 °C for 20 hours. The mixture was filtered and evaporated. The residue

was partitioned between aqueous ammonia and dichloromethane and the organic layer was dried ($Na_2SO_4$) and evaporated. The crude free base mixture was dissolved in 100 ml of ethanol, acidified with ethanolic hydrochloric acid, and treated with ether to precipitate the HCl salts of (3aS,3'S)-2-(1-azabicyclo[2.2.2]-oct-3-yl)-2,3,3a,4,5,6-hexahydro-1H-benz[de]isoquinolin-1-one and the (3aR,3'S)-diastereomer. Two recrystalizations from ethanol afforded the pure HCl salt [Compound M (HCl)] of the (3aS,3'S)-diastereomer, m.p. 296-297 °C, $[\alpha]_D$ -98 (c 0.5 $H_2O$), yield 6 grams). The free base (Compound M) has a m.p. of 87-88 °C, $[\alpha]_D$- 136 °C (c 0.25 chloroform).

The mother liquors from the above crystallizations were combined and chromatographed on silica gel (10% methanol-dichloromethane, 1% ammonia to give an enriched fraction of the [3aR,3'S]-diastereomer. Crystallization from ethyl acetate-hexane gave pure [3aR,3'S]-diastereomer base which was converted to the HCl salt [Compound N (HCl)] from ethanolether, m.p. 270-272 °C; $[\alpha]_D$ + 73 (c 0.2, $H_2O$).

D. In an analogous manner, following the hydrogenation procedure of Section C, the HCl salts of the (3aR,3'R)-diastereomer [Compound O (HCl)] with a m.p.>280 °C, $[\alpha]_D$ + 95° (c 0.2 $H_2O$) and of the (3aS,3'R)-diastereomer [Compound P (HCl)] with a m.p. 275-276 °C, $[\alpha]_D$ -68° (c 0.3, $H_2O$) can be isolated.

E. Using 10% Pd on carbon and Pearlman's catalyst Compound C of Example 5A is hydrogenated in tetrahydrofuran to give Compounds M (m.p. of HCl salt > 295 °C) and N (m.p. of HCl salt 272 °C) in a ratio of about 3:2. Using 10% Pd on carbon Compound C is hydrogenated as the (+) or (-) camphorsulfonic acid salt in ethyl actetate to Compounds L and M in a ratio of about 1:3. Using 10% Pd on carbon Compound C is hydrogenated as the acetate in ethyl acetate to Compounds L and M in a ratio of about 0.85:1. Using 10% Pd on carbon with 61.9% water (Degussa) Compound C is hydrogenated as the free base in toluene to Compounds L and M in a ratio of 2.1:1. Using 5% Pd/$BaSO_4$ Compound C is hydrogenated in ethyl acetate to Compounds L and M in a ratio of 2.7:1.

## EXAMPLE 8

A. (*S*)-2-(1-AZABICYCLO[2.2.2]OCT-3-YL]-2,4,5,6-TETRAHYDROBENZ[*de*]ISOQUINOLIN-1-ONE HYDROCHLORIDE (A compound of Formula I as a pharmaceutically acceptable acid addition salt)

(*S*)-2-(1-azabicyclo[2.2.2]oct-3-yl]-2,4,5,6-tetrahydrobenz[*de*]isoquinolin-1-one is recrystallized from *n*-propanol/hydrochloric acid to form the corresponding hydrochloride salt.

B. Other hydrochloride salts of the free base compounds of Examples 1-7 are similarly prepared.

## EXAMPLE 9

A. (*S*)-2-(1-AZABICYCLO[2.2.2]OCT-3-YL]-2,4,5,6-TETRAHYDROBENZ[*de*]ISOQUINOLIN-1-ONE (A compound of Formula I as a free base)

(*S*)-2-(1-azabicyclo[2.2.2]oct-3-yl]-2,4,5,6-tetrahydrobenz[*de*]isoquinolin-1-one hydrochloride is treated with a molar excess of ethanolic potassium hydroxide to form the corresponding free base (Compound C), m.p. 117-118 °C.

B. Other free base compounds are similarly obtained from the corresponding acid addition salts of the compounds prepared in Example 1-8.

## EXAMPLE 10

(*S*)-2-(1-AZABICYCLO[2.2.2]OCT-3-YL)-2,4,5,6-TETRAHYDRO-1*H*-BENZ[*de*]ISOQUINOLIN-1-ONE *N*-OXIDE (A compound of Formula I as the *N*-oxide)

*m*-Chloroperoxybenzoic acid (0.82g, 4.7 mmol) was added in small portions at 0 °C to a solution of (*S*)-2-(1-azabicyclo[2.2.2]oct-3-yl)-2,4,5,6-tetrahydro-1*H*-benz[*de*]isoquinolin-1-one (Example 5A) (1.16 g, 3.9 mmol) in dichloromethane (50 ml). The reaction mixture was stirred for additional 0.5 hour at 0 °C. The solvent was removed under reduced pressure and the residue was purified by column chromatography (10% methanol in dichloromethane and 1% ammonium hydroxide) to give the *N*-oxide of (*S*)-2-(1-azabicyclo-[2.2.2]oct-3-yl)-2,4,5,6-tetrahydro-1*H*-benz[*de*]isoquinolin-1-one (Compound Q) (0.75 g; 62% yield) as an amorphous solid; m.p. 73-75 °C.

Other *N*-oxides of this invention are prepared by substituting other compounds of Examples 4-6 into the above procedure.

EXAMPLE 11

5-$HT_3$ RECEPTOR SCREENING ASSAY

This example describes an *in vitro* assay for determining the 5-$HT_3$ receptor affinity of the compounds of this invention. The method is essentially the method described by Kilpatrick et al., previously cited, which measures the affinity of a compound for the 5-$HT_3$ receptor of the rat cerebral cortex. Compounds of this invention are screened for affinity at the 5-$HT_3$ receptor of rat cerebral cortex radiolabelled with [$^3$H]-quipazine.

Membranes are prepared from the cerebral cortices of rat brains homogenized in 50 mM Tris buffer (pH 7.4 at 4°C) using a Polytron P10 tissue disrupter (setting 10, 2 x 10 sec bursts). The homogenate is centrifuged at 48,000 x 9 for 12 min and the pellet obtained is washed, by resuspension and centrifugation, three times in homogenizing buffer. The tissue pellets are resuspended in the assay buffer, and are stored under liquid nitrogen until required.

The binding assays are conducted using a Tris-Krebs assay buffer of the following composition (mM): NaCl, 154; KCl, 5.4; $KH_2PO_4$, 1.2; $CaCl_2.2H_2O$, 2.5; $MgCl_2$, 1.0; glucose, 11; Tris, 10. Assays are conducted at 25°C at 7.4 in a final volume of 0.25. Zacopride (1.0 $\mu$M) is used to define the non-specific binding (NSB). 5$HT_3$ receptors present in rat cortical membranes are labelled using 0.3-0.7 nM [$^3$H]quipazine (specific activity 50-66 Ci/mmol; New England Nuclear) in the presence of 0.1 $\mu$M paroxetine to prevent [$^3$H]quipazine binding to 5-HT uptake sites. The rat cortex membranes are incubated with [$^3$H]quipazine in the presence of 10 different concentrations of compound to be tested at concentrations ranging from $1x10^{-12}$ to $1x10^{-4}$ molar. Incubations are conducted for 45 min at 25°C and are terminated by vacuum filtration over Whatman GF/B glass fiber filters using a Brandel 48 well cell harvester. After filtration the filters are washed for 8 sec with 0.1 M NaCl. The filters are pretreated with 0.3% polyethyleneimine 18 hr prior to use in order to reduce filter binding of the radioligand. Radioactivity retained on the filters is determined by liquid scintillation counting.

The concentration of compound producing 50% inhibition of radioligand binding is determined by use of an iterative curve fitting procedure. Affinities are expressed as the negative logarithm of the $IC_{50}$ value ($pIC_{50}$). Compounds of this invention exhibit $pIC_{50}$ values showing 5-$HT_3$ receptor antagonist affinity, e.g. values greater than 6.

EXAMPLE 12

GASTRIC EMPTYING OF TEST MEAL IN RATS

This Example describes an *in vivo* method of determining the gastrointestinal activity of the compounds of the invention. The method is the method described by Droppleman et al., previously cited, which measures gastric emptying of test meal in rats.

The following formulation is used as the test meal administered to the rats. Twenty g of cellulose gum (Hercules Inc., Wilmington, Delaware) is slowly added to 200 ml of cold distilled water while being mixed in a Waring blender at approximately 20,000 rpm. Mixture of the cellulose gum continues (approximately 5 min) until complete dispersion and hydration of the cellulose gum has taken place. Three beef bouillon cubes are dissolved in 100 ml of warm water and then blended into the cellulose solution followed by 16 g of purified casein (Sigma Chemical Co., St. Louis, MO), 8 g of powdered confectioners sugar, 8 g of cornstarch, and 1 g of powdered charcoal. Each ingredient is added slowly and mixed thoroughly resulting in approximately 325 ml of a dark gray to black, homogenous paste. The meal was then refrigerated overnight to allow for trapped air to escape. Prior to the assay the meal is removed from the refrigerator to allow it to warm to room temperature. Once to room temperature, 3 ml of meal is filled into 5 ml disposable syringes for oral dosing of animals, with each animal requiring one filled syringe. Five test meal samples are weighed on an analytical balance and these weights were averaged to find a mean test meal weight that is later used in the calculations of meal emptied.

Mature (170 to 204 g) male Sprague-Dawley rats are deprived of food for 24 hrs with water ad libitum. On the morning of the study each animal is weighed and randomly assigned to treatment groups consisting of ten animals per group. Starting at 0 hr each animal receives either control, test, or reference substance (metoclopramide) by intraperitoneal injection. At time 0.5 hr each animal is orally dosed with 3 ml of test meal. At time 1.5 hr each animal is sacrificed by carbon dioxide inhalation. The stomach is removed by opening each animal's abdomen and carefully clamping and cutting the esophagus and cutting just below the pyloric sphincter of the stomach. Each stomach is placed, taking care not to lose any of the stomach

contents, on a small, previously weighed and correspondingly labeled 7 ml weigh boat and immediately weighed on an analytical balance. Following weighing each stomach is cut open along the lesser curvature of the stomach and was rinsed with tap water and gently blotted dry to remove excess moisture. After reweighing the empty stomach, the difference between the weight of the full stomach and the empty stomach minus the weigh boat weight indicates the amount of of test meal remaining in the stomach, and this value is subtracted from the average weight of 3 ml of test meal to yield the quantity of test meal emptied during the 1.5 hour post injection time period. The mean and standard deviation amount of meal emptied by test and reference groups are compared to the mean and standard deviation of meal emptied by the control group by use of Dunnett's t-test (Statistical Association Journal, December 1955, 1096-1121). Per cent difference from control is also calculated for the treatment groups.

The compounds of this invention when administered intraperitoneally increase gastric emptying of a test meal in rats as shown by the following results:

| Compound | (N) | Mean ± 50 (gm.)[c] | Δ%[c] |
|---|---|---|---|
| control‡ | (25) | 1.77 ± 0.20 | --- |
| N(HCl) | ( 6) | 2.44 ± 0.18* | 22.5 |
| M(HCl) | (10) | 2.32 ± 0.25 | 16.3 |
| C(HCl) | (10) | 2.03 ± 0.36* | 27.2 |
| Q | ( 9) | 2.27 ± 0.20* | 32.7 |
| metoclopramide‡ | (29) | 2.42 ± 0.26* | 37.3 |

* Significance from control; PL 0.05.
‡ Mean results from 3 separate experimental runs.
c Percentage increase from control is calcaluted and statistical analysis is performed using controls particular to each experimental run.

EXAMPLE 13

CISPLATIN-INDUCED EMESIS IN FERRETS

This study shows the effects of compounds of Formula I, given intravenously (iv), on cisplatin-induced emesis in ferrets.

Adult, male, castrated ferrets are allowed food and water ad libitum both prior to and throughout the testing period. Each animal is randomly chosen and anesthetized with a metofane/oxygen mixture, weighed and assigned to one of three test groups. While anesthetized an incision is made along the ventral cervical region approximately two to four centimeters in length. The jugular vein is then isolated and subsequently cannulated with a capped saline filled PE-50 polyethylene tubing. The cannula is exteriorized at the base of the skull and the incision closed with wound clips. The animals are then returned to their cages and allowed to recover from anesthesia before intravenous (iv) administration of either vehicle (1.0 ml/kg) or test compound (1.00 mg/kg). Within 2.0 minutes of the administration of a test compound (iv), an iv dose of cisplatin (10 mg/kg) is given. The animals are then observed for five hrs (post dosing) and emetic responses (i.e., vomiting and/or retching) are recorded. For purposes of this Example and of Example 16, vomiting is defined as the successful evacuation of stomach contents, while a single episode of retching is defined as rapid and successive efforts to vomit (within a one minute time period). At the end of this observation period each animal is euthanised by a lethal barbiturate injection.

Emetic responses are represented as (1) time to onset of emesis, (2) total vomiting episodes and (3) total retching episodes. Means and standard deviations of the test groups are compared to those of the reference groups. Significance is determined by Student's t-test when comparing a single treatment group to the vehicle control or by Dunnett's comparative analysis when more than one treatment group is

compared to a single vehicle.

Intravenously administered compounds of Formula I are anti-emetic in this assay as shown by the following results:

| Treatment | Dose, P.O. (mg/kg) ) | N | Time to Onset | Retching Episodes | Vomiting Episodes |
|---|---|---|---|---|---|
| Vehicle | 1.0 ml/kg | 5 | 33.6±6.9 | 11.0±2.9 | 14.2±5.5 |
| N(HCl) | 0.1 | 6 | 79.7±16.6 | 3.5±2.3 | 3.3±2.7 |
| Vehicle | 1.0 ml/kg | 5 | 33.6±6.9 | 11.0±2.9 | 14.2±5.5 |
| M(HCl) | 0.1 | 5 | 90.0±21.2 | 0.8±1.3 | 0.8±1.3 |
| Vehicle | 1.0 ml/kg | 5 | 54.0±10.9 | 11.4±4.3 | 13.2±7.3 |
| C(HCl) | 0.1 | 6 | 86.5±2.1 | 1.0±1.5 | 0.7±1.0 |
| Vehicle | 1.0 ml/kg | 6 | 50.0±4.0 | 11.7±2.3 | 16.5±5.8 |
| M(HCl) | 0.1 | 6 | 76.5±43.6 | 3.5±3.7 | 5.3±7.2 |
| Vehicle | 1.0 ml/kg | 6 | | 11.8±4.6 | 12.5±4.5 |
| V(HBr) | 0.1 | 5 | | 7.4±3.3 | 6.8±3.4 |
| Vehicle | 1.0 ml/kg | 6 | 37.2±5.0 | 16.5±2.6 | 19.5±3.2 |
| M(HCl) | 0.1 | 6 | N/A | 0.5±1.2 | 0±0 |

Proceeding as in this Example, but administering the test compounds by oral route, the anti-emetic effects of compounds of Formula I may be evaluated. Orally administered compounds of Formula I are anti-emetic in this assay.

EXAMPLE 14

5-$HT_3$ ANTAGONIST ACTIVITY IN RATS (VON BEZOLD-JARISCH REFLEX)

This Example describes an *in vivo* method for determining the 5-$HT_3$ antagonist activity of the compounds of the invention. The method is a modified version of the methods described by Butler et al., Cohen et al., and Fozard, all previously cited, which measure 5-$HT_3$ antagonist in rats; where 2-methyl-5-hydroxytryptamine was used rather than 5-HT itself.

Male Sprague-Dawley rats, 250-380 g are anesthetized with urethane (1.4 g/kg, ip), and cannulated (trachea, left femoral vein and either duodenum or jugular vein). The heart rate is recorded, using Gould ECG/Biotech amplifiers. After at least 30 min equilibration, each rat is titrated (iv) with 2-methyl-5-hydroxytryptamine (2-m-5HT) and a minimal dose that induced a sufficient and consistent bradycardia is chosen.

For an intravenous dose range (potency) study, the rat is challenged with the selected dose of 2-m-5HT every 12 min. A test compound is administered intravenously in increasing doses 5 min before each injection of 2-m-5HT, until the response to 2-m-5HT is blocked. A separate group of rats receiving vehicle is similarly tested.

For duration studies, a single dose of a test compound or vehicle is injected intravenously or intraduodenally to the rat and the rat is challenged with 2-m-5HT at 5, 15, 30, 60, 120, 180, 240, 300 and, in some studies, 360, 420 and 480 min post dose.

For both potency and duration studies heart rate (beats/min) is recorded continuously for the duration of the study. The peak decrease in heart rate evoked by 2-m-5HT is monitored, using a computer. The change in responses to 2-m-5HT before and after administration of the vehicle or the compound is calculated. This is expressed as percent inhibition from the predose value. Data are analyzed using a one-way repeated measures ANOVA and followed by pairwise comparison to vehicle control using Fisher's LSD strategy. From a dose-response curve so constructed, an $ID_{50}$ value is obtained to represent the dose that inhibited

50% of the bradycardic effect induced by 2-m-5HT.

Compounds of this invention are active in this assay. Specifically, Compounds C(HCl), M, M(HCl), N-(HCl) and R(HCl) are equal in activity to Odansetron or more active than Odansetron (ID50 ~ 3.2 mg/kg).

## EXAMPLE 15

### THE ANXIOLYTIC BEHAVIOR MODEL

This Example describes an *in vivo* method for determining the CNS activity, i.e. the anxiolytic activity, of compounds of the invention.

Naive male C5Bl/6J mice, 18-20 g, are kept in groups of 10 mice in quarters controlled for sound, temperature and humidity. Food and water are available ad libitum. The mice are kept on a 12 hr light and 12 hr dark cycle, with lights on at 6:00 a.m. and off at 6:00 p.m. All experiments begin after at least 7 days have lapsed after arrival at the site.

The automated apparatus used for the detection of changes in exploration was obtained from Omni-Tech Electronics Columbus Ohio and was similar to that of Crawley and Goodwin (1980), as described in Kilfoil et al., cited previously. Briefly, the chamber consisted of a plexiglass box (44 x 21 x 21 cm), divided into two chambers by a black plexiglass partition. The partition dividing the two chambers contained a 13 x 5 cm opening through which the animal could easily pass. The dark chamber (42 cm x 21 cm x 30 cm) has clear sides and a white floor. A fluorescent tube light (40 watt) placed above the chambers provides the only illumination. The Digiscan Animal Activity Monitor System RXYZCM16 (Omni-Tech Electronics) is used to record the exploratory activity of animals within the test chambers.

Prior to administration of vehicle ($DDH_2O$) or active compound at doses of 1.0 mg - 10 mg/kg all animals were given 60 min. to acclimatize to the laboratory environment. All animals having received intraperitoneal injections of either active or vehicle are returned to their home cages for a 15 min. pretreatment period. From here, each animal is individually (separately) placed in the center of the light area of one of the test apparatuses and monitored for 10 min. Measurements include time spent in each compartment, general locomotor activity, rears and latency (the time for the mouse to move to the dark chamber when first placed in the center of the lighted area):

| COMPOUND | SHUTTLE ACTIVITY | | LOCOMOTOR ACTIVITY (LIGHT AREA) | | TIME DARK AREA (SEC) | | LATENCY (SEC) | | % ACTIVITY DARK AREA 2* | |
|---|---|---|---|---|---|---|---|---|---|---|
| | Mean ± SD | Δ% | Mean ± SD | Δ% | Mean ± SD | Δ% | Mean ± SD | Δ% | Mean ± SD | Δ% |
| Ondansetron | 121.0±12.4* | 7.5 | 1528± 77.8* | 52.6 | 499.4±14.8* | -12.8 | 6.7±1.5 | 4.7 | 78.8±2.4* | -13.1 |
| E(HCl) | 266.5±43.4* | 95.8 | 1677± 66.9* | 34.6 | 504.8±13.2* | -9.4 | 2.6±0.2 | 4.0 | 78.1±2.3 | -7.5 |
| C (HCl) | 159.8±22.8 | 40.2 | 1534± 72.9* | 41.1 | 452.4±17.9* | -20.1 | 7.6±0.9 | 80.9 | 77.4±2.7* | -12.3 |
| M (HCl) | 59.8± 8.2 | 31.9 | 1844± 54.4* | 57.6 | 454.5±17.4* | -18.2 | 8.1±1.2 | 86.9 | 71.3±2.8 | -18.1 |
| P (HCl) | 148.4±14.8* | 92.7 | 1696±112* | 33.4 | 479.0±12.5* | -12.5 | 7.6±1.1 | 65.2 | 74.4±1.9 | -14.6 |

* Significance from control; $p < 0.05$

Percentage change from control (Δ%) is calculated and statistical analysis is performed using controls particular to each experimental run.

2* Unaltered or decreased % locomotor activity in the dark area is an indication that increased exploratory activity is not the result of an overall increase in locomotor activity but is the reflection of anxiolytic activity.

The two compartment exploratory model of Crawley and Goodwin demonstrates that anxiolytic (antianxiety) compounds of Formula I increase the amount of time spent in the light area, increase the amount of shuttle activity in the light area and either do not affect or increase locomotor activity in the light area.

The above results demonstrate that the compounds of this invention are effective in this assay.

EXAMPLE 16

CISPLATIN-INDUCED EMESIS IN DOGS

The following describes the procedure for determining the intravenous (i.v.) effects of compounds of Formula I on cisplatin-induced emesis in dogs.

Male and female dogs (6-15 kg) are fed one cup of dry dog food. One hour following feeding, cisplatin (cis-diamminedichloroplatinum) is administered i.v. at 3 mg/kg. Sixty minutes after the administration of cisplatin, either vehicle or test compound is injected i.v. at 0.1 ml/kg and 1.0 mg/kg, respectively. The dogs are then observed continuously for a 5 hour period and the emetic responses (i.e., vomiting and/or retching) are recorded.

Emetic responses are represented as (1) time to onset of emesis, (2) total vomiting episodes and (3) total retching episodes. Means and standard deviations of the test groups are compared to those of the reference groups. Significance is determined by Student's t-test when comparing a single treatment group to the vehicle control or by Dunnett's comparative analysis when more than one treatment group is compared to a single vehicle.

Compounds of Formula I exhibit anti-emetic activity in this assay.

EXAMPLE 17

The Mouse Light/Dark Withdrawal Anxiety Test

The following procedure describes a method to determine whether compounds of Formula I affect the anxiety that occurs after abruptly ceasing chronic treatment with drugs of abuse.

Naive male BKW mice (25-30 g) are caged in groups of ten in quarters controlled for sound, temperature and humidity. Food and water are available ad libitum. The mice are kept on a 12 hour light cycle and 12 hour dark cycle, with lights on at 6:00 a.m. and off at 6:00 p.m. All experiments begin at least 7 days after arrival on site.

Anxiolysis is determined by the two-compartment exploratory model of Crawley and Goodwin (see Example 15). Measurements include time spent in the light compartment, locomotion activity (grid crossings/5 min.), rears and latency (the time for the mouse to move to the dark chamber when first placed in the center of the lighted area).

Increased exploratory activity in the lighted area is induced by treating the mice for 14 days with alcohol (8.0 % w/v in drinking water), nicotine (0.1 mg/kg, i.p., twice daily) or cocaine (1.0 mg/kg, i.p., twice daily). Anxiolysis is assessed 1, 3, 7 and 14 days after commencement of the drug regime. The treatment is abruptly ceased and exploratory activity in the lighted area is determined 8, 24 and 48 hours thereafter. Vehicle or test compounds are administered during the withdrawal phase by intraperitoneal injection. Activity is measured as inhibition of the decrease in anxiolytic behavior after the alcohol, cocaine or nicotine treatment is ceased.

Compounds of Formula I decrease the anxiety associated with drug withdrawal in this model as shown by the following results:

| | Time in Dark (%) | Latency (sec.) | Rears/5 Min in Light | Crossings/ 5 Min in Light |
|---|---|---|---|---|
| Control | 58.3±5.9 | 8.0±0.7 | 22.4± 2.4 | 24.4± 2.7 |
| diaz W/D | 70.0±8.0* | 1.8±0.1* | 8.4± 0.9* | 7.8± 0.9* |
| W/D+C(HCl)° | 29.4±3.2*‡ | 27.8±2.9*‡ | 97.8±10.7*‡ | 113.2±13.1*‡ |
| Control | 59.0±6.0 | 9.6±1.5 | 26.0± 2.8 | 33.0± 3.6 |
| nic W/D | 69.7±7.0* | 2.0±0.01* | 9.6± 1.1* | 10.4± 1.4* |
| W/D+C(HCl)° | 29.0±3.0*‡ | 19.7±3.5*‡ | 90.1±10.0*‡ | 100.0±11.0*‡ |
| Control | 58.4±6.0 | 7.3±0.9 | 28.6± 3.2 | 37.0± 4.0 |
| alc W/D | 80.0±8.2* | 2.0±0.3* | 12.3± 1.8* | 14.3± 1.7* |
| W/D+C(HCl)° | 63.7±6.6‡ | 9.6±1.4‡ | 73.5±7.6*‡ | 88.0± 9.1*‡ |
| Contrl | 58.0±5.9 | 9.8±1.5 | 30.2± 3.3 | 34.2± 3.6 |
| coc W/D | 74.5±7.5* | 1.8±0.2* | 8.6± 1.0* | 8.0± 0.9* |
| W/D+C(HCl)° | 25.8±2.7*‡ | 20.0±2.9*‡ | 91.0±10.2*‡ | 117.0±13.0*‡ |

W/D = withdrawal
diaz = diazepam; nic = nicotine; alc = alcohol;
coc = cocaine
* significance from control; $p < 0.01$
‡ significance from W/D; $p < 0.01$
° 1 µ/g/kg of C(HCl) is administered i.p.

EXAMPLE 18

THE MOUSE HABITUATION/COGNITIVE ENHANCEMENT TEST

The following describes a model to determine the cognitive enhancing effects of compounds of Formula I.

Young adult and aged BKW mice are caged in groups of ten in quarters controlled for sound, temperature and humidity. Food and water are available ad libitum. The mice are kept on a 12 hour light cycle and 12 hour dark cycle, with lights on at 6:00 a.m. and off at 6:00 p.m. All experiments begin at least 7 days after arrival on site.

Anxiolysis is determined by the two-compartment exploratory model of Crawley and Goodwin (see Example 15). Measurements include time spent in the dark area, locomotion activity (grid crossings/5 min.), rears and latency (the time for the mouse to move to the dark chamber when first placed in the center of the lighted area).

Mice are exposed to the two-compartment test area over a 4 day period. The young mice habituate to the test area by day 3 and spend less time exploring the lighted area, whereas exploratory activity remains constant for the aged mice through day 4. Vehicle or test compounds are administered to the aged mice by intraperitoneal injection. Activity is measured as a decrease in exploratory activity on days 2, 3, and 4.

Compounds of Formula I enhance cognition in this model as shown by the following results for Compound C(HCl):

| | Time Dark Area (%)[1] | Day 3 Latency (sec.)[2] | Rears[3] | Locomotor Activity[4] |
|---|---|---|---|---|
| young (control) | 83.7±7.8 | 2.7±0.6 | 14.6±1.4 | 26.2±1.9 |
| aged (control) | 32.6±3.1 | 18.0±2.3 | 49.2±4.6 | 58.1±5.7 |
| aged treated | 75.2±6.2 | 4.7±0.6 | 13.8±1.5 | 21.0±1.9 |

1 Percentage of time over a minute period spent in the dark chamber.
2 The time for the mouse to move to the dark chamber when first placed in the center of the lighted chamber.
3 Number of rears/5 min. in the lighted chamber.
4 Number of grid crossings/5 min. in the lighted chamber.

## EXAMPLE 19

Intravenous 1-Month Toxicity Study in Rat

A. The following describes the procedure for determining the effects of chronic intravenous (i.v.) administration of compounds of Formula I in the rat.

Male and female rats are administered i.v. bolus injections of a compound of Formula I at 0.1, 1.0 and 10.0 mg/kg once daily for 1 month. A separate group of rats are treated similarly with vehicle to serve as experimental controls.

During the course of treatment body weight, food intake and clinical observations are recorded weekly. Ophthalmalogic examinations and urinalysis are conducted during the last week of treatment.

After 1 month of treatment all rats are necropsied and clinical chemistry and hematology evaluations of blood samples are performed.

B. The procedure described in Part A was carried out for Compound C(HCl) of Example 5A with the following results:

### Clinical Observations

All rats were clinically normal at all observations.

### Mortality

There were no unscheduled deaths during the study.

The group average body weights of male rats given 0.1 mg/kg/day of Compound C(HCl) were comparable to vehicle-treated males. Male rats given 1 to 10 mg/kg/day of the compound gained slightly less weight (5% to 8%) than control males. In contrast, females in all groups treated with Compound C(HCl) gained 17% to 23% more weight than control females. Differences in weight gain of either sex, when present, were not dose dependent.

### Food Intake

Food intake was comparable among all groups.

### Ophthalmologic Examinations

No treatment-related ophthalmologic changes were present.

Clinical Pathology

There were no treatment-related differences in hematology or clinical chemistry results in animals given 0.1 or 1 mg/kg/day of Compound C(HCl) or in males given 10 mg/kg/day. Female rats given 10 mg/kg/day had slightly lower erythrocyte counts and hemoglobin and hematocrit measurements than controls. Additionally, females in this group had slightly higher sodium levels than controls. No treatment-related changes in urinalysis data were present.

Pathology

No gross or microscopic pathologic hanges of drug-related toxicity were present in male or female rats given 0.1, 1, or 10 mg/kg/day of Compound C(HCl). Liver weights and liver-weight-to-body-weight ratios were higher in females given 10 mg/kg/day than in vehicle-control females.

C. No undue manifestations of chronic toxicity were observed with other compounds of Formula I.

EXAMPLE 20

ACID ADDITION SALTS

To a solution of Compound C of Example 5A (about 0.3 kg) in *i*-propanol is added a solution of HCl gas dissolved in *i*-propanol keeping the temperature below 25°C until no more solid precipitates. The solid is isolated and washed with *i*-propanol. The solid is dissolved in *i*-propanol with the aid of deionized water which is subsequently removed by azeotropic distillation. The solution is cooled and aged for at least 2hrs. The product is isolated, washed with *i*-propanol and dried at 50-75°C under reduced pressure. The product may be upgraded by recrystallization from *i*-propanol using deionized water to aid in dissolution. The mother liquor may be reworked by concentration and recrystallization from *i*-propanol with the aid of deionized water for dissolution. The yield of Compound C(HCl) is 60-95%, and the m.p. conforms to that given in Example 5A.

EXAMPLE 21

PREPARATION OF COMPOUNDS OF FORMULA I WHERE N IS 2, THE DASHED LINE REPRESENTS A BOND AND $R^1$ IS A SUBSTITUENT OTHER THAN HYDROGEN

A. A solution of 830 mg of (*S*)-N-(1-azabicyclo[2.2.2]oct-3-yl)-2-methoxy-5,6,7,8-tetrahydro-1-naphthalene carboxamide (2.6 mmol) in 75 ml tetrahydrofuran is cooled to -50°C and 6.6 mmol of n-butyl lithium in hexane are added. After the addition is complete the reaction mixture is allowed to warm up to -20°C for about 30 minutes while a deep red solution is obtained. The solution is cooled to -40°C and 0.5 ml dimethyl formamide added in one portion, and then allowed to warm to room temperature, and quenched with 10% aqueous hydrochloric acid. The layers are separated and the aqueous layer made basic with ION aqueous sodium hydroxide solution and extracted with ethyl acetate. The ethyl acetate was dried with brine and anhydrous magnesium sulfate and evaporated. After flash chromatography with 5% methanol in dichloromethane and 1% ammonia 80 mg of (*S*)-2-(1-azabicyclo[2.2.2]oct-3-yl)-9-methoxy-2,4,5,6-tetrahydro-1*H*-benz[*de*]isoquinolin-1-one (Compound T) is obtained which is converted to the hydrochloride salt [Compound T (HCl) m.p. 270-271°C, $[\alpha]^{25}{}_D$ -21.1° (C 0.27, $H_2O$): C, 65.74; H, 7.03; N, 7.67. Found: C 65.48; H, 7.04; N, 7.56] in ethanol hydrochloric acid/ether.

B. By following the procedure of Part A of this example with 1.81 grams of the 4-methoxy carboxamide of Part C of Example 2, 1.6 grams of (*S*)-2-(1-azabicyclo [2.2.2]oct-3-yl)-7-methoxy-2,4,5,6-tetrahydro-1*H*-benz[*de*] isoquinolin-1-one are obtained as the hydrochloride salt [Compound X (HCl); m.p. 296/7°C (decomposition)].

C. By following a procedure similar to that of Part A of this example with 1.02 mmol of (*S*)-N-(1-azabicyclo[2.2.2]oct-3-yl)-4-benzyloxy-5,6,7,8-tetrahydro-1-naphthalene-carboxamide and 2.6 mmol of n-butyl-lithium and initial cooling to -70°C and cooling to -60°C before addition of dimethylformamide 110 mg of the 7-benzyloxy analog are obtained as the hydrochloride salt (Compound S (HCl); m.p. 244/5°C.

D. A solution of 100 mg (.31 mmol) of Compound X (HCl) of Part B of this example in 5 ml of 48% HBr is heated to 80-90°C for 16 hours and the completion of the reaction monitored with TLC. The reaction mixture is concentrated under reduced pressure, 5 ml dioxan are added and the mixture concentrated again. The residue obtained is dissolved in 3 ml of hot *i*-propanol, the solution is filtered while hot,

concentrated to 1.5 ml and stored at room temperature. 40 mg of brown crystals of (*S*)-2-(1-azabicyclo-[2.2.2]oct-3-yl)-7-hydroxy-2,4,5,6-tetrahydro-1*H*-benz[*de*]isoquinolin-1-one hydrochloride are obtained as brown crystals and dried in vacuo (Compound V (HBr) m.p. 319-21 °C).

E. Using 500 mg of Compound X (HCl) and the procedure of Part D of this example 180 mg of Compound V (HBr) are obtained: $[\alpha]^{25}_D$ + 41 ° (c 0.02 $H_2O$)

F. A mixture of 50 mg of Compound S (HCl) of Part C of this example and 15 mg of 10% Pd on carbon in 7 ml ethanol are stirred under hydrogen for 15 hours at room temperature. The progress of the reaction is monitored by TLC and shows conversion to Compound V which conformed to the compound obtained in Part D of this example. The catalyst is removed by filtration and the filtrate concentrated under reduced pressure. 17.4 mg of Compound V (HCl) are obtained after recrystallization from ethanol.

EXAMPLE 22

TABLET FORMULATION

5% by wt. of Compound C
69% by wt. of Lactose, spray dried, NF
25% by wt. of Microcrystalline Cellulose, NF
1% by wt. of Magnesium Stearate

**Claims**

**Claims for the following Contracting States : AT, BE, CH, DE, DK, FR, GB, IT, LI, LU, NL, SE**

**1.** A compound of Formula I

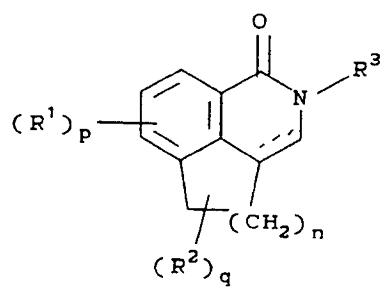

I

in which

the dashed line denotes an optional double bond;
n is 1, 2 or 3;
p is 0, 1, 2 or 3;
q is 0, 1 or 2;
each $R^1$ is independently selected from halogen, hydroxy, lower $C_{1-6}$ alkoxy (optionally substituted with phenyl), lower $C_{1-6}$ alkyl, nitro, amino, amino-carbonyl, (lower $C_{1-6}$ alkyl)amino, di(lower $C_{1-6}$ alkyl)amino, and (lower $C_{1-6}$ alkanoyl)amino;
each $R^2$ is lower $C_{1-6}$ alkyl; and
$R^3$ is selected from

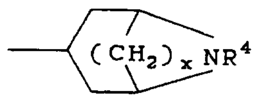

(a)

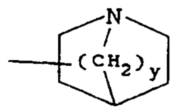

(b)

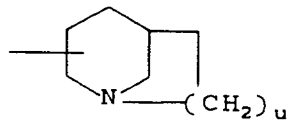

(c)

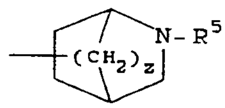

(d)

in which
u, x, y, and z are all independently an integer from 1 to 3; and
$R^4$ and $R^5$ are independently $C_{1-7}$ alkyl, $C_{3-8}$ cycloalkyl, $C_{3-8}$ cycloalkyl-$C_{1-2}$ alkyl, or a group $(CH_2)_tR_6$ where t is 1 or 2 and $R_6$ is thienyl, pyrrolyl or furyl optionally further substituted by one or two substituents selected from $C_{1-6}$ alkyl, $C_{1-6}$ alkoxy, trifluoromethyl or halogen, or is phenyl optionally substituted by one or two substituents selected from $C_{1-4}$ alkoxy, trifluoromethyl, halogen, nitro, carboxy, esterified carboxy, and $C_{1-4}$ alkyl (optionally substituted by hydroxy, $C_{1-4}$ alkoxy, carboxy, esterified carboxy or *in vivo* hydrolyzable acyloxy); or
a pharmaceutically acceptable salt thereof or an *N*-oxide thereof; or
an individual isomer or mixture of isomers thereof.

**2.** The compound of Claim 1 wherein
p is 0, 1 or 2;
n is 1 or 2;
q is 0;
$R^1$ is halogen, lower $C_{1-6}$ alkoxy or amino; and if $R^3$ comprises
$R^4$ and $R^5$, they are each lower $C_{1-6}$ alkyl.

**3.** The compound of Claim 2 wherein n is 1.

**4.** The compound of Claim 3 wherein p is 0, the dashed line represents a double bond and if $R^3$ comprises $R^4$ and $R^5$ they are each methyl.

**5.** The compound of Claim 2 or 4 wherein $R^3$ is
1-azabicyclo[2.2.2]oct-3-yl;
1-azabicyclo[2.2.2]oct-4-yl;
*endo*-9-methyl-9-azabicyclo[3.3.1]non-3-yl;
*endo*-8-methyl-8-azabicyclo[3.2.1]oct-3-yl;
*exo*-8-methyl-8-azabicyclo[3.2.1]oct-3-yl; or
*endo*-1-azabicyclo[3.3.1]non-4-yl.

**6.** The compound of Claim 5 wherein $R^3$ is 1-azabicyclo[2.2.2]oct-3-yl, namely 2-(1-azabicyclo[2.2.2]oct-3-yl)-1,2,4,5-tetrahydro-cyclopent[*de*]isoquinolin-1-one.

**7.** The compound of Claim 6 which is 2-(1-azabicyclo[2.2.2]oct-3-yl)-1,2,4,5-tetrahydro-cyclopenta[*de*]-isoquinolin-1-one hydrochloride.

**8.** The compound of Claim 6 which is (*S*)-2-(1-azabicyclo[2.2.2]oct-3-yl)-1,2,4,5-tetrahydro-cyclopenta[*de*]-isoquinolin-1-one hydrochloride.

**9.** The compound of Claim 5 wherein $R^3$ is 8-methyl-8-azabicyclo[3.2.1]oct-3-yl, namely, 2-(8-methyl-8-azabicyclo[3.2.1]-oct-3-yl)-1,2,4,5-tetrahydro-cyclopent[*de*]isoquinolin-1-one.

**10.** The compound of Claim 9 wherein $R^3$ is *endo*-8-methyl-8-azabicyclo[3.2.1]oct-3-yl, namely, 2-(*endo*-8-methyl-8-azabicyclo[3.2.1]-oct-3-yl)-1,2,4,5-tetrahydro-cyclopent[*de*]isoquinolin-1-one.

**11.** The compound of Claim 2 wherein n is 2.

**12.** The compound of Claim 11 wherein p is 0, the dashed line represents a double bond and if $R^3$ comprises $R^4$ and $R^5$, they are each methyl.

**13.** The compound of Claim 12 wherein $R^3$ is
1-azabicyclo[2.2.2]oct-3-yl;
1-azabicyclo[2.2.2]oct-4-yl;
*endo*-9-methyl-9-azabicyclo[3.3.1]non-3-yl;
*endo*-8-methyl-8-azabicyclo[3.2.1]oct-3-yl;
*exo*-8-methyl-8-azabicylo[3.2.1]oct-3-yl; or
*endo*-1-azabicyclo[3.3.1]non-4-yl.

**14.** The compound of Claim 13 wherein $R^3$ is 1-aza-bicyclo[2.2.2]oct-4-yl, namely 2-(1-azabicyclo[2.2.2]oct-4-yl)-2,4,5,6-tetrahydro-1*H*-benz[*de*]isoquinolin-1-one.

**15.** The compound of Claim 13 wherein $R^3$ is *exo*-8-methyl-8-azabicyclo[3.2.1]oct-3-yl, namely 2-(*exo*-8-methyl-8-azabicyclo[3.2.1]oct-3-yl)-2,4,5,6-tetrahydro-1*H*-benz[*de*]isoquinolin-1-one.

**16.** The compound of Claim 13 wherein $R^3$ is endo-8-methyl-8-azabicyclo[3.2.1]oct-3-yl, namely 2-(endo-8-methyl-8-azabicyclo[3.2.1]oct-3-yl)-2,4,5,6-tetrahydro-1*H*-benz[*de*]isoquinolin-1-one.

**17.** The compound of Claim 13 wherein $R^3$ is 1-azabicyclo[2.2.2]oct-3-yl, namely 2-(1-azabicyclo[2.2.2]oct-3-yl)-2,4,5,6-tetrahydro-1*H*-benz[*de*]isoquinolin-1-one.

**18.** The compound of Claim 17 which is (*S*)-2-(1-azabicyclo[2.2.2]oct-3-yl)-2,4,5,6-tetrahydro-1*H*-benz[*de*]-isoquinolin-1-one hydrochloride.

**19.** The compound of Claim 17 which is (*S*)-2-(1-azabicyclo[2.2.2]oct-3-yl)-2,4,5,6-tetrahydro-1*H*-benz[*de*]-isoquinolin-1-one as the free base.

**20.** The compound of Claim 17 which is (*R*)-2-(1-azabicyclo[2.2.2]oct-3-yl)-2,4,5,6-tetrahydro-1*H*-benz[*de*]-isoquinolin-1-one.

**21.** The compound of Claim 13 wherein $R^3$ is *endo*-9-methyl-9-azabicyclo[3.3.1]non-3-yl, namely 2-(*endo*-9-methyl-9-azabicyclo[3.3.1]non-3-yl)-2,4,5,6-tetrahydro-1*H*-benz[*de*]isoquinolin-1-one.

**22.** The compound of Claim 13 wherein $R^3$ is *endo*-1-azabicyclo[3.3.1]non-4-yl,namely 2-(*endo*-1-azabicyclo[3.3.1]non-4-yl)-2,4,5,6-tetrahydro-1*H*-benz[*de*]isoquinolin-1-one.

**23.** The compound of Claim 2 wherein p is 0, the dashed line represents two hydrogens, and if $R^3$ comprises $R^4$ and $R^5$, they are each methyl.

**24.** The compound of Claim 23 wherein $R^3$ is
1-azabicyclo[2.2.2]oct-3-yl;
1-azabicyclo[2.2.2]oct-4-yl;
*endo*-9-methyl-9-azabicyclo[3.3.1]non-3-yl;
*endo*-8-methyl-8-azabicyclo[3.2.1]oct-3-yl;
*exo*-8-methyl-8-azabicyclo[3.2.1]oct-3-yl; or

*endo*-1-azabicyclo[3.3.1]non-4-yl.

**25.** The compound of Claim 24 wherein n is 1.

**26.** The compound of Claim 24 wherein n is 2.

**27.** The compound of Claim 26 wherein $R^3$ is 1-azabicyclo[2.2.2]oct-3-yl, namely 2-(1-azabicyclo-[2.2.2]oct-3-yl)-2,3,3a,4,5,6-hexahydro-1*H*-benz[*de*]isoquinolin-1-one, in particular the (3aS,3'S) isomer thereof and its hydrochloride salt.

**28.** The compound of Claim 1 wherein
n is 3;
p is 0, 1 or 2;
q is 0;
$R^1$ is halogen, lower $C_{1-6}$ alkoxy or amino; and if $R^3$ comprises
$R^4$ and $R^5$, they are each lower $C_{1-6}$ alkyl.

**29.** The compound of Claim 28 wherein p is 0, the dashed line represents a double bond, and if $R^3$ comprises $R^4$ and $R^5$, they are each methyl.

**30.** The compound of Claim 29 wherein $R^3$ is
1-azabicyclo[2.2.2]oct-3-yl;
1-azabicyclo[2.2.2]oct-4-yl;
*endo*-9-methyl-9-azabicyclo[3.3.1]non-3-yl;
*endo*-8-methyl-8-azabicyclo[3.2.1]oct-3-yl;
*exo*-8-methyl-8-azabicyclo[3.2.1]oct-3-yl; or
*endo*-1-azabicyclo[3.3.1]non-4-yl.

**31.** The compound of Claim 30 wherein $R^3$ is 1-azabicyclo[2.2.2]oct-3-yl, namely (*RS*)-2-(1-azabicyclo-[2.2.2]oct-3-yl)-1,2,4,5,6,7-hexahydrocyclohept[*de*]isoquinolin-1-one.

**32.** The N-oxide of the compound of Claim 1 wherein
p is 0, 1 or 2;
q is 0;
$R^1$ is halogen, lower $C_{1-6}$ alkoxy or amino; and if $R^3$ comprises
$R^4$ and $R^5$, they are each lower $C_{1-6}$ alkyl.

**33.** The compound of Claim 32 wherein p is 0, and if $R^3$ comprises $R^4$ and $R^5$, they are each methyl.

**34.** The compound of Claim 33 wherein n is 2 and $R^3$ is 1-azabicyclo[2.2.2]oct-3-yl.

**35.** A compound according to any of the Claims 1 to 34 for use as a pharmaceutical.

**36.** A pharmaceutical composition comprising a therapeutically effective amount of a compound of Claim 1 to 34, preferably in combination with a pharmaceutically acceptable excipient.

**37.** The use of a compound of Claim 1 to 34 in the manufacture of a medicament for treating a condition chosen from emesis, a gastro-intestinal disorder, CNS disorder, a cardiovascular disorder and pain or a condition in which the $5\text{-}HT_3$ receptor plays a role.

**38.** A process for the preparation of a compound of Formula I

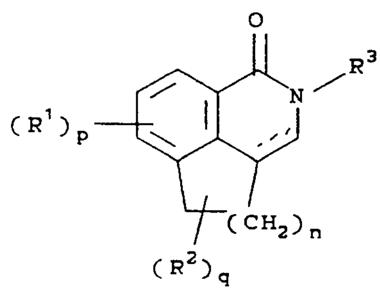

I

in which

n is 1, 2 or 3;

p is 0, 1, 2 or 3;

q is 0,1 or 2;

each $R^1$ is independently selected from halogen, hydroxy, lower $C_{1-6}$ alkoxy (optionally substituted with phenyl), lower $C_{1-6}$ alkyl, nitro, amino, amino carbonyl, (lower $C_{1-6}$ alkyl)amino, di(lower $C_{1-6}$ alkyl)amino, and (lower $C_{1-6}$ alkanoyl)amino;

each $R^2$ is lower $C_{1-6}$ alkyl; and

$R^3$ is selected from

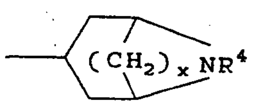

(a)

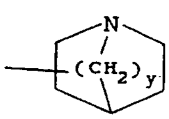

(b)

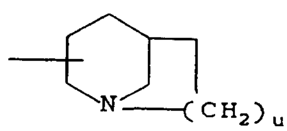

(c)

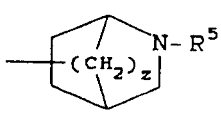

(d)

in which

u, x, y and z are all independently an integer from 1 to 3; and

$R_4$ and $R_5$ are independently $C_{1-7}$ alkyl, $C_{3-8}$ cycloalkyl, $C_{3-8}$ cycloalkyl-$C_{1-2}$ alkyl, or a group $(CH_2)_tR_6$ where t is 1 or 2 and $R_6$ is thienyl, pyrrolyl or furyl optionally further substituted by one or two substituents selected from $C_{1-6}$ alkyl, $C_{1-6}$ alkoxy, trifluoromethyl or halogen, or is phenyl optionally substituted by one or two substituents selected from $C_{1-4}$ alkoxy, trifluoromethyl, halogen, nitro, carboxy, esterified carboxy, and $C_{1-4}$ alkyl (optionally substituted by hydroxy, $C_{1-4}$ alkoxy, carboxy, esterified carboxy or *in vivo* hydrolyzable acyloxy); or a pharmaceutically acceptable salt thereof or an *N*-oxide thereof, or an individual isomer or mixture of isomers thereof, which process comprises one or more of the following steps:

(a) reactively contacting a compound of Formula II

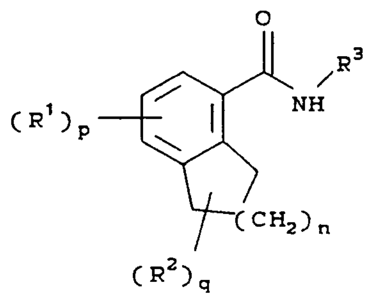

II

in which n, p, q, $R^1$, $R^2$, and $R^3$ are as defined above with a formylating agent in the presence of a strong base to form a compound of Formula I wherein the dashed line is a double bond,
(b) reducing the double bond represented by the dashed line in Formula I by hydrogenation to form a compound of Formula I wherein the dashed line represents 2 hydrogens,
(c) converting a salt of a compound of Formula I to the corresponding free compound,
(d) condensing a compound of the formula $R^3L$, wherein $R^3$ has the above meanings and L is a leaving group with a compound of the Formula XIII,

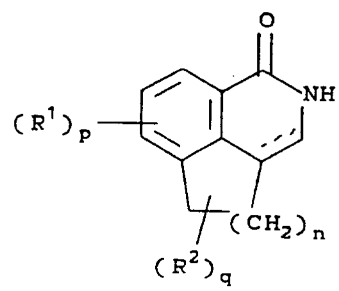

XIII

wherein $R^1$, $R^2$, n, p, q, and the dashed line have the above meanings,
(e) converting a compound of Formula I to the corresponding pharmaceutically acceptable salt,
(f) oxidizing a compound of Formula I to form the corresponding *N*-oxide of the $R^3$ component of Formula I, or reducing an *N*-oxide of the $R^3$ component to the corresponding amine,
(g) reducing a $R^1$ nitro substituent to a $R^1$ amino substituent or alkylating or acylating a $R^1$ amino substituent or alkylating a $R^1$ hydroxy substituent or dealkylating a $R^1$ alkoxy substitutent or debenzylating a $R^1$ benzyloxy substituent to the corresponding compound of Formula I,
(h) hydrogenating in positions 3a, 4, 5 and 6 a compound of the Formula XIV

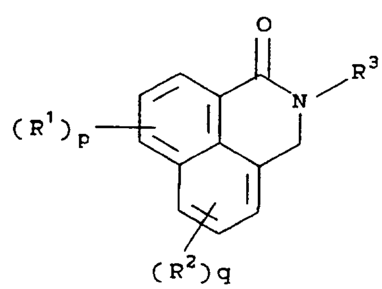

XIV

wherein $R^1$, $R^2$, $R^3$, p and q have the above meanings,
(i) separating a mixture of isomers or diastereomers of a compound of Formula I into a single isomer or a diastereomer, or
(j) conducting any of steps (a) through (i) with optically active reactants.

**39.** A process according to Claim 38 wherein $R^3$ is
1-azabicyclo[2.2.2]oct-3-yl;
1-azabicyclo[2.2.2]oct-4-yl;
*endo*-9-methyl-9-azabicyclo[3.3.1]non-3-yl;
*exo*-8-methyl-8-azabicyclo[3.2.1]oct-3-yl; or
*endo*-1-azabicyclo[3.3.1]non-4-yl.

**40.** A compound represented by the formula

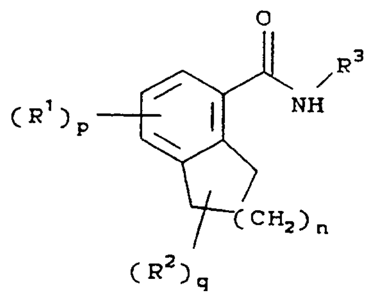

II

wherein
n is 1, 2 or 3;
p is 0, 1, 2 or 3;
q is 0, 1 or 2;
each $R^1$ is independently selected from halogen, hydroxy, lower $C_{1-6}$ alkoxy (optionally substituted with phenyl), lower $C_{1-6}$ alkyl, nitro, amino, amino carbonyl, (lower $C_{1-6}$ alkyl)amino, di(lower $C_{1-6}$ alkyl)amino, and (amino $C_{1-6}$ alkanoyl)amino;
each $R^2$ is lower $C_{1-6}$ alkyl; and
$R^3$ is selected from

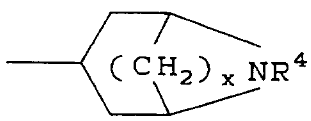

(a)

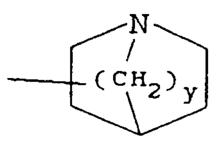

(b)

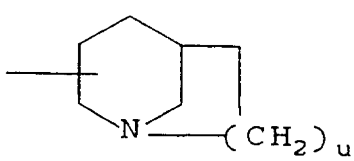

(c)

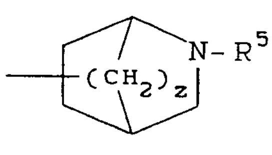

(d)

in which
u, x, y and z are all independently an integer from 1 to 3; and

$R^4$ and $R^5$ are independently $C_{1-7}$ alkyl, $C_{3-8}$ cycloalkyl, $C_{3-8}$ cycloalkyl-$C_{1-2}$ alkyl, or a group $(CH_2)_tR_6$ where t is 1 or 2 and $R_6$ is thienyl, pyrrolyl or furyl optionally further substituted by one or two substituents selected from $C_{1-6}$ alkyl, $C_{1-6}$ alkoxy, trifluoromethyl or halogen, or is phenyl optionally substituted by one or two substituents selected from $C_{1-4}$ alkoxy, trifluoromethyl, halogen, nitro, carboxy, esterified carboxy, and $C_{1-4}$ alkyl (optionally substituted by hydroxy, $C_{1-4}$ alkoxy, carboxy, esterified carboxy or *in vivo* hydrolyzable acyloxy);
or an individual isomer or mixture of isomers thereof, or salt thereof.

**Claims for the following Contracting States : ES, GR**

**1.** A process for the preparation of a compound of Formula I

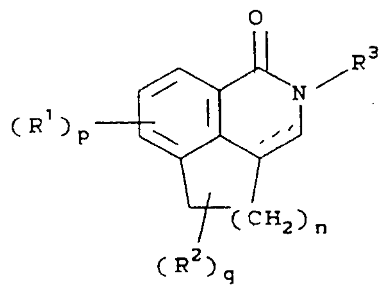

I

in which
the dashed line denotes an optional double bond;
n is 1, 2 or 3;
p is 0, 1, 2 or 3;
q is 0, 1 or 2;
each $R^1$ is independently selected from halogen, hydroxy, lower $C_{1-6}$ alkoxy (optionally substituted with phenyl), lower $C_{1-6}$ alkyl, nitro, amino, amino-carbonyl, (lower $C_{1-6}$ alkyl)amino, di(lower $C_{1-6}$ alkyl)amino, and (lower $C_{1-6}$ alkanoyl)amino;
each $R^2$ is lower $C_{1-6}$ alkyl; and
$R^3$ is selected from

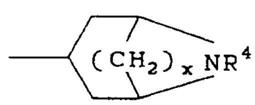

(a)

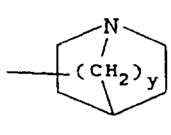

(b)

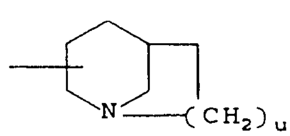

(c)

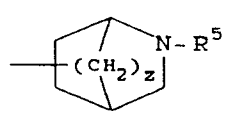

(d)

in which
u, x, y, and z are all independently an integer from 1 to 3; and

$R^4$ and $R^5$ are independently $C_{1-7}$ alkyl, $C_{3-8}$ cycloalkyl, $C_{3-8}$ cycloalkyl-$C_{1-2}$ alkyl, or a group $(CH_2)_tR_6$ where t is 1 or 2 and $R_6$ is thienyl, pyrrolyl or furyl optionally further substituted by one or two substituents selected from $C_{1-6}$ alkyl, $C_{1-6}$ alkoxy, trifluoromethyl or halogen, or is phenyl optionally substituted by one or two substituents selected from $C_{1-4}$ alkoxy, trifluoromethyl, halogen, nitro, carboxy, esterified carboxy, and $C_{1-4}$ alkyl (optionally substituted by hydroxy, $C_{1-4}$ alkoxy, carboxy, esterified carboxy or in vivo hydrolyzable acyloxy); or a pharmaceutically acceptable salt thereof or an N-oxide thereof; or an individual isomer or mixture of isomers thereof, which process comprises one or more of the following steps:

(a) reactively contacting a compound of Formula II

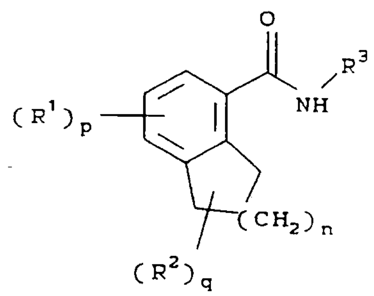

II

in which n, p, q, $R^1$, $R^2$ and $R^3$ are as defined above with a formylating agent in the presence of a strong base to form a compound of Formula I wherein the dashed line is a double bond,

(b) reducing the double bond represented by the dashed line in Formula I by hydrogenation to form a compound of Formula I wherein the dashed line represents 2 hydrogens,

(c) converting a salt of a compound of Formula I to the corresponding free compound,

(d) condensing a compound of the formula $R^3L$, wherein $R^3$ has the above meanings and L is a leaving group with a compound of the Formula XIII,

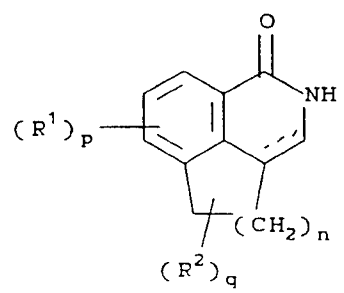

XIII

wherein $R^1$, $R^2$, n, p, q, and the dashed line have the above meanings,

(e) converting a compound of Formula I to the corresponding pharmaceutically acceptable salt,

(f) oxidizing a compound of Formula I to form the corresponding N-oxide of the $R^3$ component of Formula I, or reducing an N-oxide of the $R^3$ component to the corresponding amine,

(g) reducing a $R^1$ nitro substituent to a $R^1$ amino substituent or alkylating or acylating a $R^1$ amino substituent or alkylating a $R^1$ hydroxy substituent or dealkylating a $R^1$ alkoxy substituent or debenzylating a $R^1$ benzyloxy substituent to the corresponding compound of Formula I,

(h) hydrogenating in positions 3a, 4, 5 and 6 a compound of the Formula XIV

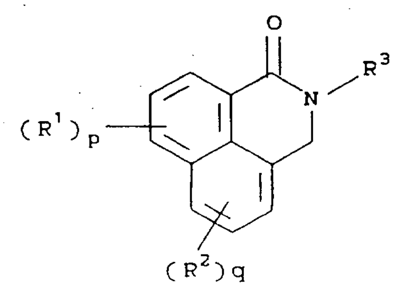

XIV

wherein $R^1$, $R^2$, $R^3$, p and q have the above meanings,
(i) separating a mixture of isomers or diastereomers of a compound of Formula I into a single isomer or a diastereomer, or
(j) conducting any of steps (a) through (i) with optically active reactants.

**2.** The process of Claim 1 wherein
p is 0, 1 or 2;
n is 1 or 2;
q is 0;
$R^1$ is halogen, lower $C_{1-6}$ alkoxy or amino; and if $R^3$ comprises
$R^4$ and $R^5$, they are each lower $C_{1-6}$ alkyl.

**3.** The process of Claim 2 wherein n is 1.

**4.** The process of Claim 3 wherein p is 0, the dashed line represents a double bond and if $R^3$ comprises $R^4$ and $R^5$ they are each methyl.

**5.** The process of Claim 2 or 4 wherein $R^3$ is
1-azabicyclo[2.2.2]oct-3-yl;
1-azabicyclo[2.2.2]oct-4-yl;
*endo*-9-methyl-9-azabicyclo[3.3.1]non-3-yl;
*endo*-8-methyl-8-azabicyclo[3.2.1]oct-3-yl;
*exo*-8-methyl-8-azabicyclo[3.2.1]oct-3-yl; or
*endo*-1-azabicyclo[3.3.1]non-4-yl.

**6.** The process of Claim 5 wherein a compound in which $R^3$ is 1-azabicyclo[2.2.2]oct-3-yl, namely 2-(1-azabicyclo[2.2.2]oct-3-yl)-1,2,4,5-tetrahydro-cyclopent[de]isoquinolin-1-one, is prepared.

**7.** The process of Claim 6 wherein 2-(1-azabicyclo[2.2.2]oct-3-yl)-1,2,4,5-tetrahydro-cyclopenta[de]-isoquinolin-1-one hydrochloride is prepared.

**8.** The process of Claim 6 wherein (S)-2-(1-azabicyclo[2.2.2]oct-3-yl)-1,2,4,5-tetrahydro-cyclopenta[de]-isoquinolin-1-one hydrochloride is prepared.

**9.** The process of Claim 5 wherein $R^3$ is 8-methyl-8-azabicyclo[3.2.1]oct-3-yl, namely, 2-(8-methyl-8-azabicyclo[3.2.1]-oct-3-yl)-1,2,4,5-tetrahydro-cyclopent[de]isoquinolin-1-one, is prepared.

**10.** The process of Claim 9 wherein a compound in which $R^3$ is endo-8-methyl-8-azabicyclo[3.2.1]oct-3-yl, namely, 2-(endo-8-methyl-8-azabicyclo[3.2.1]-oct-3-yl)-1,2,4,5-tetrahydro-cyclopent[de]isoquinolin-1-one, is prepared.

**11.** The process of Claim 2 wherein n is 2.

**12.** The process of Claim 11 wherein p is 0, the dashed line represents a double bond and if $R^3$ comprises $R^4$ and $R^5$, they are each methyl.

**13.** The process of Claim 12 wherein $R^3$ is
1-azabicyclo[2.2.2]oct-3-yl;
1-azabicyclo[2.2.2]oct-4-yl;
*endo*-9-methyl-9-azabicyclo[3.3.1]non-3-yl;
*endo*-8-methyl-8-azabicyclo[3.2.1]oct-3-yl;
*exo*-8-methyl-8-azabicylo[3.2.1]oct-3-yl; or
*endo*-1-azabicyclo[3.3.1]non-4-yl.

**14.** The process of Claim 13 wherein a compound in which $R^3$ is 1-aza-bicyclo[2.2.2]oct-4-yl, namely 2-(1-azabicyclo[2.2.2]oct-4-yl)-2,4,5,6-tetrahydro-1H-benz[de]isoquinolin-1-one, is prepared.

**15.** The process of Claim 13 wherein a compound in which $R^3$ is exo-8-methyl-8-azabicyclo[3.2.1]oct-3-yl, namely 2-(exo-8-methyl-8-azabicyclo[3.2.1]oct-3-yl)-2,4,5,6-tetrahydro-1H-benz[de]isoquinolin-1-one, is prepared.

**16.** The process of Claim 13 wherein a compound in which $R^3$ is endo-8-methyl-8-azabicyclo[3.2.1]oct-3-yl, namely 2-(endo-8-methyl-8-azabicyclo[3.2.1]oct-3-yl)-2,4,5,6-tetrahydro-1H-benz[de]isoquinolin-1-one, is prepared.

**17.** The process of Claim 13 wherein a compound in which $R^3$ is 1-azabicyclo[2.2.2]oct-3-yl, namely 2-(1-azabicyclo[2.2.2]oct-3-yl)-2,4,5,6-tetrahydro-1H-benz[de]isoquinolin-1-one, is prepared.

**18.** The process of Claim 17 wherein (S)-2-(1-azabicyclo[2.2.2]oct-3-yl)-2,4,5,6-tetrahydro-1H-benz[de]-isoquinolin-1-one hydrochloride is prepared.

**19.** The process of Claim 17 wherein (S)-2-(1-azabicyclo[2.2.2]oct-3-yl)-2,4,5,6-tetrahydro-1H-benz[de]-isoquinolin-1-one as the free base is prepared.

**20.** The process of Claim 17 wherein (R)-2-(1-azabicyclo[2.2.2]oct-3-yl)-2,4,5,6-tetrahydro-1H-benz[de]-isoquinolin-1-one is prepared.

**21.** The process of Claim 13 wherein a compound wherein $R^3$ is endo-9-methyl-9-azabicyclo[3.3.1]non-3-yl, namely 2-(endo-9-methyl-9-azabicyclo[3.3.1]non-3-yl)-2,4,5,6-tetrahydro-1H-benz[de]isoquinolin-1-one, is prepared.

**22.** The process of Claim 13 wherein a compound in which $R^3$ is endo-1-azabicyclo[3.3.1]non-4-yl, namely 2-(endo-1-azabicyclo[3.3.1]non-4-yl)-2,4,5,6-tetrahydro-1H-benz[de]isoquinolin-1-one, is prepared.

**23.** The process of Claim 2 wherein p is 0, the dashed line represents two hydrogens, and if $R^3$ comprises $R^4$ and $R^5$, they are each methyl.

**24.** The process of Claim 23 wherein $R^3$ is
1-azabicyclo[2.2.2]oct-3-yl;
1-azabicyclo[2.2.2]oct-4-yl;
*endo*-9-methyl-9-azabicyclo[3.3.1]non-3-yl;
*endo*-8-methyl-8-azabicyclo[3.2.1]oct-3-yl;
*exo*-8-methyl-8-azabicyclo[3.2.1]oct-3-yl; or
*endo*-1-azabicyclo[3.3.1]non-4-yl.

**25.** The process of Claim 24 wherein n is 1.

**26.** The process of Claim 24 wherein n is 2.

**27.** The process of Claim 26 wherein a compound wherein $R^3$ is 1-azabicyclo[2.2.2]oct-3-yl, namely 2-(1-azabicyclo[2.2.2]oct-3-yl)-2,3,3a,4,5,6-hexahydro-1H-benz[de]isoquinolin-1-one, in particular the (3aS, 3'S) isomer thereof and its hydrochloride salt, are prepared.

**28.** The process of Claim 1 wherein
n is 3;
p is 0, 1 or 2;
q is 0;
$R^1$ is halogen, lower $C_{1-6}$ alkoxy or amino; and if $R^3$ comprises $R^4$ and $R^5$, they are each lower $C_{1-6}$ alkyl.

**29.** The process of Claim 28 wherein p is 0, the dashed line represents a double bond, and if $R^3$ comprises $R^4$ and $R^5$, they are each methyl.

**30.** The process of Claim 29 wherein $R^3$ is
1-azabicyclo[2.2.2]oct-3-yl;
1-azabicyclo[2.2.2]oct-4-yl;
*endo*-9-methyl-9-azabicyclo[3.3.1]non-3-yl;
*endo*-8-methyl-8-azabicyclo[3.2.1]oct-3-yl;
*exo*-8-methyl-8-azabicyclo[3.2.1]oct-3-yl; or
*endo*-1-azabicyclo[3.3.1]non-4-yl.

**31.** The process of Claim 30 wherein a compound in which $R^3$ is 1-azabicyclo[2.2.2]oct-3-yl, namely (RS)-2-(1-azabicyclo-[2.2.2]oct-3-yl)-1,2,4,5,6,7-hexahydro-cyclohept[de]isoquinolin-1-one, is prepared.

**32.** The process of Claim 1 wherein the N-oxide of a compound is prepared, in which
p is 0, 1 or 2;
q is 0;
$R^1$ is halogen, lower $C_{1-6}$ alkoxy or amino; and if $R^3$ comprises $R^4$ and $R^5$, they are each lower $C_{1-6}$ alkyl.

**33.** The process of Claim 32 wherein p is 0, and if $R^3$ comprises $R^4$ and $R^5$, they are each methyl.

**34.** The process of Claim 33 wherein n is 2 and $R^3$ is 1-azabicyclo[2.2.2]oct-3-yl.

**35.** A process for preparing a compound represented by the formula

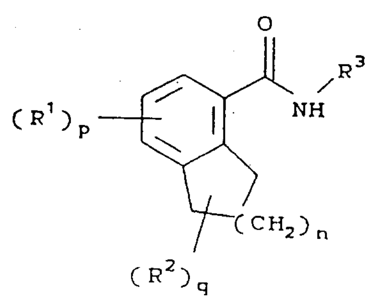

II

wherein
n is 1, 2 or 3;
p is 0, 1, 2 or 3;
q is 0, 1 or 2;
each $R^1$ is independently selected from halogen, hydroxy, lower $C_{1-6}$ alkoxy (optionally substituted with phenyl), lower $C_{1-6}$ alkyl, nitro, amino, amino carbonyl, (lower $C_{1-6}$ alkyl)amino, di(lower $C_{1-6}$ alkyl)amino, and (lower $C_{1-6}$ alkanoyl)amino;
each $R^2$ is lower $C_{1-6}$ alkyl; and
$R^3$ is selected from

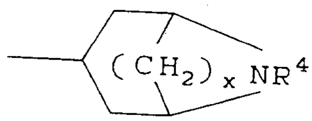

(a)

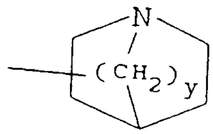

(b)

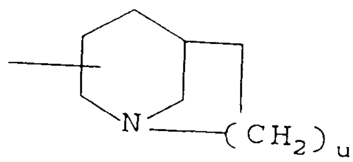

(c)

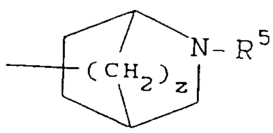

(d)

in which

u, x, y and z are all independently an integer from 1 to 3; and

$R^4$ and $R^5$ are independently $C_{1-7}$ alkyl, $C_{3-8}$ cycloalkyl, $C_{3-8}$ cycloalkyl-$C_{1-2}$alkyl, or a group $(CH_2)_tR_6$ where t is 1 or 2 and $R_6$ is thienyl, pyrrolyl or furyl optionally further substituted by one or two substituents selected from $C_{1-6}$ alkyl, $C_{1-6}$ alkoxy, trifluoromethyl or halogen, or is phenyl optionally substituted by one or two substituents selected from $C_{1-4}$ alkoxy, trifluoromethyl, halogen, nitro, carboxy, esterified carboxy, and $C_{1-4}$ alkyl (optionally substituted by hydroxy, $C_{1-4}$ alkoxy, carboxy, esterified carboxy or in vivo hydrolyzable acyloxy);

or an individual isomer or mixture of isomers thereof, or salt thereof,

which process comprises reacting a compound of Formula III

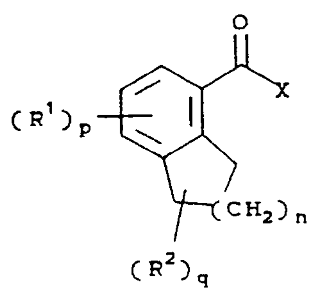

III

wherein

X is OH, OR (R = alkyl) or halogen; and

n, p, q, $R^1$ and $R^2$ are as defined above,

with an amine of Formula $R^3NH_2$, wherein $R^3$ is as defined above.

**Patentansprüche**

**Patentansprüche für folgende Vertragsstaaten : AT, BE, CH, DE, DK, FR, GB, IT, LI, LU, NL, SE**

1. Verbindung der Formel I

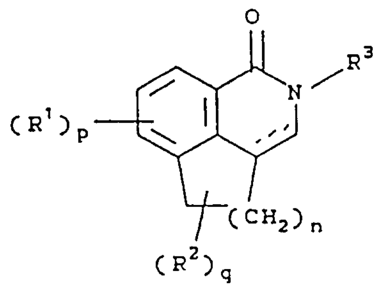

I

in der
die gestrichelte Linie eine fakultative Doppelbindung bezeichnet;
n 1, 2 oder 3 ist;
p 0, 1, 2 oder 3 ist;
q 0, 1 oder 2 ist;
jedes $R^1$ unabhängig ausgewählt ist aus Halogen, Hydroxy, Nieder-$C_{1-6}$-alkoxy (gegebenenfalls mit Phenyl substituiert), Nieder-$C_{1-6}$-alkyl, Nitro, Amino, Aminocarbonyl, (Nieder-$C_{1-6}$-alkyl)amino, Di-(nieder-$C_{1-6}$-alkyl)amino und (Nieder-$C_{1-6}$-alkanoyl)amino;
jedes $R^2$ Nieder-$C_{1-6}$-alkyl ist; und
$R^3$ ausgewählt ist aus

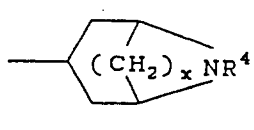

(a)

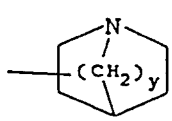

(b)

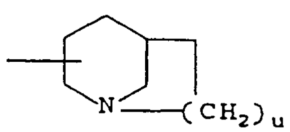

(c)

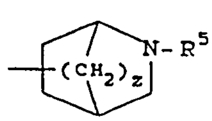

(d)

worin
u, x, y und z alle unabhängig eine ganze Zahl von 1 bis 3 sind; und
$R^4$ und $R^5$ unabhängig $C_{1-7}$-Alkyl, $C_{3-8}$-Cycloalkyl, $C_{3-8}$-Cycloalkyl-$C_{1-2}$-alkyl oder eine Gruppe $(CH_2)_tR_6$ sind, worin t 1 oder 2 ist und $R_6$ Thienyl, Pyrrolyl oder Furyl ist, gegebenenfalls weiter substituiert durch einen oder zwei Substituenten, die aus $C_{1-6}$-Alkyl, $C_{1-6}$-Alkoxy, Trifluormethyl oder Halogen ausgewählt sind, oder Phenyl ist, gegebenenfalls durch einen oder zwei Substituenten substituiert, die ausgewählt sind aus $C_{1-4}$-Alkoxy, Trifluormethyl, Halogen, Nitro, Carboxy, verestertem

Carboxy und $C_{1-4}$-Alkyl (gegebenenfalls durch Hydroxy, $C_{1-4}$-Alkoxy, Carboxy, verestertes Carboxy oder in-vivo-hydrolysierbares Acyloxy substituiert); oder ein pharmazeutisch annehmbares Salz derselben oder ein N-Oxid derselben; oder
ein einzelnes Isomer oder eine Mischung von Isomeren derselben.

**2.** Verbindung nach Anspruch 1, in der
p 0, 1 oder 2 ist;
n 1 oder 2 ist;
q 0 ist;
$R^1$ Halogen, Nieder-$C_{1-6}$-alkoxy oder Amino ist;
und, falls $R^3$ $R^4$ und $R^5$ umfaßt, diese jeweils Nieder-$C_{1-6}$-alkyl sind.

**3.** Verbindung nach Anspruch 2, in der n 1 ist.

**4.** Verbindung nach Anspruch 3, in der p 0 ist, die gestrichelte Linie eine Doppelbindung darstellt und, falls $R^3$ $R^4$ und $R^5$ umfaßt, diese jeweils Methyl sind.

**5.** Verbindung nach Anspruch 2 oder 4, in der $R^3$ 1-Azabicyclo[2.2.2]oct-3-yl;
1-Azabicyclo[2.2.2]oct-4-yl;
endo-9-Methyl-9-azabicyclo[3.3.1]non-3-yl;
endo-8-Methyl-8-azabicyclo[3.2.1]oct-3-yl;
exo-8-Methyl-8-azabicyclo[3.2.1]oct-3-yl; oder
endo-1-Azabicyclo[3.3.1]non-4-yl ist.

**6.** Verbindung nach Anspruch 5, in der $R^3$ 1-Azabicyclo[2.2.2]oct-3-yl ist, nämlich 2-(1-Azabicyclo[2.2.2]-oct-3-yl)-1,2,4,5-tetrahydrocyclopent[de]isochinolin-1-on.

**7.** Verbindung nach Anspruch 6, die 2-(1-Azabicyclo[2.2.2]oct-3-yl)-1,2,4,5-tetrahydrocyclopent[de]-isochinolin-1-on-hydrochlorid ist.

**8.** Verbindung nach Anspruch 6, die (S)-2-(1-Azabicyclo[2.2.2]oct-3-yl)-1,2,4,5-tetrahydrocyclopent[de]-isochinolin-1-on-hydrochlorid ist.

**9.** Verbindung nach Anspruch 5, in der $R^3$ 8-Methyl-8-azabicyclo[3.2.1]oct-3-yl ist, nämlich 2-(8-Methyl-8-azabicyclo[3.2.1]oct-3-yl)-1,2,4,5-tetrahydrocyclopent[de]isochinolin-1-on.

**10.** Verbindung nach Anspruch 9, in der $R^3$ endo-8-Methyl-8-azabicyclo[3.2.1]oct-3-yl ist, nämlich 2-(endo-8-Methyl-8-azabicyclo[3.2.1]oct-3-yl)-1,2,4,5-tetrahydrocyclopent[de]isochinolin-1-on.

**11.** Verbindung nach Anspruch 2, in der n 2 ist.

**12.** Verbindung nach Anspruch 11, in der p 0 ist, die gestrichelte Linie eine Doppelbindung darstellt und, falls $R^3$ $R^4$ und $R^5$ umfaßt, diese jeweils Methyl sind.

**13.** Verbindung nach Anspruch 12, in der $R^3$ 1-Azabicyclo[2.2.2]oct-3-yl;
1-Azabicyclo[2.2.2]oct-4-yl;
endo-9-Methyl-9-azabicyclo[3.3.1]non-3-yl;
endo-8-Methyl-8-azabicyclo[3.2.1]oct-3-yl;
exo-8-Methyl-8-azabicyclo[3.2.1]oct-3-yl; oder
endo-1-Azabicyclo[3.3.1]non-4-yl ist.

**14.** Verbindung nach Anspruch 13, in der $R^3$ 1-Azabicyclo[2.2.2]oct-4-yl ist, nämlich 2-(1-Azabicyclo[2.2.2]-oct-4-yl)-2,4,5,6-tetrahydro-1H-benz[de]isochinolin-1-on.

**15.** Verbindung nach Anspruch 13, in der $R^3$ exo-8-Methyl-8-azabicyclo[3.2.1]oct-3-yl ist, nämlich 2-(exo-8-Methyl-8-azabicyclo[3.2.1]oct-3-yl)-2,4,5,6-tetrahydro-1H-benz[de]isochinolin-1-on.

**16.** Verbindung nach Anspruch 13, in der $R^3$ endo-8-Methyl-8-azabicyclo[3.2.1]oct-3-yl ist, nämlich 2-(endo-8-Methyl-8-azabicyclo[3.2.1]oct-3-yl)-2,4,5,6-tetrahydro-1H-benz[de]isochinolin-1-on.

**17.** Verbindung nach Anspruch 13, in der $R^3$ 1-Azabicyclo[2.2.2]oct-3-yl ist, nämlich 2-(1-Azabicyclo[2.2.2]-oct-3-yl)-2,4,5,6-tetrahydro-1H-benz[de]isochinolin-1-on.

**18.** Verbindung nach Anspruch 17, die (S)-2-(1-Azabicyclo[2.2.2]oct-3-yl)-2,4,5,6-tetrahydro-1H-benz[de]-isochinolin-1-on-hydrochlorid ist.

**19.** Verbindung nach Anspruch 17, die (S)-2-(1-Azabicyclo[2.2.2]oct-3-yl)-2,4,5,6-tetrahydro-1H-benz[de]-isochinolin-1-on als freie Base ist.

**20.** Verbindung nach Anspruch 17, die (R)-2-(1-Azabicyclo[2.2.2]oct-3-yl)-2,4,5,6-tetrahydro-1H-benz[de]-isochinolin-1-on ist.

**21.** Verbindung nach Anspruch 13, in der $R^3$ endo-9-Methyl-9-azabicyclo[3.3.1]non-3-yl ist, nämlich 2-(endo-9-Methyl-9-azabicyclo[3.3.1]non-3-yl)-2,4,5,6-tetrahydro-1H-benz[de]isochinolin-1-on.

**22.** Verbindung nach Anspruch 13, in der $R^3$ endo-1-Azabicyclo[3.3.1]non-4-yl ist, nämlich 2-(endo-1-Azabicyclo[3.3.1]non-4-yl)-2,4,5,6-tetrahydro-1H-benz[de]isochinolin-1-on.

**23.** Verbindung nach Anspruch 2, in der p 0 ist, die gestrichelte Linie zwei Wasserstoffe darstellt und, falls $R^3$ $R^4$ und $R^5$ umfaßt, diese jeweils Methyl sind.

**24.** Verbindung nach Anspruch 23, in der $R^3$ 1-Azabicyclo[2.2.2]oct-3-yl;
1-Azabicyclo[2.2.2]oct-4-yl;
endo-9-Methyl-9-azabicyclo[3.3.1]non-3-yl;
endo-8-Methyl-8-azabicyclo[3.2.1]oct-3-yl;
exo-8-Methyl-8-azabicyclo[3.2.1]oct-3-yl; oder
endo-1-Azabicyclo[3.3.1]non-4-yl ist.

**25.** Verbindung nach Anspruch 24, in der n 1 ist.

**26.** Verbindung nach Anspruch 24, in der n 2 ist.

**27.** Verbindung nach Anspruch 26, in der $R^3$ 1-Azabicyclo[2.2.2]oct-3-yl ist, nämlich 2-(1-Azabicyclo[2.2.2]-oct-3-yl)-2,3,3a,4,5,6-hexahydro-1H-benz[de]isochinolin-1-on, insbesondere das (3aS, 3'S)-Isomer desselben, und sein Hydrochloridsalz.

**28.** Verbindung nach Anspruch 1, in der
n 3 ist;
p 0, 1 oder 2 ist;
q 0 ist;
$R^1$ Halogen, Nieder-$C_{1-6}$-alkoxy oder Amino ist;
und, falls $R^3$ $R^4$ und $R^5$ umfaßt, diese jeweils Nieder-$C_{1-6}$-alkyl sind.

**29.** Verbindung nach Anspruch 28, in der p 0 ist, die gestrichelte Linie eine Doppelbindung darstellt und, falls $R^3$ $R^4$ und $R^5$ umfaßt, diese jeweils Methyl sind.

**30.** Verbindung nach Anspruch 29, in der $R^3$ 1-Azabicyclo[2.2.2]oct-3-yl;
1-Azabicyclo[2.2.2]oct-4-yl;
endo-9-Methyl-9-azabicyclo[3.3.1]non-3-yl;
endo-8-Methyl-8-azabicyclo[3.2.1]oct-3-yl;
exo-8-Methyl-8-azabicyclo[3.2.1]oct-3-yl; oder
endo-1-Azabicyclo[3.3.1]non-4-yl ist.

**31.** Verbindung nach Anspruch 30, in der $R^3$ 1-Azabicyclo[2.2.2]oct-3-yl ist, nämlich (RS)-2-(1-Azabicyclo-[2.2.2]oct-3-yl)-1,2,4,5,6,7-hexahydrocyclohept[de]isochinolin-1-on.

**32.** Das N-Oxid der Verbindung nach Anspruch 1, in dem p 0, 1 oder 2 ist;
q 0 ist;
$R^1$ Halogen, Nieder-$C_{1-6}$-alkoxy oder Amino ist;
und, falls $R^3$ $R^4$ und $R^5$ umfaßt, diese jeweils Nieder-$C_{1-6}$-alkyl sind.

**33.** Verbindung nach Anspruch 32, in der p 0 ist und, falls $R^3$ $R^4$ und $R^5$ umfaßt, diese jeweils Methyl sind.

**34.** Verbindung nach Anspruch 33, in der n 2 ist und $R^3$ 1-Azabicyclo[2.2.2]oct-3-yl ist.

**35.** Verbindung nach irgendeinem der Ansprüche 1 bis 34 zur Verwendung als Arzneistoff.

**36.** Pharmazeutische Zusammensetzung, umfassend eine therapeutisch wirksame Menge einer Verbindung nach Anspruch 1 bis 34, vorzugsweise in Kombination mit einem pharmazeutisch annehmbaren Hilfsstoff.

**37.** Verwendung einer Verbindung nach Anspruch 1 bis 34 bei der Herstellung eines Medikaments zur Behandlung eines Zustands, der ausgewählt ist aus Erbrechen, einer gastrointestinalen Störung, ZNS-Störung, kardiovaskulären Störung und Schmerzen oder einem Zustand, in dem der 5-$HT_3$-Rezeptoreine Rolle spielt.

**38.** Verfahren zur Herstellung einer Verbindung der Formel I

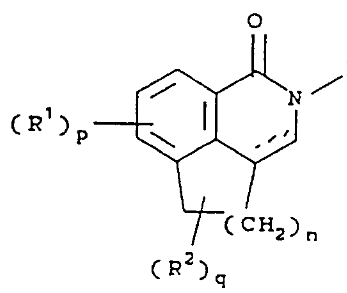

I

in der
n 1, 2 oder 3 ist;
p 0, 1, 2 oder 3 ist;
q 0, 1 oder 2 ist;
jedes $R^1$ unabhängig ausgewählt ist aus Halogen, Hydroxy, Nieder-$C_{1-6}$-alkoxy (gegebenenfalls mit Phenyl substituiert), Nieder-$C_{1-6}$-alkyl, Nitro, Amino, Aminocarbonyl, (Nieder-$C_{1-6}$-alkyl) amino, Di-(nieder-$C_{1-6}$-alkyl) amino und (Nieder-$C_{1-6}$-alkanoyl)amino;
jedes $R^2$ Nieder-$C_{1-6}$-alkyl ist; und
$R^3$ ausgewählt ist aus

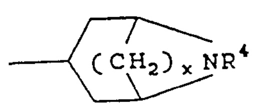

(a)

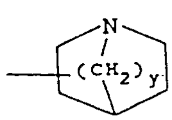

(b)

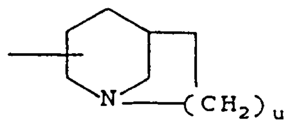

(c)

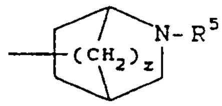

(d)

worin

u, x, y und z alle unabhängig eine ganze Zahl von 1 bis 3 sind; und

$R^4$ und $R^5$ unabhängig $C_{1-7}$-Alkyl, $C_{3-8}$-Cycloalkyl, $C_{3-8}$-Cycloalkyl-$C_{1-2}$-alkyl oder eine Gruppe $(CH_2)_tR_6$ sind, worin t 1 oder 2 ist und $R_6$ Thienyl, Pyrrolyl oder Furyl ist, gegebenenfalls weiter substituiert durch einen oder zwei Substituenten, die aus $C_{1-6}$-Alkyl, $C_{1-6}$-Alkoxy, Trifluormethyl oder Halogen ausgewählt sind, oder Phenyl ist, gegebenenfalls durch einen oder zwei Substituenten substituiert, die ausgewählt sind aus $C_{1-4}$-Alkoxy, Trifluormethyl, Halogen, Nitro, Carboxy, verestertem Carboxy und $C_{1-4}$-Alkyl (gegebenenfalls durch Hydroxy, $C_{1-4}$-Alkoxy, Carboxy, verestertes Carboxy oder in-vivo-hydrolysierbares Acyloxy substituiert); oder eines pharmazeutisch annehmbaren Salzes derselben oder eines N-Oxids derselben oder eines einzelnen Isomers oder einer Mischung von Isomeren derselben, wobei das Verfahren einen oder mehrere der folgenden Schritte umfaßt:

(a) das reaktive Inkontaktbringen einer Verbindung der Formel II

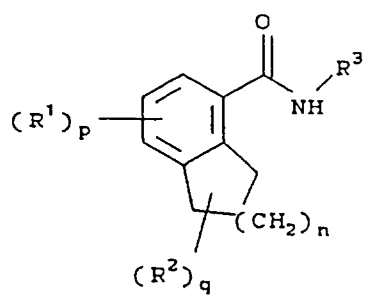

II

in der n, p, q, $R^1$, $R^2$ und $R^3$ wie oben definiert sind, mit einem Formylierungsmittel in Gegenwart einer starken Base, um eine Verbindung der Formel I zu bilden, in der die gestrichelte Linie eine Doppelbindung ist,

(b) das Reduzieren der Doppelbindung, die durch die gestrichelte Linie in Formel I dargestellt wird, durch Hydrierung, um eine Verbindung der Formel I zu bilden, in der die gestrichelte Linie zwei Wasserstoffe darstellt,

(c) das Überführen eines Salzes einer Verbindung der Formel I in die entsprechende freie Verbindung,

(d) das Kondensieren einer Verbindung der Formel $R^3L$, in der $R^3$ die obigen Bedeutungen aufweist und L eine Abgangsgruppe ist, mit einer Verbindung der Formel XIII,

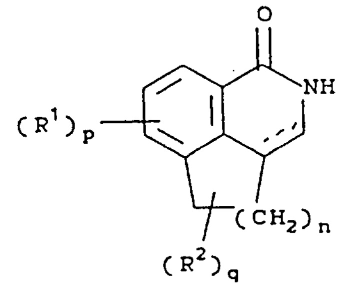

XIII

in der $R^1$, $R^2$, n, p, q und die gestrichelte Linie die obigen Bedeutungen aufweisen,

(e) das Überführen einer Verbindung der Formel I in das entsprechende pharmazeutisch annehmbare Salz,

(f) das Oxidieren einer Verbindung der Formel I, um das entsprechende N-Oxid der $R^3$-Komponente der Formel I zu bilden, oder das Reduzieren eines N-Oxids der $R^3$-Komponente in das entsprechende Amin,

(g) das Reduzieren eines $R^1$-Nitrosubstituenten zu einem $R^1$-Aminosubstituenten oder das Alkylieren oder Acylieren eines $R^1$-Aminosubstituenten oder das Alkylieren eines $R^1$-Hydroxysubstituenten oder das Desalkylieren eines $R^1$-Alkoxysubstituenten oder das Desbenzylieren eines $R^1$-Benzyloxysubstituenten zu der entsprechenden Verbindung der Formel I,

(h) das Hydrieren in den Stellungen 3a, 4, 5 und 6 einer Verbindung der Formel XIV

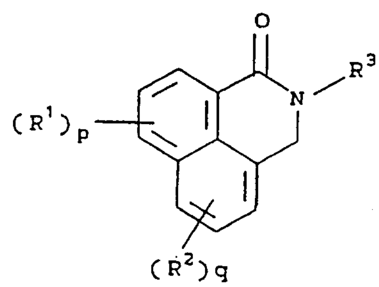

XIV

in der $R^1$, $R^2$, $R^3$, p und q die obigen Bedeutungen aufweisen,

(i) das Auftrennen einer Mischung von Isomeren oder Diastereomeren einer Verbindung der Formel I in ein einzelnes Isomer oder ein Diastereomer oder

(j) das Durchführen irgendeines der Schritte (a) bis (i) mit optisch aktiven Reaktanten.

**39.** Verfahren nach Anspruch 38, in dem $R^3$ 1-Azabicyclo[2.2.2]oct-3-yl;
1-Azabicyclo[2.2.2]oct-4-yl;
endo-9-Methyl-9-azabicyclo[3.3.1]non-3-yl;
exo-8-Methyl-8-azabicyclo[3.2.1]oct-3-yl; oder
endo-1-Azabicyclo[3.3.1]non-4-yl ist.

**40.** Verbindung, die dargestellt wird durch die Formel

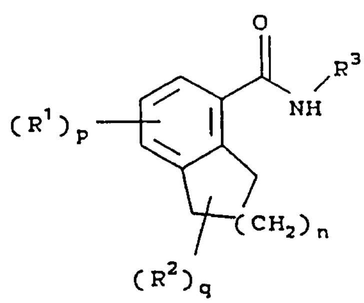

II

in der
n 1, 2 oder 3 ist;
p 0, 1, 2 oder 3 ist;
q 0, 1 oder 2 ist;
jedes $R^1$ unabhängig ausgewählt ist aus Halogen, Hydroxy, Nieder-$C_{1-6}$-alkoxy (gegebenenfalls mit Phenyl substituiert), Nieder-$C_{1-6}$-alkyl, Nitro, Amino, Aminocarbonyl, (Nieder-$C_{1-6}$-alkyl)amino, Di-(nieder-$C_{1-6}$-alkyl)amino und (Nieder-$C_{1-6}$-alkanoyl)amino;
jedes $R^2$ Nieder-$C_{1-6}$-alkyl ist; und
$R^3$ ausgewählt ist aus

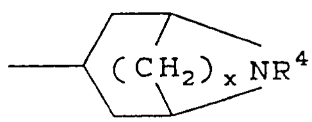
(a)

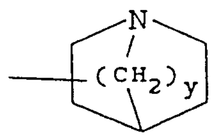
(b)

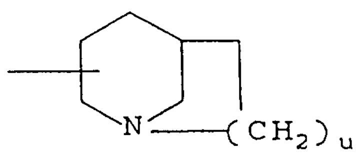
(c)

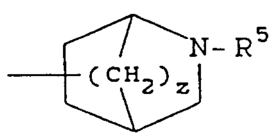
(d)

worin
u, x, y und z alle unabhängig eine ganze Zahl von 1 bis 3 sind; und
$R^4$ und $R^5$ unabhängig $C_{1-7}$-Alkyl, $C_{3-8}$-Cycloalkyl, $C_{3-8}$-Cycloalkyl-$C_{1-2}$-alkyl oder eine Gruppe $(CH_2)_tR_6$ sind, worin t 1 oder 2 ist und $R_6$ Thienyl, Pyrrolyl oder Furyl ist, gegebenenfalls weiter substituiert durch einen oder zwei Substituenten, die aus $C_{1-6}$-Alkyl, $C_{1-6}$-Alkoxy, Trifluormethyl oder Halogen ausgewählt sind, oder Phenyl ist, gegebenenfalls durch einen oder zwei Substituenten substituiert, die ausgewählt sind aus $C_{1-4}$-Alkoxy, Trifluormethyl, Halogen, Nitro, Carboxy, verestertem Carboxy und $C_{1-4}$-Alkyl (gegebenenfalls durch Hydroxy, $C_{1-4}$-Alkoxy, Carboxy, verestertes Carboxy oder in-vivo-hydrolysierbares Acyloxy substituiert);
oder ein einzelnes Isomer oder eine Mischung von Isomeren derselben oder ein Salz derselben.

**Patentansprüche für folgende Vertragsstaaten : ES, GR**

**1.** Verfahren zur Herstellung einer Verbindung der Formel I

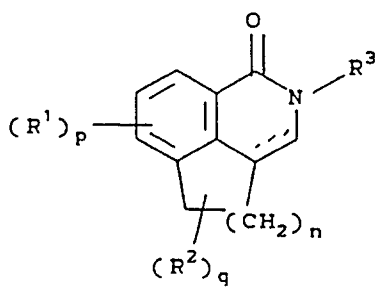

I

in der
die gestrichelte Linie eine fakultative Doppelbindung bezeichnet;
n 1, 2 oder 3 ist;
p 0, 1, 2 oder 3 ist;
q 0, 1 oder 2 ist;
jedes $R^1$ unabhängig ausgewählt ist aus Halogen, Hydroxy, Nieder-$C_{1-6}$-alkoxy (gegebenenfalls mit Phenyl substituiert), Nieder-$C_{1-6}$-alkyl, Nitro, Amino, Aminocarbonyl, (Nieder-$C_{1-6}$-alkyl)amino, Di-(nieder-$C_{1-6}$-alkyl)amino und (Nieder-$C_{1-6}$-alkanoyl)amino;
jedes $R^2$ Nieder-$C_{1-6}$-alkyl ist; und
$R^3$ ausgewählt ist aus

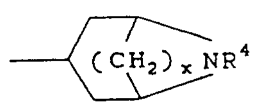

(a)

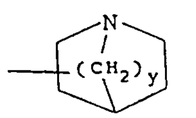

(b)

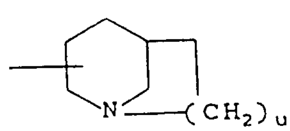

(c)

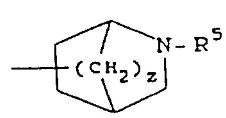

(d)

worin
u, x, y und z alle unabhängig eine ganze Zahl von 1 bis 3 sind; und
$R^4$ und $R^5$ unabhängig $C_{1-7}$-Alkyl, $C_{3-8}$-Cycloalkyl, $C_{3-8}$-Cycloalkyl-$C_{1-2}$-alkyl oder eine Gruppe $(CH_2)_tR_6$ sind, worin t 1 oder 2 ist und $R_6$ Thienyl, Pyrrolyl oder Furyl ist, gegebenenfalls weiter substituiert durch einen oder zwei Substituenten, die aus $C_{1-6}$-Alkyl, $C_{1-6}$-Alkoxy, Trifluormethyl oder Halogen ausgewählt sind, oder Phenyl ist, gegebenenfalls durch einen oder zwei Substituenten substituiert, die ausgewählt sind aus $C_{1-4}$-Alkoxy, Trifluormethyl, Halogen, Nitro, Carboxy, verestertem Carboxy und $C_{1-4}$-Alkyl (gegebenenfalls durch Hydroxy, $C_{1-4}$-Alkoxy, Carboxy, verestertes Carboxy

oder in-vivo-hydrolysierbares Acyloxy substituiert); oder eines pharmazeutisch annehmbaren Salzes derselben oder eines N-Oxids derselben oder eines einzelnen Isomers oder einer Mischung von Isomeren derselben, wobei das Verfahren einen oder mehrere der folgenden Schritte umfaßt:

(a) das reaktive Inkontaktbringen einer Verbindung der Formel II

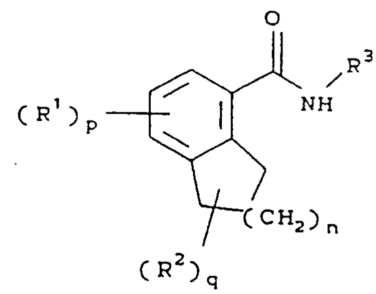

II

in der n, p, q, $R^1$, $R^2$ und $R^3$ wie oben definiert sind, mit einem Formylierungsmittel in Gegenwart einer starken Base, um eine Verbindung der Formel I zu bilden, in der die gestrichelte Linie eine Doppelbindung ist,

(b) das Reduzieren der Doppelbindung, die durch die gestrichelte Linie in Formel I dargestellt wird, durch Hydrierung, um eine Verbindung der Formel I zu bilden, in der die gestrichelte Linie zwei Wasserstoffe darstellt,

(c) das Überführen eines Salzes einer Verbindung der Formel I in die entsprechende freie Verbindung,

(d) das Kondensieren einer Verbindung der Formel $R^3L$, in der $R^3$ die obigen Bedeutungen aufweist und L eine Abgangsgruppe ist, mit einer Verbindung der Formel XIII,

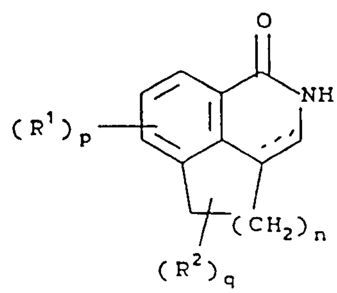

XIII

in der $R^1$, $R^2$, n, p, q und die gestrichelte Linie die obigen Bedeutungen aufweisen,

(e) das Überführen einer Verbindung der Formel I in das entsprechende pharmazeutisch annehmbare Salz,

(f) das Oxidieren einer Verbindung der Formel I, um das entsprechende N-Oxid der $R^3$-Komponente der Formel I zu bilden, oder das Reduzieren eines N-Oxids der $R^3$-Komponente in das entsprechende Amin,

(g) das Reduzieren eines $R^1$-Nitrosubstituenten zu einem $R^1$-Aminosubstituenten oder das Alkylieren oder Acylieren eines $R^1$-Aminosubstituenten oder das Alkylieren eines $R^1$-Hydroxysubstituenten oder das Desalkylieren eines $R^1$-Alkoxysubstituenten oder das Desbenzylieren eines $R^1$-Benzyloxysubstituenten zu der entsprechenden Verbindung der Formel I,

(h) das Hydrieren in den Stellungen 3a, 4, 5 und 6 einer Verbindung der Formel XIV

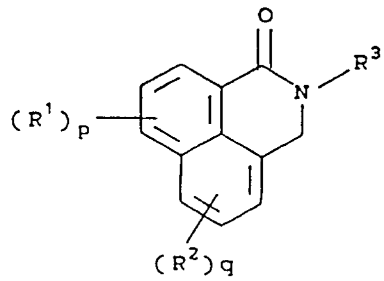

XIV

in der $R^1$, $R^2$, $R^3$, p und q die obigen Bedeutungen aufweisen,
(i) das Auftrennen einer Mischung von Isomeren oder Diastereomeren einer Verbindung der Formel I in ein einzelnes Isomer oder ein Diastereomer oder
(j) das Durchführen irgendeines der Schritte (a) bis (i) mit optisch aktiven Reaktanten.

**2.** Verfahren nach Anspruch 1, in dem
p 0, 1 oder 2 ist;
n 1 oder 2 ist;
g 0 ist;
$R^1$ Halogen, Nieder-$C_{1-6}$-alkoxy oder Amino ist;
und, falls $R^3$ $R^4$ und $R^5$ umfaßt, diese jeweils Nieder-$C_{1-6}$-alkyl sind.

**3.** Verfahren nach Anspruch 2, in dem n 1 ist.

**4.** Verfahren nach Anspruch 3, in dem p 0 ist, die gestrichelte Linie eine Doppelbindung darstellt und, falls $R^3$ $R^4$ und $R^5$ umfaßt, diese jeweils Methyl sind.

**5.** Verfahren nach Anspruch 2 oder 4, in dem $R^3$ 1-Azabicyclo[2.2.2]oct-3-yl;
1-Azabicyclo[2.2.2]oct-4-yl;
endo-9-Methyl-9-azabicyclo[3.3.1]non-3-yl;
endo-8-Methyl-8-azabicyclo[3.2.1]oct-3-yl;
exo-8-Methyl-8-azabicyclo[3.2.1]oct-3-yl; oder
endo-1-Azabicyclo[3.3.1]non-4-yl ist.

**6.** Verfahren nach Anspruch 5, in dem eine Verbindung hergestellt wird, in der $R^3$ 1-Azabicyclo[2.2.2]oct-3-yl ist, nämlich 2-(1-Azabicyclo[2.2.2]oct-3-yl)-1,2,4,5-tetrahydrocyclopent[de]isochinolin-1-on.

**7.** Verfahren nach Anspruch 6, in dem 2-(1-Azabicyclo[2.2.2]oct-3-yl)-1,2,4,5-tetrahydrocyclopent[de]-isochinolin-1-on-hydrochlorid hergestellt wird.

**8.** Verfahren nach Anspruch 6, in dem (S)-2-(1-Azabicyclo[2.2.2]oct-3-yl)-1,2,4,5-tetrahydrocyclopent[de]-isochinolin-1-on-hydrochlorid hergestellt wird.

**9.** Verfahren nach Anspruch 5, in dem $R^3$ 8-Methyl-8-azabicyclo[3.2.1]oct-3-yl ist, nämlich 2-(8-Methyl-8-azabicyclo[3.2.1]oct-3-yl)-1,2,4,5-tetrahydrocyclopent[de]isochinolin-1-on hergestellt wird.

**10.** Verfahren nach Anspruch 9, in dem eine Verbindung hergestellt wird, in der $R^3$ endo-8-Methyl-8-azabicyclo[3.2.1]oct-3-yl ist, nämlich 2-(endo-8-Methyl-8-azabicyclo[3.2.1]oct-3-yl)-1,2,4,5-tetrahydrocyclopent[de]isochinolin-1-on.

**11.** Verfahren nach Anspruch 2, in dem n 2 ist.

**12.** Verfahren nach Anspruch 11, in dem p 0 ist, die gestrichelte Linie eine Doppelbindung darstellt und, falls $R^3$ $R^4$ und $R^5$ umfaßt, diese jeweils Methyl sind.

13. Verfahren nach Anspruch 12, in dem $R^3$ 1-Azabicyclo[2.2.2]oct-3-yl;
1-Azabicyclo[2.2.2]oct-4-yl;
endo-9-Methyl-9-azabicyclo[3.3.1]non-3-yl;
endo-8-Methyl-8-azabicyclo[3.2.1]oct-3-yl;
exo-8-Methyl-8-azabicyclo[3.2.1]oct-3-yl; oder
endo-1-Azabicyclo[3.3.1]non-4-yl ist.

14. Verfahren nach Anspruch 13, in dem eine Verbindung hergestellt wird, in der $R^3$ 1-Azabicyclo[2.2.2]oct-4-yl ist, nämlich 2-(1-Azabicyclo[2.2.2]oct-4-yl)-2,4,5,6-tetrahydro-1H-benz[de]isochinolin-1-on.

15. Verfahren nach Anspruch 13, in dem eine Verbindung hergestellt wird, in der $R^3$ exo-8-Methyl-8-azabicyclo[3.2.1]oct-3-yl ist, nämlich 2-(exo-8-Methyl-8-azabicyclo[3.2.1]oct-3-yl)-2,4,5,6-tetrahydro-1H-benz[de]isochinolin-1-on.

16. Verfahren nach Anspruch 13, in dem eine Verbindung hergestellt wird, in der $R^3$ endo-8-Methyl-8-azabicyclo[3.2.1]oct-3-yl ist, nämlich 2-(endo-8-Methyl-8-azabicyclo[3.2.1]oct-3-yl)-2,4,5,6-tetrahydro-1H-benz[de]isochinolin-1-on.

17. Verfahren nach Anspruch 13, in dem eine Verbindung hergestellt wird, in der $R^3$ 1-Azabicyclo[2.2.2]oct-3-yl ist, nämlich 2-(1-Azabicyclo[2.2.2]oct-3-yl)-2,4,5,6-tetrahydro-1H-benz[de]isochinolin-1-on.

18. Verfahren nach Anspruch 17, in dem (S)-2-(1-Azabicyclo[2.2.2]oct-3-yl)-2,4,5,6-tetrahydro-1H-benz[de]-isochinolin-1-on-hydrochlorid hergestellt wird.

19. Verfahren nach Anspruch 17, in dem (S)-2-(1-Azabicyclo[2.2.2]oct-3-yl)-2,4,5,6-tetrahydro-1H-benz[de]-isochinolin-1-on als freie Base hergestellt wird.

20. Verfahren nach Anspruch 17, in dem (R)-2-(1-Azabicyclo[2.2.2]oct-3-yl)-2,4,5,6-tetrahydro-1H-benz[de]-isochinolin-1-on hergestellt wird.

21. Verfahren nach Anspruch 13, in dem eine Verbindung hergestellt wird, in der $R^3$ endo-9-Methyl-9-azabicyclo[3.3.1]non-3-yl ist, nämlich 2-(endo-9-Methyl-9-azabicyclo[3.3.1]non-3-yl)-2,4,5,6-tetrahydro-1H-benz[de]isochinolin-1-on.

22. Verfahren nach Anspruch 13, in dem eine Verbindung hergestellt wird, in der $R^3$ endo-1-Azabicyclo-[3.3.1]non-4-yl ist, nämlich 2-(endo-1-Azabicyclo[3.3.1]non-4-yl)-2,4,5,6-tetrahydro-1H-benz[de]-isochinolin-1-on.

23. Verfahren nach Anspruch 2, in dem p 0 ist, die gestrichelte Linie zwei Wasserstoffe darstellt und, falls $R^3$ $R^4$ und $R^5$ umfaßt, diese jeweils Methyl sind.

24. Verfahren nach Anspruch 23, in dem $R^3$ 1-Azabicyclo[2.2.2]oct-3-yl;
1-Azabicyclo[2.2.2]oct-4-yl;
endo-9-Methyl-9-azabicyclo[3.3.1]non-3-yl;
endo-8-Methyl-8-azabicyclo[3.2.1]oct-3-yl;
exo-8-Methyl-8-azabicyclo[3.2.1]oct-3-yl; oder
endo-1-Azabicyclo[3.3.1]non-4-yl ist.

25. Verfahren nach Anspruch 24, in dem n 1 ist.

26. Verfahren nach Anspruch 24, in dem n 2 ist.

27. Verfahren nach Anspruch 26, in dem eine Verbindung, in der $R^3$ 1-Azabicyclo[2.2.2]oct-3-yl ist, nämlich 2-(1-Azabicyclo[2.2.2]oct-3-yl)-2,3,3a,4,5,6-hexahydro-1H-benz[de]isochinolin-1-on, insbesondere das (3aS, 3'S)-Isomer desselben, und sein Hydrochloridsalz hergestellt werden.

28. Verfahren nach Anspruch 1, in dem
n 3 ist;

p 0, 1 oder 2 ist;
q 0 ist;
$R^1$ Halogen, Nieder-$C_{1-6}$-alkoxy oder Amino ist;
und, falls $R^3$ $R^4$ und $R^5$ umfaßt, diese jeweils Nieder-$C_{1-6}$-alkyl sind.

**29.** Verfahren nach Anspruch 28, in dem p 0 ist, die gestrichelte Linie eine Doppelbindung darstellt und, falls $R^3$ $R^4$ und $R^5$ umfaßt, diese jeweils Methyl sind.

**30.** Verfahren nach Anspruch 29, in dem $R^3$ 1-Azabicyclo[2.2.2]oct-3-yl;
1-Azabicyclo[2.2.2]oct-4-yl;
endo-9-Methyl-9-azabicyclo[3.3.1]non-3-yl;
endo-8-Methyl-8-azabicyclo[3.2.1]oct-3-yl;
exo-8-Methyl-8-azabicyclo[3.2.1]oct-3-yl; oder
endo-1-Azabicyclo[3.3.1]non-4-yl ist.

**31.** Verfahren nach Anspruch 30, in dem eine Verbindung hergestellt wird, in der $R^3$ 1-Azabicyclo[2.2.2]oct-3-yl ist, nämlich (RS)-2-(1-Azabicyclo[2.2.2]oct-3-yl)-1,2,4,5,6,7-hexahydrocyclohept[de]isochinolin-1-on.

**32.** Verfahren nach Anspruch 1, in dem das N-Oxid einer Verbindung hergestellt wird, in der
p 0, 1 oder 2 ist;
q 0 ist;
$R^1$ Halogen, Nieder-$C_{1-6}$-alkoxy oder Amino ist;
und, falls $R^3$ $R^4$ und $R^5$ umfaßt, diese jeweils Nieder-$C_{1-6}$-alkyl sind.

**33.** Verfahren nach Anspruch 32, in dem p 0 ist und, falls $R^3$ $R^4$ und $R^5$ umfaßt, diese jeweils Methyl sind.

**34.** Verfahren nach Anspruch 33, in dem n 2 ist und $R^3$ 1-Azabicyclo[2.2.2]oct-3-yl ist.

**35.** Verfahren zur Herstellung einer Verbindung, die dargestellt wird durch die Formel

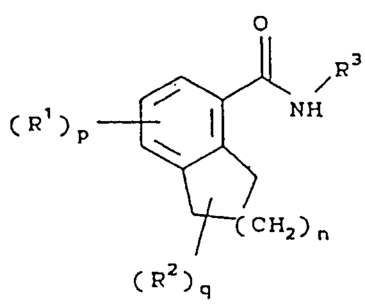

II

in der
n 1, 2 oder 3 ist;
p 0, 1, 2 oder 3 ist;
q 0, 1 oder 2 ist;
jedes $R^1$ unabhängig ausgewählt ist aus Halogen, Hydroxy, Nieder-$C_{1-6}$-alkoxy (gegebenenfalls mit Phenyl substituiert), Nieder-$C_{1-6}$-alkyl, Nitro, Amino, Aminocarbonyl, (Nieder-$C_{1-6}$-alkyl)amino, Di-(nieder-$C_{1-6}$-alkyl)amino und (Nieder-$C_{1-6}$-alkanoyl)amino;
jedes $R^2$ Nieder-$C_{1-6}$-alkyl ist; und
$R^3$ ausgewählt ist aus

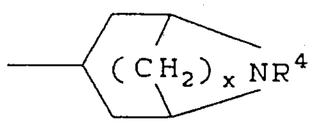

(a)

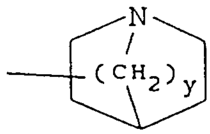

(b)

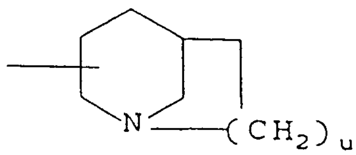

(c)

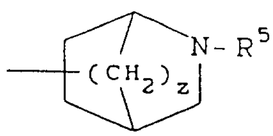

(d)

worin

u, x, y und z alle unabhängig eine ganze Zahl von 1 bis 3 sind; und

$R^4$ und $R^5$ unabhängig $C_{1-7}$-Alkyl, $C_{3-8}$-Cycloalkyl, $C_{3-8}$-Cycloalkyl-$C_{1-2}$-alkyl oder eine Gruppe $(CH_2)_tR_6$ sind, worin t 1 oder 2 ist und $R_6$ Thienyl, Pyrrolyl oder Furyl ist, gegebenenfalls weiter substituiert durch einen oder zwei Substituenten, die aus $C_{1-6}$-Alkyl, $C_{1-6}$-Alkoxy, Trifluormethyl oder Halogen ausgewählt sind, oder Phenyl ist, gegebenenfalls durch einen oder zwei Substituenten substituiert, die ausgewählt sind aus $C_{1-4}$-Alkoxy, Trifluormethyl, Halogen, Nitro, Carboxy, verestertem Carboxy und $C_{1-4}$-Alkyl (gegebenenfalls durch Hydroxy, $C_{1-4}$-Alkoxy, Carboxy, verestertes Carboxy oder in-vivo-hydrolysierbares Acyloxy substituiert);

oder eines einzelnen Isomers oder einer Mischung von Isomeren derselben oder eines Salzes derselben,

wobei das Verfahren die Umsetzung einer Verbindung der Formel III

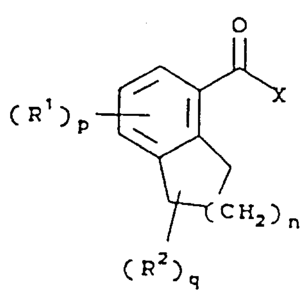

III

in der

X OH, OR (R = Alkyl) oder Halogen ist; und

n, p, q, $R^1$ und $R^2$ wie oben definiert sind,

mit einem Amin der Formel $R^3NH_2$ umfaßt, worin $R^3$ wie oben definiert ist.

**Revendications**

**Revendications pour les Etats contractants suivants : AT, BE, CH, DE, DK, FR, GB, IT, LI, LU, NL, SE**

**1.** Composé de formule I

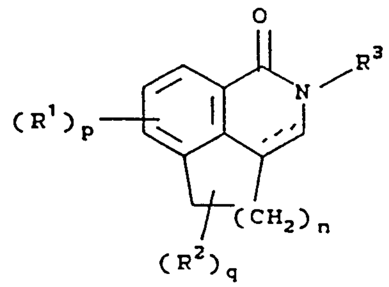

I

dans laquelle

le segment en traits interrompus désigne une double liaison facultative ;

n a la valeur 1, 2 ou 3 ;

p a la valeur 0, 1, 2 ou 3 ;

q a la valeur 0, 1 ou 2 ;

chaque $R^1$ est choisi indépendamment entre un halogène, un groupe hydroxy, alkoxy inférieur en $C_1$ à $C_6$ (facultativement substitué avec un radical phényle), alkyle inférieur en $C_1$ à $C_6$, nitro, amino, amino-carbonyle, (alkyle inférieur en $C_1$ à $C_6$)amino, di(alkyle inférieur en $C_1$ à $C_6$)amino et (alcanoyle inférieur en $C_1$ à $C_6$)amino ;

chaque $R^2$ est un groupe alkyle inférieur en $C_1$ à $C_6$ ; et

$R^3$ est choisi entre

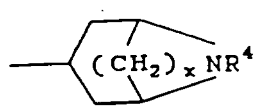

(a)

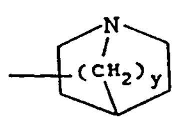

(b)

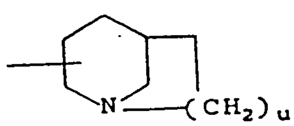

(c)

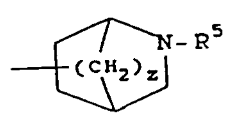

(d)

où

u, x, y et z représentent tous, indépendamment, un nombre entier de 1 à 3 ; et

$R^4$ et $R^5$ représentent, indépendamment, un groupe alkyle en $C_1$ à $C_7$, cycloalkyle en $C_3$ à $C_8$, (cycloalkyle en $C_3$ à $C_8$)-(alkyle en $C_1$ ou $C_2$) ou un groupe $(CH_2)_tR_6$ dans lequel t a la valeur 1 ou 2 et $R_6$ est un groupe thiényle, pyrrolyle ou furyle portant en outre facultativement un ou deux substituants choisis entre un radical alkyle en $C_1$ à $C_6$, alkoxy en $C_1$ à $C_6$, trifluorométhyle ou halogéno, ou représente un groupe phényle portant facultativement un ou deux substituants choisis entre des radicaux alkoxy en $C_1$ à $C_4$, trifluorométhyle, halogéno, nitro, carboxy, carboxy estérifié et alkyle en $C_1$

à $C_4$ (portant facultativement un substituant hydroxy, alkoxy en $C_1$ à $C_4$), carboxy, carboxy estérifié ou acyloxy hydrolysable in vivo) ; ou un sel ou un N-oxyde de ce composé acceptable du point de vue pharmaceutique ; ou

un isomère individuel ou un mélange d'isomères de ce composé.

**2.** Composé suivant la revendication 1, dans lequel

p a la valeur 0, 1 ou 2 ;

n a la valeur 1 ou 2 ;

q est égal à 0 ;

$R^1$ est un halogène, un groupe alkoxy inférieur en $C_1$ à $C_6$ ou amino ;

et si $R^3$ comprend

$R^4$ et $R^5$, chacun d'eux représente un groupe alkyle inférieur en $C_1$ à $C_6$.

**3.** Composé suivant la revendication 2, dans lequel n est égal à 1.

**4.** Composé suivant la revendication 3, dans lequel p est égal à 0, le segment en traits interrompus représente une double liaison et si $R^3$ comprend $R^4$ et $R^5$, chacun d'eux est un groupe méthyle.

**5.** Composé suivant la revendication 2 ou 4, dans lequel $R^3$ représente un groupe

1-azabicyclo[2.2.2]oct-3-yle ;

1-azabicyclo[2.2.2]oct-4-yle ;

endo-9-méthyl-9-azabicyclo[3.3.1]non-3-yle ;

endo-8-méthyl-8-azabicyclo[3.2.1]oct-3-yle ;

exo-8-méthyl-8-azabicyclo[3.2.1]oct-3-yle ; ou

endo-1-azabicyclo[3.3.1]non-4-yle.

**6.** Composé suivant la revendication 5, dans lequel $R^3$ est un groupe 1-azabicyclo[2.2.2]oct-3-yle, à savoir la 2-(1-azabicyclo[2.2.2]oct-3-yl)-1,2,4,5-tétrahydrocyclopent[de]isoquinoléine-1-one.

**7.** Composé suivant la revendication 6, qui est le chlorhydrate de 2-(1-azabicyclo[2.2.2]oct-3-yl)-1,2,4,5-tétrahydrocyclopenta[de]isoquinoléine-1-one.

**8.** Composé suivant la revendication 6, qui est le chlorhydrate de (S)-2-(1-azabicyclo[2.2.2]oct-3-yl)-1,2,4,5-tétrahydrocyclopenta[de]isoquinoléine-1-one.

**9.** Composé suivant la revendication 5, dans lequel $R^3$ est le groupe 8-méthyl-8-azabicyclo[3.2.1]oct-3-yle, à savoir la 2-(8-méthyl-8-azabicyclo[3.2.1]-oct-3-yl)-1,2,4,5-tétrahydro-cyclopent[de]isoquinoléine-1-one.

**10.** Composé suivant la revendication 9, dans lequel $R^3$ est le groupe endo-8-méthyl-8-azabicyclo[3.2.1]oct-3-yle,à savoir la 2-(endo-8-méthyl-8-azabicyclo[3.2.1]-oct-3-yl)-1,2,4,5-tétrahydro-cyclopent[de]-isoquinoléine-1-one.

**11.** Composé suivant la revendication 2, dans lequel n est égal à 2.

**12.** Composé suivant la revendication 11, dans lequel p est égal à 0, le segment en traits interrompus représente une double liaison et si $R^3$ comprend $R^4$ et $R^5$, chacun d'eux est un groupe méthyle.

**13.** Composé suivant la revendication 12, dans lequel $R^3$ est un groupe :

1-azabicyclo[2.2.2]oct-3-yle ;

1-azabicyclo[2.2.2]oct-4-yle ;

endo-9-méthyl-9-azabicyclo[3.3.1]non-3-yle ;

endo-8-méthyl-8-azabicyclo[3.2.1]oct-3-yle ;

exo-8-méthyl-8-azabicyclo[3.2.1]oct-3-yle ; ou

endo-1-azabicyclo[3.3.1]none-4-yle.

**14.** Composé suivant la revendication 13, dans lequel $R^3$ est le groupe 1-aza-bicyclo[2.2.2]oct-4-yle, à savoir la 2-(1-azabicyclo[2.2.2]oct-4-yl)-2,4,5,6-tétrahydro-1H-benz[de]isoquinoléine-1-one.

**15.** Composé suivant la revendication 13, dans lequel $R^3$ est le groupe exo-8-méthyl-8-azabicyclo[3.2.1]oct-3-yle, à savoir la 2-(exo-8-méthyl-8-azabicyclo[3.2.1]oct-3-yl)-2,4,5,6-tétrahydro-1H-benz[de]-isoquinoléine-1-one.

**16.** Composé suivant la revendication 13, dans lequel $R^3$ est le groupe endo-8-méthyl-8-azabicyclo[3.2.1]-oct-3-yle,à savoir la 2-(endo-8-méthyl-8-azabicyclo[3.2.1]oct-3-yl)-2,4,5,6-tétrahydro-1H-benz[de]-isoquinoléine-1-one.

**17.** Composé suivant la revendication 13, dans lequel $R^3$ est le groupe 1-azabicyclo[2.2.2]oct-3-yle, à savoir la 2-(1-azabicyclo[2.2.2]oct-3-yl)-2,4,5,6-tétrahydro-1H-benz[de]isoquinoléine-1-one.

**18.** Composé suivant la revendication 17, qui est le chlorhydrate de (S)-2-(1-azabicyclo[2.2.2]oct-3-yl)-2,4,5,6-tétrahydro-1H-benz[de]isoquinoléine-1-one.

**19.** Composé suivant la revendication 17, qui est la (S)-2-(1-azabicyclo[2.2.2]oct-3-yl)-2,4,5,6-tétrahydro-1H-benz[de]isoquinoléine-1-one sous forme de la base libre.

**20.** Composé suivant la revendication 17, qui est la (R)-2-(1-azabicyclo[2.2.2]oct-3-yl)-2,4,5,6-tétrahydro-1H-benz[de]isoquinoléine-1-one.

**21.** Composé suivant la revendication 13, dans lequel $R^3$ est le groupe endo-9-méthyl-9-azabicyclo[3.3.1]-non-3-yle,à savoir la 2-(endo-9-méthyl-9-azabicyclo[3.3.1]non-3-yl)-2,4,5,6-tétrahydro-1H-benz[de]-isoquinoléine-1-one.

**22.** Composé suivant la revendication 13, dans lequel $R^3$ est le groupe endo-1-azabicyclo[3.3.1]non-4-yle, à savoir la 2-(endo-1-azabicyclo[3.3.1]non-4-yl)-2,4,5,6-tétrahydro-1H-benz[de]isoquinoléine-1-one.

**23.** Composé suivant la revendication 2, dans lequel p est égal à 0, le segment en traite interrompus représente doux atomes d'hydrogène et si $R^3$ comprend $R^4$ et $R^5$, chacun d'eux est un groupe méthyle.

**24.** Composé suivant la revendication 23, dans lequel $R^3$ représente un groupe :
1-azabicyclo[2.2.2]oct-3-yle ;
1-azabicyclo[2.2.2]oct-4-yle ;
endo-9-méthyl-9-azabicyclo[3.3.1]non-3-yle ;
endo-8-méthyl-8-azabicyclo[3.2.1]oct-3-yle ;
exo-8-méthyl-8-azabicyclo[3.2.1]oct-3-yle ; ou
endo-1-azabicyclo[3.3.1]non-4-yle.

**25.** Composé suivant la revendication 24, dans lequel n est égal à 1.

**26.** Composé suivant la revendication 24, dans lequel n est égal à 2.

**27.** Composé suivant la revendication 26, dans lequel $R^3$ est le groupe 1-azabicyclo[2.2.2]oct-3-yle, à savoir la 2-(1-azabicyclo[2.2.2]oct-3-yl)-2,3,3a,4,5,6-hexahydro-1H-benz[de]isoquinoléine-1-one, en particulier son isomère (3aS, 3'S) et son chlorhydrate.

**28.** Composé suivant la revendication 1, dans lequel
n est égal à 3 ;
p a la valeur 0, 1 ou 2 ;
q est égal à 0 ;
$R^1$ est un halogène, un groupe alkoxy inférieur en $C_1$ à $C_6$ ou amino ;
et si $R^3$ comprend
$R^4$ et $R^5$, chacun d'eux représente un groupe alkyle inférieur en $C_1$ à $C_6$.

**29.** Composé suivant la revendication 28, dans lequel p est égal à 0, le segment en traits interrompus représente une double liaison, et si $R^3$ comprend $R^4$ et $R^5$, chacun d'eux est un groupe méthyle.

**30.** Composé suivant la revendication 29, dans lequel $R^3$ est un groupe :
1-azabicyclo[2.2.2]oct-3-yle ;
1-azabicyclo[2.2.2]oct-4-yle ;
endo-9-méthyl-9-azabicyclo[3.3.1]non-3-yle ;
endo-8-méthyl-8-azabicyclo[3.2.1]oct-3-yle ;
exo-8-méthyl-8-azabicyclo[3.2.1]oct-3-yle ; ou
endo-1-azabicyclo[3.3.1]non-4-yle.

**31.** Composé suivant la revendication 30, dans lequel $R^3$ est le groupe 1-azabicyclo[2.2.2]oct-3-yle, à savoir la (RS)-2-(1-azabicyclo[2.2.2]oct-3-yl)-1,2,4,5,6,7-hexahydrocyclohept[de]isoquinoléine-1-one.

**32.** N-oxyde du composé suivant la revendication 1, dans lequel
p a la valeur 0, 1 ou 2 ;
q est égal à 0 ;
$R^1$ est un halogène, un groupe alkoxy inférieur en $C_1$ à $C_6$ ou amino ;
et si $R^3$ comprend
$R^4$ et $R^5$, chacun d'eux représente un groupe alkyle inférieur en $C_1$ à $C_6$.

**33.** Composé suivant la revendication 32, dans lequel p est égal à 0 et, si $R^3$ comprend $R^4$ et $R^5$, chacun d'eux est un groupe méthyle.

**34.** Composé suivant la revendication 33, dans lequel n est égal à 2 et $R^3$ est un groupe 1-azabicyclo-[2.2.2]oct-3-yle.

**35.** Composé suivant l'une quelconque des revendications 1 à 34, destiné à être utilisé comme produit pharmaceutique.

**36.** Composition pharmaceutique, comprenant une quantité à effet thérapeutique d'un composé suivant les revendications 1 à 34, de préférence en association avec un excipient acceptable du point de vue pharmaceutique.

**37.** Utilisation d'un composé suivant les revendications 1 à 34 dans la préparation d'un médicament destiné au traitement d'un état choisi entre émèse, trouble gastrointestinal, trouble du système nerveux central, trouble cardio-vasculaire et douleur ou état dans lequel le récepteur de 5-$HT_3$ joue un rôle.

**38.** Procédé de production d'un composé de formule I

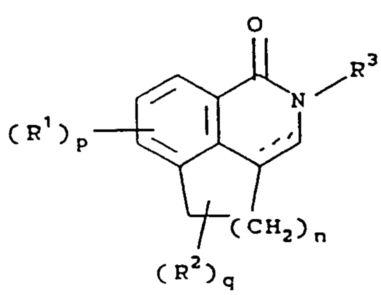

I

dans laquelle
n a la valeur 1, 2 ou 3 ;
p a la valeur 0, 1, 2 ou 3 ;
q a la valeur 0, 1 ou 2 ;
chaque $R^1$ est choisi indépendamment entre un halogène, un groupe hydroxy, alkoxy inférieur en $C_1$ à $C_6$ (facultativement substitué avec un radical phényle), alkyle inférieur en $C_1$ à $C_6$, nitro, amino, amino-carbonyle, (alkyle inférieur en $C_1$ à $C_6$)amino, di(alkyle inférieur en $C_1$ à $C_6$)amino et (alcanoyle inférieur en $C_1$ à $C_6$)amino ;
chaque $R^2$ est un groupe alkyle inférieur en $C_1$ à $C_6$ ; et

$R^3$ est choisi entre

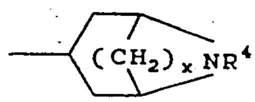

(a)

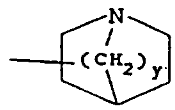

(b)

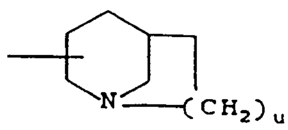

(c)

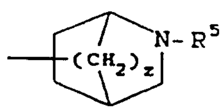

(d)

où

u, x, y et z représentent tous, indépendamment, un nombre entier de 1 à 3 ; et

$R^4$ et $R^5$ représentent, indépendamment, un groupe alkyle en $C_1$ à $C_7$, cycloalkyle en $C_3$ à $C_8$, (cycloalkyle en $C_3$ à $C_8$)-(alkyle en $C_1$ ou $C_2$) ou un groupe $(CH_2)_tR_6$ dans lequel t a la valeur 1 ou 2 et $R_6$ est un groupe thiényle, pyrrolyle ou furyle portant en outre facultativement un ou deux substituants choisis entre un radical alkyle en $C_1$ à $C_6$, alkoxy en $C_1$ à $C_6$, trifluorométhyle ou halogéno, ou représente un groupe phényle portant facultativement un ou deux substituants choisis entre des radicaux alkoxy en $C_1$ à $C_4$, trifluorométhyle, halogéno, nitro, carboxy, carboxy estérifié et alkyle en $C_1$ à $C_4$ (portant facultativement un substituant hydroxy, alkoxy en $C_1$ à $C_4$), carboxy, carboxy estérifié ou acyloxy hydrolysable in vivo) ; ou d'un sel ou d'un N-oxyde pharmaceutiquement acceptable de ce composé, ou d'un isomère individuel ou d'un mélange d'isomères de ce composé, procédé qui comprend une ou plusieurs des étapes suivantes :

(a) mise en contact réactive d'un composé de formule II

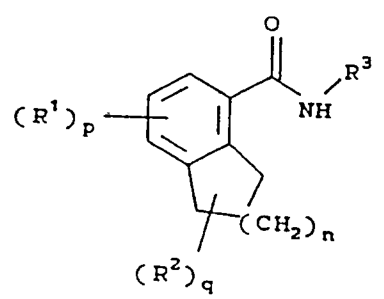

II

dans laquelle n, p, q, $R^1$ , $R^2$ et $R^3$ son tels que définis ci-dessus, avec un agent de formylation en présence d'une base forte pour former un composé de formule I dans laquelle le segment en traits interrompus est une double liaison,

(b) réduction de la double liaison représentée par le segment en traits interrompus dans la formule I par hydrogénation pour former un composé de formule I dans laquelle le segment en traits interrompus représente deux atomes d'hydrogène,

(c) transformation d'un sel d'un composé de formule I en le composé libre correspondant,

(d) condensation d'un composé de formule $R^3L$ dans laquelle $R^3$ a la définition indiquée ci-dessus et L est un groupe partant, avec un composé de formule XIII,

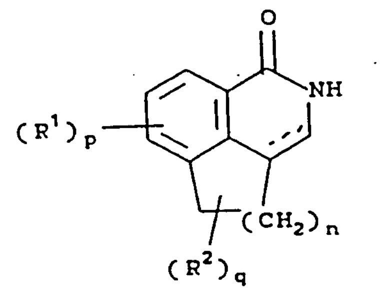

XIII

dans laquelle $R^1$, $R^2$, n, p, q et le segment en traits interrompus ont les définitions indiquées ci-dessus,

(e) transformation d'un composé de formule I en le sel pharmaceutiquement acceptable correspondant,

(f) oxydation d'un composé de formule I pour former le N-oxyde correspondant du composant $R^3$ de formule I, ou réduction d'un N-oxyde du composant $R^3$ en l'amine correspondante,

(g) réduction d'un substituant nitro $R^1$ en un substituant amino $R^1$ ou alkylation ou acylation d'un substituant amino $R^1$ ou alkylation d'un substituant hydroxy $R^1$ ou désalkylation d'un substituant alkoxy $R^1$ ou débenzylation d'un substituant benzyloxy $R^1$ en le compose correspondant de formule I,

(h) hydrogénation dans les positions 3a, 4, 5 et 6 d'un composé de formule XIV

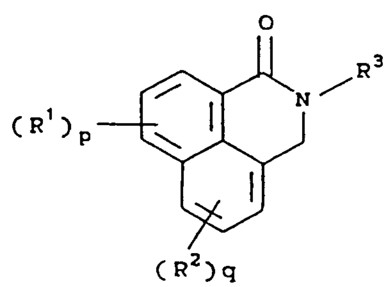

XIV

dans laquelle $R^1$, $R^2$, $R^3$, p et q ont les définitions données ci-dessus,

(i) fractionnement d'un mélange d'isomères ou de diastéréoisomères d'un composé de formule I en un isomère individuel ou un diastéréoisomère, ou bien

(j) conduite de l'une quelconque des étapes (a) à (i) avec des corps réactionnels optiquement actifs.

**39.** Procédé suivant la revendication 38, dans lequel $R^3$ est un groupe
1-azabicyclo[2.2.2]oct-3-yle ;
1-azabicyclo[2.2.2]oct-4-yle ;
endo-9-méthyl-9-azabicyclo[3.3.1]non-3-yle ;
exo-8-méthyl-8-azabicyclo[3.2.1]oct-3-yle ; ou
endo-1-azabicyclo[3.3.1]non-4-yle.

**40.** Composé représenté par la formule

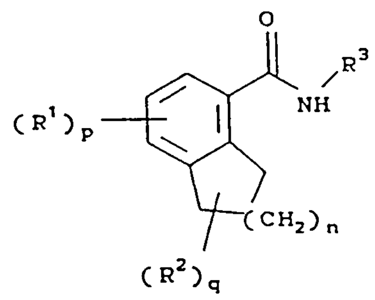

II

dans laquelle

n a la valeur 1, 2 ou 3 ;

p a la valeur 0, 1, 2 ou 3 ;

q a la valeur 0, 1 ou 2 ;

chaque $R^1$ est choisi indépendamment entre un halogène, un radical hydroxy, alkoxy inférieur en $C_1$ à $C_6$ (facultativement substitué avec un radical phényle), alkyle inférieur en $C_1$ à $C_6$, nitro, amino, aminocarbonyle,(alkyle inférieur en $C_1$ à $C_6$)amino, di(alkyle inférieur en $C_1$ à $C_6$)amino et (alcanoyle inférieur en $C_1$ à $c_6$)amino ;

chaque $R^2$ est un groupe alkyle inférieur en $C_1$ à $C_6$ ; et

$R^3$ est choisi entre

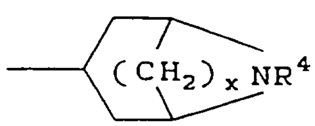

(a)

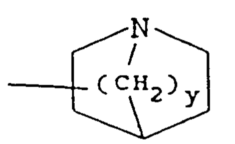

(b)

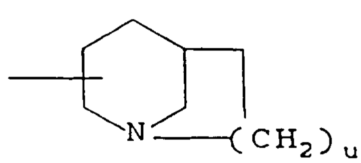

(c)

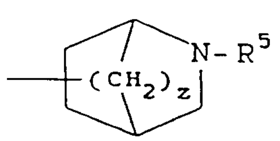

(d)

où

u, x, y et z représentent tous, indépendamment, un nombre entier de 1 à 3 ; et

$R^4$ et $R^5$ représentent, indépendamment, un groupe alkyle en $C_1$ à $C_7$, cycloalkyle en $C_3$ à $C_8$, (cycloalkyle en $C_3$ à $C_8$)-(alkyle en $C_1$ ou $C_2$) ou un groupe $(CH_2)_tR_6$ dans lequel t a la valeur 1 ou 2 et $R_6$ est un groupe thiényle, pyrrolyle ou furyle portant en outre facultativement un ou deux substituants choisis entre des radicaux alkyle en $C_1$ à $C_6$, alkoxy en $C_1$ à $C_6$, trifluorométhyle ou halogéno, ou représente un groupe phényle portant facultativement un ou deux substituants choisis entre des radicaux alkoxy en $C_1$ à $C_4$, trifluorométhyle, halogéno, nitro, carboxy, carboxy estérifié et alkyle en $C_1$

à $C_4$ (portant facultativement un substituant hydroxy, alkoxy en $C_1$ à $C_4$, carboxy, carboxy estérifié ou acyloxy hydrolysable in vivo) ;
ou un isomère individuel ou un mélange d'isomères de ce composé, ou un sel de ce composé.

**Revendications pour les Etats contractants suivants : ES, GR**

**1.** Procédé de production d'un composé de formule I

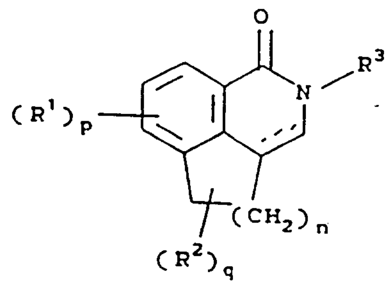

I

dans laquelle
le segment en traits interrompus représente une double liaison facultative ;
n a la valeur 1, 2 ou 3 ;
p a la valeur 0, 1, 2 ou 3 ;
q a la valeur 0, 1 ou 2 ;
chaque $R^1$ est choisi indépendamment entre un halogène, un groupe hydroxy, alkoxy inférieur en $C_1$ à $C_6$ (facultativement substitué avec un radical phényle), alkyle inférieur en $C_1$ à $C_6$, nitro, amino, amino-carbonyle, (alkyle inférieur en $C_1$ à $C_6$)amino, di(alkyle inférieur en $C_1$ à $C_6$)amino et (alcanoyle inférieur en $C_1$ à $C_6$)amino ;
chaque $R^2$ est un groupe alkyle inférieur en $C_1$ à $C_6$ ; et
$R^3$ est choisi entre

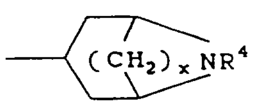

(a)

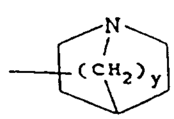

(b)

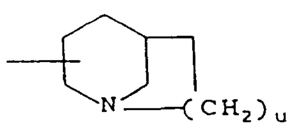

(c)

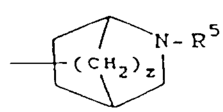

(d)

où
u, x, y et z représentent tous, indépendamment, un nombre entier de 1 à 3 ; et
$R^4$ et $R^5$ représentent, indépendamment, un groupe alkyle en $C_1$ à $C_7$, cycloalkyle en $C_3$ à $C_8$, (cycloalkyle en $C_3$ à $C_8$)-(alkyle en $C_1$ ou $C_2$) ou un groupe $(CH_2)_tR_6$ dans lequel t a la valeur 1 ou 2 et $R_6$ est un groupe thiényle, pyrrolyle ou furyle portant en outre facultativement un ou deux substituants choisis entre un radical alkyle en $C_1$ à $C_6$, alkoxy en $C_1$ à $C_6$, trifluorométhyle ou halogéno, ou

représente un groupe phényle portant facultativement un ou deux substituants choisis entre des radicaux alkoxy en $C_1$ à $C_4$, trifluorométhyle, halogéno, nitro, carboxy, carboxy estérifié et alkyle en $C_1$ à $C_4$ (portant facultativement un substituant hydroxy, alkoxy en $C_1$ à $C_4$), carboxy, carboxy estérifié ou acyloxy hydrolysable in vivo) ; ou d'un sel ou d'un N-oxyde pharmaceutiquement acceptable de ce composé ; ou d'un isomère individuel ou d'un mélange d'isomères de ce composé, procédé qui comprend une ou plusieurs des étapes suivantes :

(a) mise en contact réactive d'un composé de formule II

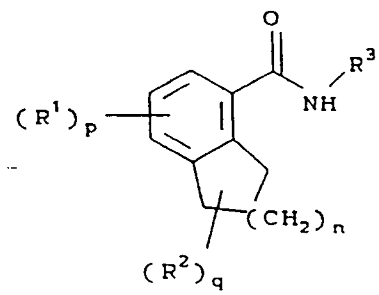

II

dans laquelle n, p, q, $R^1$ , $R^2$ et $R^3$ son tels que définis ci-dessus, avec un agent de formylation en présence d'une base forte pour former un composé de formule I dans laquelle le segment en traits interrompus est une double liaison,

(b) réduction de la double liaison représentée par le segment en traits interrompus dans la formule I par hydrogénation pour former un composé de formule I dans laquelle le segment en traits interrompus représente deux atomes d'hydrogène,

(c) transformation d'un sel d'un composé de formule I en le composé libre correspondant,

(d) condensation d'un composé de formule $R^3L$ dans laquelle $R^3$ a la définition indiquée ci-dessus et L est un groupe partant, avec un composé de formule XIII,

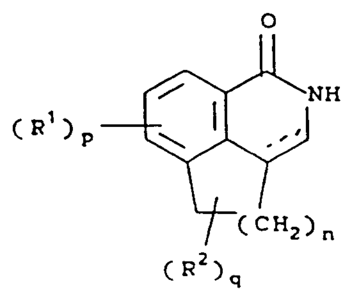

XIII

dans laquelle $R^1$, $R^2$, n, p, q et le segment en traits interrompus ont les définitions indiquées ci-dessus,

(e) transformation d'un composé de formule I en le sel pharmaceutiquement acceptable correspondant,

(f) oxydation d'un composé de formule I pour former le N-oxyde correspondant du composant $R^3$ de formule I, ou réduction d'un N-oxyde du composant $R^3$ en l'amine correspondante,

(g) réduction d'un substituant nitro $R^1$ en un substituant amino $R^1$ ou alkylation ou acylation d'un substituant amino $R^1$ ou alkylation d'un substituant hydroxy $R^1$ ou désalkylation d'un substituant alkoxy $R^1$ ou débenzylation d'un substituant benzyloxy $R^1$ en le composé correspondant de formule I,

(h) hydrogénation dans les positions 3a, 4, 5 et 6 d'un composé de formule XIV

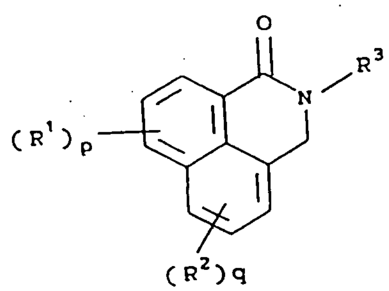

XIV

dans laquelle $R^1$, $R^2$, $R^3$, p et q ont les définitions données ci-dessus,
(i) fractionnement d'un mélange d'isomères ou de diastéréoisomères d'un composé de formule I en un isomère individuel ou d'un diastéréoisomère, ou bien
(j) conduite de l'une quelconque des étapes (a) à (i) avec des corps réactionnels optiquement actifs.

**2.** Procédé suivant la revendication 1, dans lequel
p a la valeur 0, 1 ou 2 ;
n a la valeur 1 ou 2 ;
q est égal à 0 ;
$R^1$ est un halogène, un groupe alkoxy inférieur en $C_1$ à $C_6$ ou amino ;
et si $R^3$ comprend
$R^4$ et $R^5$, chacun d'eux est un groupe alkyle inférieur en $C_1$ à $C_6$.

**3.** Procédé suivant la revendication 2, dans lequel n est égal à 1.

**4.** Procédé suivant la revendication 3, dans lequel p est égal à 0, le segment en traits interrompus représente une double liaison et si $R^3$ comprend $R^4$ et $R^5$, chacun d'eux est un groupe méthyle.

**5.** Procédé suivant la revendication 2 ou 4, dans lequel $R^3$ représente un groupe
1-azabicyclo[2.2.2]oct-3-yle ;
1-azabicyclo[2.2.2]oct-4-yle ;
endo-9-méthyl-9-azabicyclo[3.3.1]non-3-yle ;
endo-8-méthyl-8-azabicyclo[3.2.1]oct-3-yle ;
exo-8-méthyl-8-azabicyclo[3.2.1]oct-3-yle ; ou
endo-1-azabicyclo[3.3.1]non-4-yle.

**6.** Procédé suivant la revendication 5, dans lequel on prépare un composé dans lequel $R^3$ est le groupe 1-azabicyclo[2.2.2]oct-3-yle, à savoir la 2-(1-azabicyclo[2.2.2]oct-3-yl)-1,2,4,5-tétrahydrocyclopent[de]-isoquinoléine-1-one.

**7.** Procédé suivant la revendication 6, dans lequel on prépare le chlorhydrate de 2-(1-azabicyclo[2.2.2]oct-3-yl)-1,2,4,5-tétrahydrocyclopenta[de]isoquinoléine-1-one.

**8.** Procédé suivant la revendication 6, dans lequel on prépare le chlorhydrate de (S)-2-(1-azabicyclo-[2.2.2]oct-3-yl)-1,2,4,5-tétrahydrocyclopenta[de]isoquinoléine-1-one.

**9.** Procédé suivant la revendication 5, dans lequel on prépare un composé dans lequel $R^3$ est le groupe 8-méthyl-8-azabicyclo[3.2.1]oct-3-yle, à savoir la 2-(8-méthyl-8-azabicyclo[3.2.1]-oct-3-yl)-1,2,4,5-tétrahydro-cyclopent[de]isoquinoléine-1-one.

**10.** Procédé suivant la revendication 9, dans lequel on prépare un composé dans lequel $R^3$ est le groupe endo-8-méthyl-8-azabicyclo[3.2.1]oct-3-yle, à savoir la 2-(endo-8-méthyl-8-azabicyclo[3.2.1]-oct-3-yl)-1,2,4,5-tétrahydrocyclopent[de]isoquinoléine-1-one.

**11.** Procédé suivant la revendication 2, dans lequel n est égal à 2.

**12.** Procédé suivant la revendication 11, dans lequel p est égal à 0, le segment en traits interrompus représente une double liaison et si $R^3$ comprend $R^4$ et $R^5$, chacun d'eux est un groupe méthyle.

**13.** Procédé suivant la revendication 12, dans lequel $R^3$ représente un groupe :
1-azabicyclo[2.2.2]oct-3-yle ;
1-azabicyclo[2.2.2]oct-4-yle ;
endo-9-méthyl-9-azabicyclo[3.3.1]non-3-yle ;
endo-8-méthyl-8-azabicyclo[3.2.1]oct-3-yle ;
exo-8-méthyl-8-azobicyclo[3.2.1]oct-3-yle ; ou
endo-1-azabicyclo[3.3.1]non-4-yle.

**14.** Procédé suivant la revendication 13, dans lequel on prépare un composé dans lequel $R^3$ est le groupe 1-aza-bicyclo[2.2.2]oct-4-yle, à savoir la 2-(1-azabicyclo[2.2.2]oct-4-yl)-2,4,5,6-tétrahydro-1H-benz[de]-isoquinoléine-1-one.

**15.** Procédé suivant la revendication 13, dans lequel on prépare un composé dans lequel $R^3$ est le groupe exo-8-méthyl-8-azabicyclo[3.2.1]oct-3-yle, à savoir la 2-(exo-8-méthyl-8-azabicyclo[3.2.1]oct-3-yl)-2,4,5,6-tétrahydro-1H-benz[de]isoquinoléine-1-one.

**16.** Procéde suivant la revendication 13, dans lequel on prépare un composé dans lequel $R^3$ est le groupe endo-8-méthyl-8-azabicyclo[3.2.1]oct-3-yle, à savoir la 2-(endo-8-méthyl-8-azabicyclo[3.2.1]oct-3-yl)-2,4,5,6-tétrahydro-1H-benz[de]isoquinoléine-1-one.

**17.** Procédé suivant la revendication 13, dans lequel on prépare un composé dans lequel $R^3$ est le groupe 1-azabicyclo[2.2.2]oct-3-yle, à savoir la 2-(1-azabicyclo[2.2.2]oct-3-yl)-2,4,5,6-tétrahydro-1H-benz[de]-isoquinoléine-1-one.

**18.** Procédé suivant la revendication 17, dans lequel on prépare le chlorure de la (S)-2-(1-azabicyclo[2.2.2]-oct-3-yl)-2,4,5,6-tétrahydro-1H-benz[de]isoquinoléine-1-one.

**19.** Procédé suivant la revendication 17, dans lequel on prépare la (S)-2-(1-azabicyclo[2.2.2]oct-3-yl)-2,4,5,6-tétrahydro-1H-benz[de]isoquinoléine-1-one sous forme de la base libre.

**20.** Procédé suivant la revendication 17, dans lequel on prépare la (R)-2-(1-azabicyclo[2.2.2]oct-3-yl)-2,4,5,6-tétrahydro-1H-benz[de]isoquinoléine-1-one.

**21.** Procédé suivant la revendication 13, dans lequel on prépare un composé dans lequel $R^3$ est le groupe endo-9-méthyl-9-azabicyclo[3.3.1]non-3-yle, à savoir la 2-(endo-9-méthyl-9-azabicyclo[3.3.1]non-3-yl)-2,4,5,6-tétrahydro-1H-benz[de]isoquinoléine-1-one.

**22.** Procédé suivant la revendication 13, dans lequel on prépare un composé dans lequel $R^3$ est le groupe endo-1-azabicyclo[3.3.1]non-4-yle, à savoir la 2-(endo-1-azabicyclo[3.3.1]non-4-yl)-2,4,5,6-tétrahydro-1H-benz[de]isoquinoléine-1-one.

**23.** Procédé suivant la revendication 2, dans lequel p est égal à 0, le segment en traits interrompus représente deux atomes d'hydrogène et si $R^3$ comprend $R^4$ et $R^5$, chacun d'eux est un groupe méthyle.

**24.** Procédé suivant la revendication 23, dans lequel $R^3$ représente un groupe :
1-azabicyclo[2.2.2]oct-3-yle ;
1-azabicyclo[2.2.2]oct-4-yle ;
endo-9-méthyl-9-azabicyclo[3.3.1]non-3-yle ;
endo-8-méthyl-8-azabicyclo[3.2.1]oct-3-yle ;
exo-8-méthyl-8-azabicyclo[3.2.1]oct-3-yle ; ou
endo-1-azabicyclo[3.3.1]non-4-yle.

**25.** Procédé suivant la revendication 24, dans lequel n est égal à 1.

**26.** Procédé suivant la revendication 24, dans lequel n est égal à 2.

**27.** Procédé suivant la revendication 26, dans lequel on prépare un composé dans lequel $R^3$ est le groupe 1-azabicyclo[2.2.2]oct-3-yle, à savoir la 2-(1-azabicyclo[2.2.2]oct-3-yl)-2,3,3a,4,5,6-hexahydro-1H-benz-[de]isoquinoléine-1-one, en particulier son isomère (3aS, 3'S) et son chlorhydrate.

**28.** Procédé suivant la revendication 1, dans lequel
n est égal à 3 ;
p a la valeur 0, 1 ou 2 ;
q est égal à 0 ;
$R^1$ est un halogène, un groupe alkoxy inférieur en $C_1$ à $C_6$ ou amino ;
et si $R^3$ comprend
$R^4$ et $R^5$, chacun d'eux représente un groupe alkyle inférieur en $C_1$ à $C_6$.

**29.** Procédé suivant la revendication 28, dans lequel p est égal à 0, le segment en traits interrompus représente une double liaison, et si $R^3$ comprend $R^4$ et $R^5$, chacun d'eux est un groupe méthyle.

**30.** Procédé suivant la revendication 29, dans lequel $R^3$ est un groupe :
1-azabicyclo[2.2.2]oct-3-yle ;
1-azabicyclo[2.2.2]oct-4-yle ;
endo-9-méthyl-9-azabicyclo[3.3.1]non-3-yle ;
endo-8-méthyl-8-azabicyclo[3.2.1]oct-3-yle ;
exo-8-méthyl-8-azabicyclo[3.2.1]oct-3-yle ; ou
endo-1-azabicyclo[3.3.1]non-4-yle.

**31.** Procédé suivant la revendication 30, dans lequel on prépare un composé dans lequel $R^3$ est le groupe 1-azabicyclo[2.2.2]oct-3-yle, à savoir la (RS)-2-(1-azabicyclo[2.2.2]oct-3-yl)-1,2,4,5,6,7-hexahydrocyclohept[de]isoquinoléine-1-one.

**32.** Procédé suivant la revendication 1 dans lequel on prépare le N-oxyde d'un composé dans lequel
p a la valeur 0, 1 ou 2 ;
q est égal à 0 ;
$R^1$ est un halogène, un radical alkoxy inférieur en $C_1$ à $C_6$ ou amino ;
et si $R^3$ comprend
$R^4$ et $R^5$, chacun d'eux représente un groupe alkyle inférieur en $C_1$ à $C_6$.

**33.** Procédé suivant la revendication 32, dans lequel p est égal à 0, et si $R^3$ comprend $R^4$ et $R^5$, chacun d'eux est un groupe méthyle.

**34.** Procédé suivant la revendication 33, dans lequel n est égal à 2 et $R^3$ est un groupe 1-azabicyclo[2.2.2]-oct-3-yle.

**35.** Procédé de production d'un composé représenté par la formule I

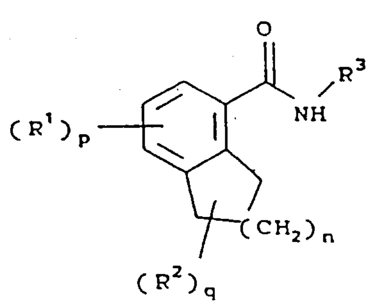

II

dans laquelle
n a la valeur 1, 2 ou 3 ;
p a la valeur 0, 1, 2 ou 3 ;

q a la valeur 0, 1 ou 2 ;

chaque $R^1$ est choisi indépendamment entre un halogène, un radical hydroxy, alkoxy inférieur en $C_1$ à $C_6$ (facultativement substitué avec un radical phényle), alkyle inférieur en $C_1$ à $C_6$, nitro, amino, amino-carbonyle, (alkyle inférieur en $C_1$ à $C_6$)amino, di(alkyle inférieur en $C_1$ à $C_6$)amino et (alcanoyle inférieur en $C_1$ à $C_6$)amino ;

chaque $R^2$ est un groupe alkyle inférieur en $C_1$ à $C_6$ ; et

$R^3$ est choisi entre

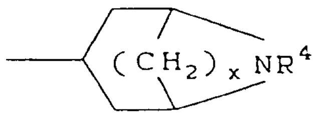

(a)

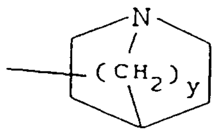

(b)

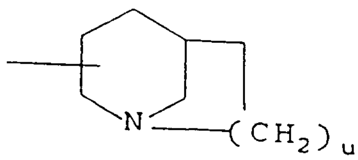

(c)

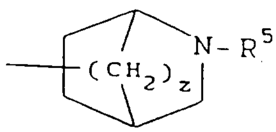

(d)

où

u, x, y et z représentent tous, indépendamment, un nombre entier de 1 à 3 ; et

$R^4$ et $R^5$ représentent, indépendamment, un groupe alkyle en $C_1$ à $C_7$, cycloalkyle en $C_3$ à $C_8$, (cycloalkyle en $C_3$ à $C_8$)-(alkyle en $C_1$ ou $C_2$) ou un groupe $(CH_2)_tR_6$ dans lequel t a la valeur 1 ou 2 et $R_6$ est un groupe thiényle, pyrrolyle ou furyle portant en outre facultativement un ou deux substituants choisis entre un radical alkyle en $C_1$ à $C_6$, alkoxy en $C_1$ à $C_6$, trifluorométhyle ou halogéno, ou représente un groupe phényle portant facultativement un ou deux substituants choisis entre un radical alkoxy en $C_1$ à $C_4$, trifluorométhyle, halogéno, nitro, carboxy, carboxy estérifié et alkyle en $C_1$ à $C_4$ - (facultativement substitué par un radical hydroxy, alkoxy en $C_1$ à $C_4$, carboxy, carboxy estérifié ou acyloxy hydrolysable in vivo) ;

ou d'un isomère individuel ou d'un mélange d'isomères de ce composé ou d'un sel de ce composé, procédé qui comprend la réaction d'un composé de formule III

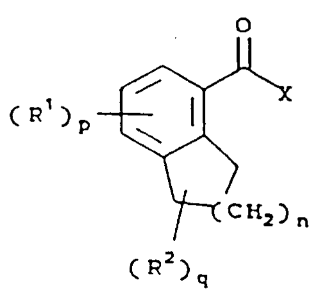

III

dans laquelle

X représente OH, OR (R = alkyle) ou un halogène ; et

n, p, q, $R^1$ et $R^2$ sont tels que définis ci-dessus,

avec une amine de formule $R^3NH_2$, dans laquelle $R^3$ est tel que défini ci-dessus.